# EUROPEAN PATENT APPLICATION

(11) **EP 4 015 515 A1**
(43) Date of publication of application: **22.06.2022**
(21) Application number: 20855736.3
(22) Date of filing: 14.08.2020
(51) Int. Cl.: C07D 405/10, C07D 471/04, C07D 405/14, C07D 409/14, H01L 51/00, H01L 51/50

(54) **HETEROCYCLIC COMPOUND AND ORGANIC LIGHT-EMITTING DEVICE COMPRISING SAME**

(30) Priority: 16.08.2019 KR 20190100204
(71) Applicant: LT Materials Co., Ltd., Yongin-si, Gyeonggi-do 17118 (KR)
(72) Inventor: LEE, Nam-Jin, Yongin-si, Gyeonggi-do 17118 (KR); JEONG, Yu-Jun, Yongin-si, Gyeonggi-do 17118 (KR); JEONG, Won-Jang, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2020/010865
(87) International publication number: WO 2021/034039

(57) **Abstract**

The present application provides a heterocyclic compound capable of significantly enhancing lifetime, efficiency, electrochemical stability and thermal stability of an organic light emitting device, and an organic light emitting device comprising the heterocyclic compound in an organic material layer.

## Description

### [Technical Field]

This application claims priority to and the benefits of Korean Patent Application No. 10-2019-0100204, filed with the Korean Intellectual Property Office on August 16, 2019, the entire contents of which are incorporated herein by reference.

The present specification relates to a heterocyclic compound, and an organic light emitting device comprising the same.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

(Patent Document 1) US Patent No. 4,356,429

### [Disclosure]

### [Technical Problem]

The present specification is directed to providing a heterocyclic compound, and an organic light emitting device comprising the same.

### [Technical Solution]

One embodiment of the present application provides a heterocyclic compound represented by the following Chemical Formula 1.

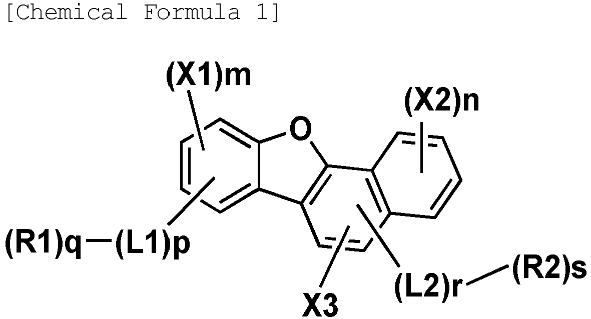

In Chemical Formula 1,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms,
R1 and R2 are the same as or different from each other, and each independently a cyano group; a substituted or unsubstituted aryl group having 6 to 40 carbon atoms; a substituted or unsubstituted heteroaryl group having 2 to 40 carbon atoms; or a substituted or unsubstituted phosphine oxide group,
X1 to X3 are the same as or different from each other, and each independently hydrogen; deuterium; or a cyano group, m is an integer of 0 to 3, n is an integer of 0 to 4, and when m and n are 2 or greater, substituents in the parentheses are the same as or different from each other,
p and r are an integer of 0 to 4,
q and s are an integer of 1 to 6, and
when p, q, r and s are 2 or greater, substituents in the parentheses are the same as or different from each other.

Another embodiment of the present application provides an organic light emitting device comprising a first electrode; a second electrode; and one or more organic material layers provided between the first electrode and the second electrode, wherein one or more layers of the organic material layers comprise the heterocyclic compound represented by Chemical Formula 1.

### [Advantageous Effects]

A compound described in the present specification can be used as a material of an organic material layer of an organic light emitting device. In the organic light emitting device, the compound is capable of performing a role of a hole injection material, a hole transfer material, a light emitting material, an electron transfer material, an electron injection material or the like. Particularly, the compound can be used as an electron transfer layer material, a hole blocking layer material or a charge generation layer material of an organic light emitting device.

Specifically, when using the heterocyclic compound represented by Chemical Formula 1 in an organic material layer, a driving voltage of the device can be lowered, light efficiency can be enhanced, and lifetime properties of the device can be enhanced.

### [Description of Drawings]

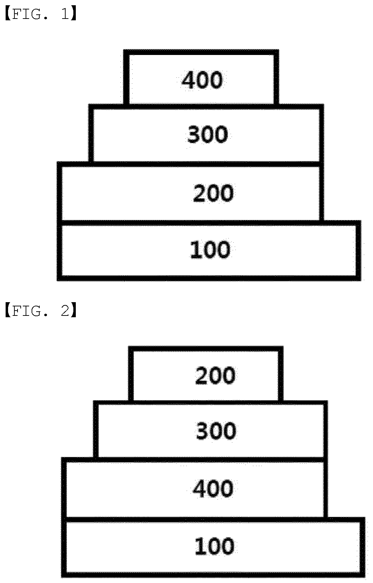

FIG. 1 to FIG. 4 are diagrams each illustrating a lamination structure of an organic light emitting device according to one embodiment of the present application.

### [Mode for Disclosure]

Hereinafter, the present specification will be described in more detail.

In the present specification, a certain part "comprising" certain constituents means capable of further comprising other constituents, and does not exclude other constituents unless particularly stated on the contrary.

The term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of a linear or branched alkyl group having 1 to 60 carbon atoms; a linear or branched alkenyl group having 2 to 60 carbon atoms; a linear or branched alkynyl group having 2 to 60 carbon atoms; a monocyclic or polycyclic cycloalkyl group having 3 to 60 carbon atoms; a monocyclic or polycyclic heterocycloalkyl group having 2 to 60 carbon atoms; a monocyclic or polycyclic aryl group having 6 to 60 carbon atoms; a monocyclic or polycyclic heteroaryl group having 2 to 60 carbon atoms; a silyl group; a phosphine oxide group; and an amine group, or being unsubstituted, or being substituted with a substituent linking two or more substituents selected from among the substituents illustrated above.

More specifically, in the present specification, "substituted or unsubstituted" may mean being substituted with one or more substituents selected from the group consisting of a monocyclic or polycyclic aryl group having 6 to 60 carbon atoms; or a monocyclic or polycyclic heteroaryl group having 2 to 60 carbon atoms, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or 2H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 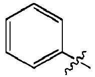 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

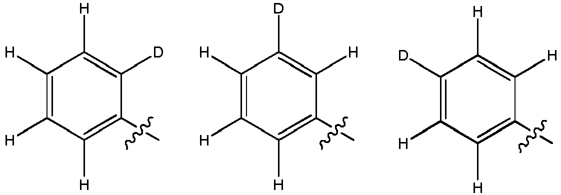

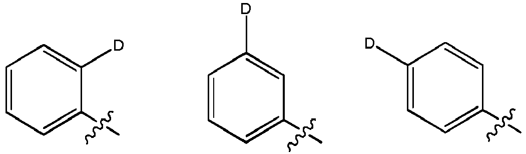

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may comprise methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group comprises monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group comprises a spiro group. The number of carbon atoms of the aryl group may be from 6 to 60, specifically from 6 to 40 and more specifically from 6 to 25. Specific examples of the aryl group may comprise a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may comprise a diphenylphosphine oxide group, a dinaphthylphosphine oxide group and the like, but are not limited thereto.

In the present specification, the silyl group is a substituent comprising Si, having the Si atom directly linked as a radical, and is represented by -SiR₁₀₄R₁₀₅R₁₀₆. R₁₀₄ to R₁₀₆ are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group comprising a spiro structure, and may have 15 to 60 carbon atoms. For example, the spiro group may comprise structures in which a 2,3-dihydro-1H-indene group or a cyclohexane group spiro bonds to a fluorenyl group. Specifically, the spiro group may comprise any one of groups of the following structural formulae.

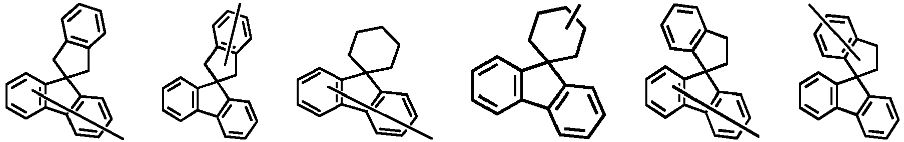

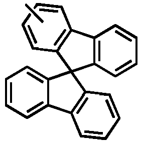

In the present specification, the heteroaryl group comprises O, S, Se, N or Si as a heteroatom, comprises monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

One embodiment of the present application provides a heterocyclic compound represented by the following Chemical Formula 1.

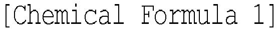

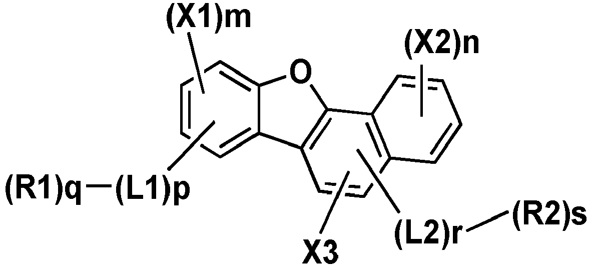

In Chemical Formula 1,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms,
R1 and R2 are the same as or different from each other, and each independently a cyano group; a substituted or unsubstituted aryl group having 6 to 40 carbon atoms; a substituted or unsubstituted heteroaryl group having 2 to 40 carbon atoms; or a substituted or unsubstituted phosphine oxide group,
X1 to X3 are the same as or different from each other, and each independently hydrogen; deuterium; or a cyano group, m is an integer of 0 to 3, n is an integer of 0 to 4, and when m and n are 2 or greater, substituents in the parentheses are the same as or different from each other,
p and r are an integer of 0 to 4,
q and s are an integer of 1 to 6, and
when p, q, r and s are 2 or greater, substituents in the parentheses are the same as or different from each other.

In the heterocyclic compound provided in one embodiment of the present application, Chemical Formula 1 is represented by the following Chemical Formula 2.

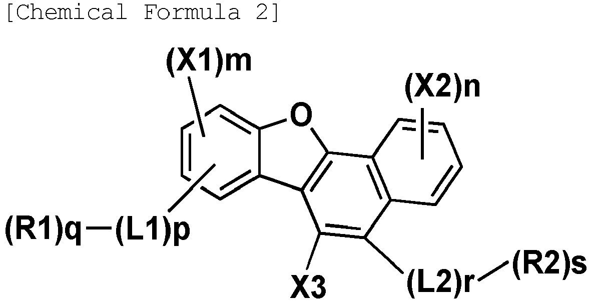

In Chemical Formula 2,
each substituent has the same definition as in Chemical Formula 1.

In the heterocyclic compound provided in one embodiment of the present application, Chemical Formula 1 is represented by any one of the following Chemical Formula 3 to Chemical Formula 6.

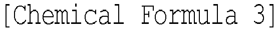

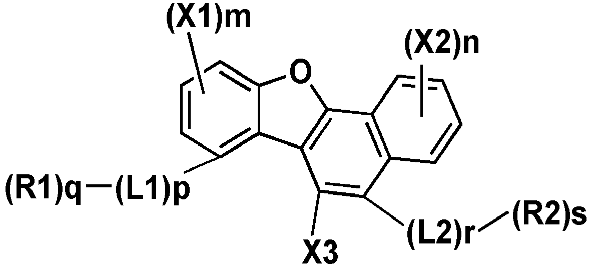

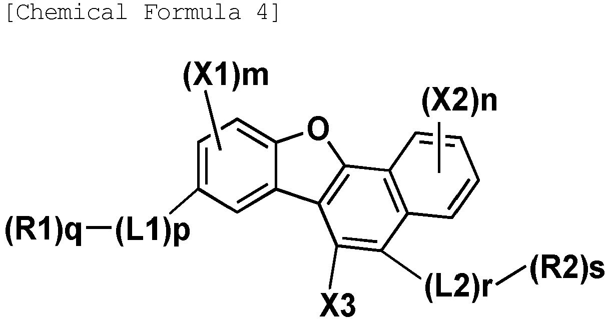

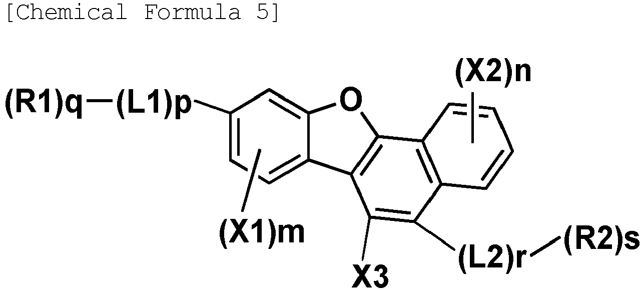

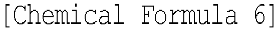

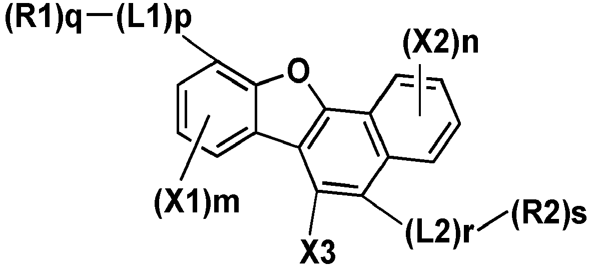

In Chemical Formula 3 to Chemical Formula 6,
each substituent has the same definition as in Chemical Formula 1.

In one embodiment of the present application, L1 and L2 are the same as or different from each other, and may be each independently a direct bond; a substituted or unsubstituted arylene group; or a substituted or unsubstituted heteroarylene group.

In another embodiment, L1 and L2 are the same as or different from each other, and may be each independently a direct bond; a substituted or unsubstituted arylene group having 6 to 60 carbon atoms; or a substituted or unsubstituted heteroarylene group having 2 to 60 carbon atoms.

In another embodiment, L1 and L2 are the same as or different from each other, and may be each independently a direct bond; a substituted or unsubstituted arylene group having 6 to 40 carbon atoms; or a substituted or unsubstituted heteroarylene group having 2 to 40 carbon atoms.

In another embodiment, L1 and L2 are the same as or different from each other, and may be each independently a direct bond; a substituted or unsubstituted arylene group having 6 to 20 carbon atoms; or a substituted or unsubstituted heteroarylene group having 2 to 20 carbon atoms.

In another embodiment, L1 and L2 are the same as or different from each other, and may be each independently a direct bond; a substituted or unsubstituted monocyclic arylene group having 6 to 10 carbon atoms; or a substituted or unsubstituted polycyclic arylene group having 10 to 20 carbon atoms.

In another embodiment, L1 and L2 are the same as or different from each other, and may be each independently a direct bond; a monocyclic arylene group having 6 to 10 carbon atoms; or a polycyclic arylene group having 10 to 20 carbon atoms.

In another embodiment, L1 and L2 are the same as or different from each other, and may be each independently a direct bond; a substituted or unsubstituted phenylene group; a substituted or unsubstituted biphenylene group; a substituted or unsubstituted naphthylene group; or a substituted or unsubstituted binaphthylene group.

In another embodiment, L1 and L2 are the same as or different from each other, and may be each independently a direct bond; a phenylene group; a biphenylene group; a naphthylene group; or a binaphthylene group.

In one embodiment of the present application, L1 and L2 may be the same as each other.

In one embodiment of the present application, L1 and L2 may be different from each other.

In one embodiment of the present application, L1 is a direct bond, and L2 may be a substituted or unsubstituted arylene group having 6 to 60 carbon atoms; or a substituted or unsubstituted heteroarylene group having 2 to 60 carbon atoms.

In one embodiment of the present application, L1 is a direct bond, and L2 may be a substituted or unsubstituted arylene group having 6 to 40 carbon atoms; or a substituted or unsubstituted heteroarylene group having 2 to 40 carbon atoms.

In one embodiment of the present application, L1 is a direct bond, and L2 may be a substituted or unsubstituted arylene group having 6 to 20 carbon atoms; or a substituted or unsubstituted heteroarylene group having 2 to 20 carbon atoms.

In one embodiment of the present application, L2 is a direct bond, and L1 may be a substituted or unsubstituted arylene group having 6 to 60 carbon atoms; or a substituted or unsubstituted heteroarylene group having 2 to 60 carbon atoms.

In one embodiment of the present application, L2 is a direct bond, and L1 may be a substituted or unsubstituted arylene group having 6 to 40 carbon atoms; or a substituted or unsubstituted heteroarylene group having 2 to 40 carbon atoms.

In one embodiment of the present application, L2 is a direct bond, and L1 may be a substituted or unsubstituted arylene group having 6 to 20 carbon atoms; or a substituted or unsubstituted heteroarylene group having 2 to 20 carbon atoms.

In one embodiment of the present application, L1 may be a direct bond; a substituted or unsubstituted arylene group having 6 to 60 carbon atoms; or a substituted or unsubstituted heteroarylene group having 2 to 60 carbon atoms.

In another embodiment, L1 may be a direct bond; a substituted or unsubstituted arylene group having 6 to 40 carbon atoms; or a substituted or unsubstituted heteroarylene group having 2 to 40 carbon atoms.

In another embodiment, L1 may be a direct bond; a substituted or unsubstituted arylene group having 6 to 20 carbon atoms; or a substituted or unsubstituted heteroarylene group having 2 to 20 carbon atoms.

In another embodiment, L1 may be a direct bond; a substituted or unsubstituted phenylene group; a substituted or unsubstituted biphenylene group; a substituted or unsubstituted naphthylene group; or a substituted or unsubstituted binaphthylene group.

In another embodiment, L1 may be a direct bond; a phenylene group; a biphenylene group; a naphthylene group; or a binaphthylene group.

In another embodiment, L1 is a direct bond.

In another embodiment, L1 is a phenylene group.

In another embodiment, L1 is a biphenylene group.

In another embodiment, L1 is a naphthylene group.

In another embodiment, L1 is a binaphthylene group.

In one embodiment of the present application, L2 may be a direct bond; a substituted or unsubstituted arylene group having 6 to 60 carbon atoms; or a substituted or unsubstituted heteroarylene group having 2 to 60 carbon atoms.

In another embodiment, L2 may be a direct bond; a substituted or unsubstituted arylene group having 6 to 40 carbon atoms; or a substituted or unsubstituted heteroarylene group having 2 to 40 carbon atoms.

In another embodiment, L2 may be a direct bond; a substituted or unsubstituted arylene group having 6 to 20 carbon atoms; or a substituted or unsubstituted heteroarylene group having 2 to 20 carbon atoms.

In another embodiment, L2 may be a direct bond; a substituted or unsubstituted phenylene group; a substituted or unsubstituted biphenylene group; a substituted or unsubstituted naphthylene group; or a substituted or unsubstituted binaphthylene group.

In another embodiment, L2 may be a direct bond; a phenylene group; a biphenylene group; a naphthylene group; or a binaphthylene group.

In another embodiment, L2 is a direct bond.

In another embodiment, L2 is a phenylene group.

In another embodiment, L2 is a biphenylene group.

In another embodiment, L2 is a naphthylene group.

In another embodiment, L2 is a binaphthylene group.

In one embodiment of the present application, X1 to X3 may be each independently hydrogen; deuterium; or a cyano group.

In one embodiment of the present application, X1 may be hydrogen; deuterium; or a cyano group.

In one embodiment of the present application, X1 is hydrogen.

In one embodiment of the present application, X1 is deuterium.

In one embodiment of the present application, X1 is a cyano group.

In one embodiment of the present application, X2 may be hydrogen; deuterium; or a cyano group.

In one embodiment of the present application, X2 is hydrogen.

In one embodiment of the present application, X2 is deuterium.

In one embodiment of the present application, X2 is a cyano group.

In one embodiment of the present application, X3 is hydrogen.

In one embodiment of the present application, X3 is deuterium.

In one embodiment of the present application, X3 is a cyano group.

In one embodiment of the present application, X1 to X3 are hydrogen.

In one embodiment of the present application, m is an integer of 0 to 3, n is an integer of 0 to 4, and when m and n are 2 or greater, substituents in the parentheses may be the same as or different from each other.

In one embodiment of the present application, m is an integer of 0 to 3.

In one embodiment of the present application, m is 3.

In one embodiment of the present application, m is 2.

In one embodiment of the present application, m is 1.

In one embodiment of the present application, m is 0.

In one embodiment of the present application, when m is 2 or greater, substituents in the parentheses are the same as or different from each other.

In one embodiment of the present application, n is an integer of 0 to 4.

In one embodiment of the present application, n is 4.

In one embodiment of the present application, n is 3.

In one embodiment of the present application, n is 2.

In one embodiment of the present application, n is 1.

In one embodiment of the present application, n is 0.

In one embodiment of the present application, when n is 2 or greater, substituents in the parentheses are the same as or different from each other.

In one embodiment of the present application, R1 and R2 are the same as or different from each other, and may be each independently a cyano group; a substituted or unsubstituted aryl group; a substituted or unsubstituted heteroaryl group; or a substituted or unsubstituted phosphine oxide group.

In another embodiment, R1 and R2 are the same as or different from each other, and may be each independently a substituted or unsubstituted aryl group having 6 to 40 carbon atoms; a substituted or unsubstituted heteroaryl group having 2 to 40 carbon atoms; or a substituted or unsubstituted phosphine oxide group.

In another embodiment, R1 and R2 are the same as or different from each other, and may be each independently a cyano group; a substituted or unsubstituted phenyl group; a substituted or unsubstituted biphenyl group; a substituted or unsubstituted naphthyl group; a substituted or unsubstituted triphenylene group; a substituted or unsubstituted dibenzofuran group; a substituted or unsubstituted dibenzothiophene group; a substituted or unsubstituted pyridine group; a substituted or unsubstituted pyrimidine group; a substituted or unsubstituted triazine group; a substituted or unsubstituted 1,10-phenanthroline group; or a substituted or unsubstituted phosphine oxide group.

In another embodiment, R1 and R2 are the same as or different from each other, and may be each independently a cyano group; a phenyl group; a biphenyl group; a naphthyl group; a triphenylene group; a dibenzofuran group; a dibenzothiophene group; a pyridine group unsubstituted or substituted with one or more substituents selected from the group consisting of a phenyl group, a biphenyl group, a naphthyl group and a pyridine group; a pyrimidine group unsubstituted or substituted with one or more substituents selected from the group consisting of a phenyl group, a biphenyl group and a naphthyl group; a triazine group unsubstituted or substituted with one or more substituents selected from the group consisting of a phenyl group, a biphenyl group and a naphthyl group; a 1,10-phenanthroline group unsubstituted or substituted with a phenyl group; or a phosphine oxide group unsubstituted or substituted with a phenyl group.

In one embodiment of the present application, R1 and R2 may be the same as each other.

In one embodiment of the present application, R1 and R2 may be different from each other.

In another embodiment, R1 may be a cyano group; a substituted or unsubstituted aryl group; a substituted or unsubstituted heteroaryl group; or a substituted or unsubstituted phosphine oxide group.

In another embodiment, R1 may be a substituted or unsubstituted aryl group having 6 to 40 carbon atoms; a substituted or unsubstituted heteroaryl group having 2 to 40 carbon atoms; or a substituted or unsubstituted phosphine oxide group.

In another embodiment, R1 may be a cyano group; a substituted or unsubstituted phenyl group; a substituted or unsubstituted biphenyl group; a substituted or unsubstituted naphthyl group; a substituted or unsubstituted triphenylene group; a substituted or unsubstituted dibenzofuran group; a substituted or unsubstituted dibenzothiophene group; a substituted or unsubstituted pyridine group; a substituted or unsubstituted pyrimidine group; a substituted or unsubstituted triazine group; a substituted or unsubstituted 1,10-phenanthroline group; or a substituted or unsubstituted phosphine oxide group.

In another embodiment, R1 may be a cyano group; a phenyl group; a biphenyl group; a naphthyl group; a triphenylene group; a dibenzofuran group; a dibenzothiophene group; a pyridine group unsubstituted or substituted with one or more substituents selected from the group consisting of a phenyl group, a biphenyl group, a naphthyl group and a pyridine group; a pyrimidine group unsubstituted or substituted with one or more substituents selected from the group consisting of a phenyl group, a biphenyl group and a naphthyl group; a triazine group unsubstituted or substituted with one or more substituents selected from the group consisting of a phenyl group, a biphenyl group and a naphthyl group; a 1,10-phenanthroline group unsubstituted or substituted with a phenyl group; or a phosphine oxide group unsubstituted or substituted with a phenyl group.

In another embodiment, R1 is a cyano group.

In another embodiment, R1 is a phenyl group.

In another embodiment, R1 is a biphenyl group.

In another embodiment, R1 is a naphthyl group.

In another embodiment, R1 is a triphenylene group.

In another embodiment, R1 is a dibenzofuran group.

In another embodiment, R1 is a pyridine group unsubstituted or substituted with one or more substituents selected from the group consisting of a phenyl group, a biphenyl group, a naphthyl group and a pyridine group.

In another embodiment, R1 is a triazine group unsubstituted or substituted with one or more substituents selected from the group consisting of a phenyl group, a biphenyl group and a naphthyl group.

In another embodiment, R1 is a 1,10-phenanthroline group unsubstituted or substituted with a phenyl group.

In another embodiment, R1 is a phosphine oxide group unsubstituted or substituted with a phenyl group.

In another embodiment, R2 may be a cyano group; a substituted or unsubstituted aryl group; a substituted or unsubstituted heteroaryl group; or a substituted or unsubstituted phosphine oxide group.

In another embodiment, R2 may be a substituted or unsubstituted aryl group having 6 to 40 carbon atoms; a substituted or unsubstituted heteroaryl group having 2 to 40 carbon atoms; or a substituted or unsubstituted phosphine oxide group.

In another embodiment, R2 may be a cyano group; a substituted or unsubstituted phenyl group; a substituted or unsubstituted biphenyl group; a substituted or unsubstituted naphthyl group; a substituted or unsubstituted triphenylene group; a substituted or unsubstituted dibenzofuran group; a substituted or unsubstituted dibenzothiophene group; a substituted or unsubstituted pyridine group; a substituted or unsubstituted pyrimidine group; a substituted or unsubstituted triazine group; a substituted or unsubstituted 1,10-phenanthroline group; or a substituted or unsubstituted phosphine oxide group.

In another embodiment, R2 may be a cyano group; a phenyl group; a biphenyl group; a naphthyl group; a triphenylene group; a dibenzofuran group; a dibenzothiophene group; a pyridine group unsubstituted or substituted with one or more substituents selected from the group consisting of a phenyl group, a biphenyl group, a naphthyl group and a pyridine group; a pyrimidine group unsubstituted or substituted with one or more substituents selected from the group consisting of a phenyl group, a biphenyl group and a naphthyl group; a triazine group unsubstituted or substituted with one or more substituents selected from the group consisting of a phenyl group, a biphenyl group and a naphthyl group; a 1,10-phenanthroline group unsubstituted or substituted with a phenyl group; or a phosphine oxide group unsubstituted or substituted with a phenyl group.

In another embodiment, R2 is a cyano group.

In another embodiment, R2 is a phenyl group.

In another embodiment, R2 is a biphenyl group.

In another embodiment, R2 is a naphthyl group.

In another embodiment, R2 is a triphenylene group.

In another embodiment, R2 is a dibenzofuran group.

In another embodiment, R2 is a pyridine group unsubstituted or substituted with one or more substituents selected from the group consisting of a phenyl group, a biphenyl group, a naphthyl group and a pyridine group.

In another embodiment, R2 is a triazine group unsubstituted or substituted with one or more substituents selected from the group consisting of a phenyl group, a biphenyl group and a naphthyl group.

In another embodiment, R2 is a 1,10-phenanthroline group unsubstituted or substituted with a phenyl group.

In another embodiment, R2 is a phosphine oxide group unsubstituted or substituted with a phenyl group.

In one embodiment of the present application, at least one of R1 and R2 may comprise a substituted or unsubstituted N-containing heteroaryl group.

In one embodiment of the present application, the substituted or unsubstituted N-containing heteroaryl group may be a heteroaryl group substituted or unsubstituted, and comprising one or more =N- bonds.

In another embodiment, the substituted or unsubstituted N-containing heteroaryl group may be a heteroaryl group substituted or unsubstituted, and comprising one or more and five or less =N- bonds.

In another embodiment, the substituted or unsubstituted N-containing heteroaryl group may be a heteroaryl group substituted or unsubstituted, and comprising one or more and three or less =N- bonds.

In one embodiment of the present application, having one or more =N- bonds means containing an N atom in a compound, and the N atom being linked through a double bond and a single bond. Specific examples thereof may comprise a pyridine group, a pyrimidine group, a triazine group, a benzimidazole group, a phenanthroline group and the like, but are not limited thereto.

In one embodiment of the present application, the substituted or unsubstituted N-containing heteroaryl group may be a substituted or unsubstituted pyridine group; a substituted or unsubstituted pyrimidine group; a substituted or unsubstituted triazine group; or a substituted or unsubstituted 1,10-phenanthroline group.

In another embodiment, the substituted or unsubstituted N-containing heteroaryl group may be a pyridine group unsubstituted or substituted with one or more substituents selected from the group consisting of a phenyl group, a biphenyl group, a naphthyl group and a pyridine group; a pyrimidine group unsubstituted or substituted with one or more substituents selected from the group consisting of a phenyl group, a biphenyl group and a naphthyl group; a triazine group unsubstituted or substituted with one or more substituents selected from the group consisting of a phenyl group, a biphenyl group and a naphthyl group; or a 1,10-phenanthroline group unsubstituted or substituted with a phenyl group.

In one embodiment of the present application, the substituted or unsubstituted N-containing heteroaryl group may be represented by any one of the following Chemical Formula A1 to Chemical Formula A30.

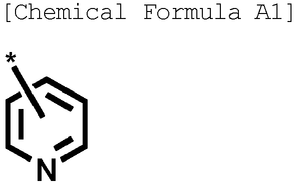

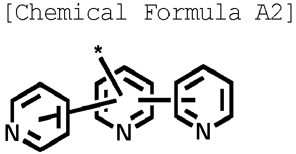

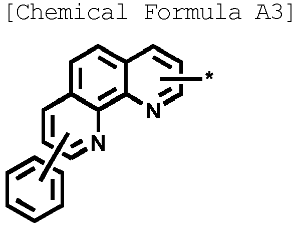

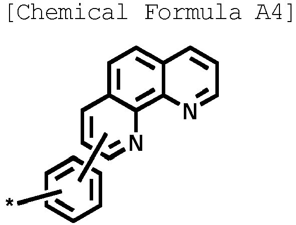

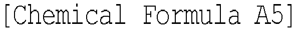

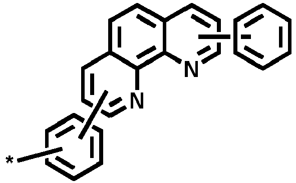

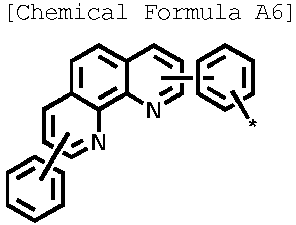

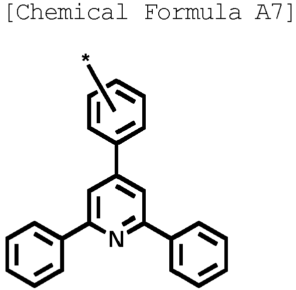

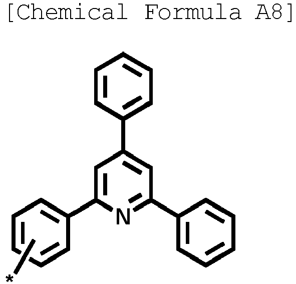

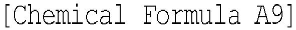

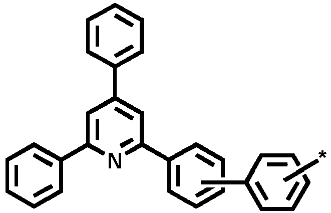

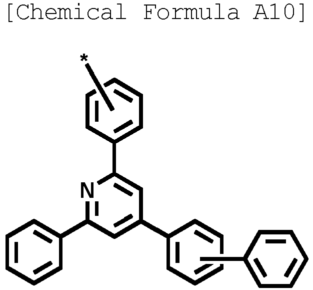

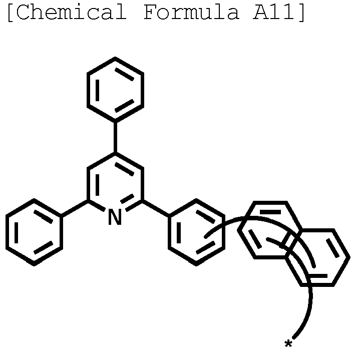

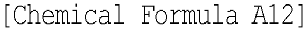

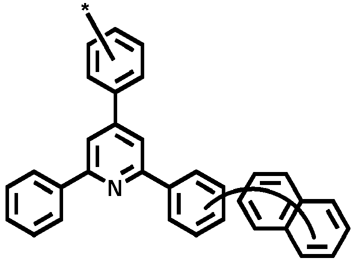

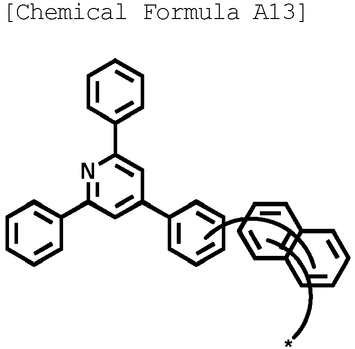

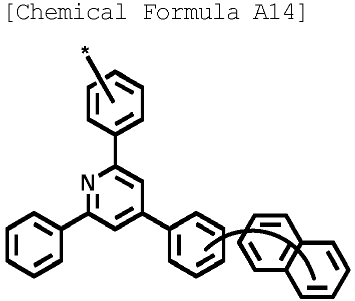

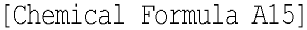

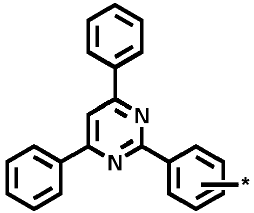

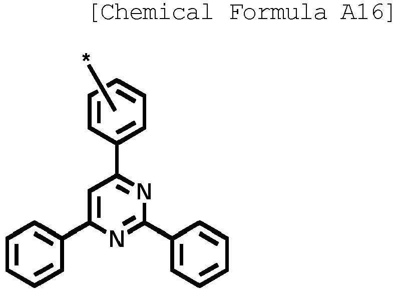

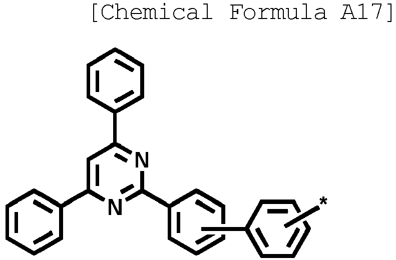

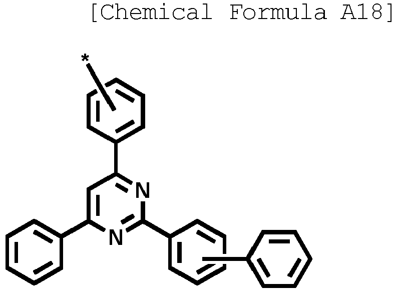

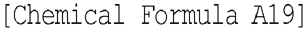

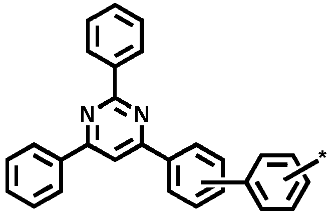

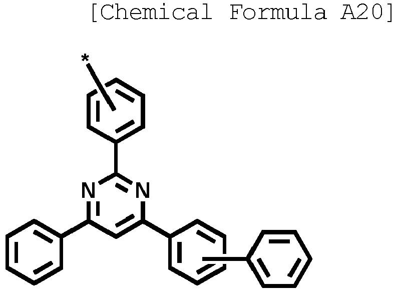

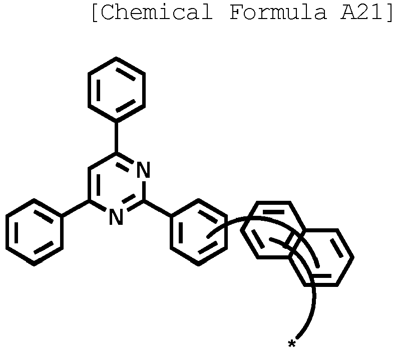

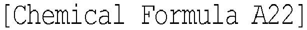

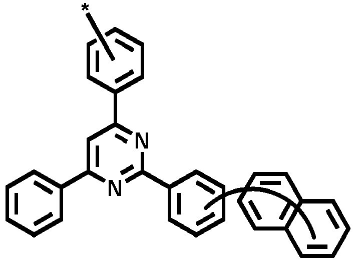

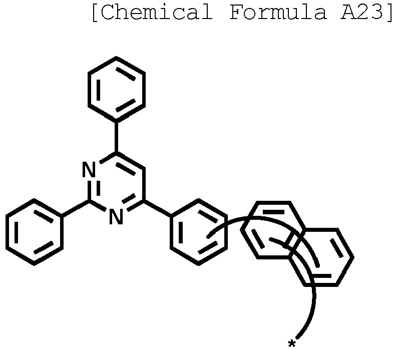

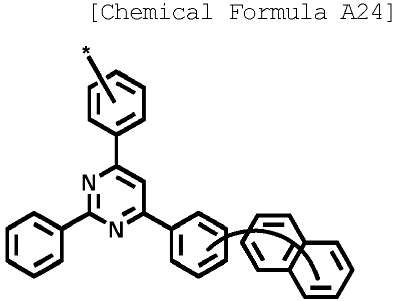

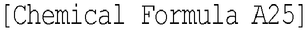

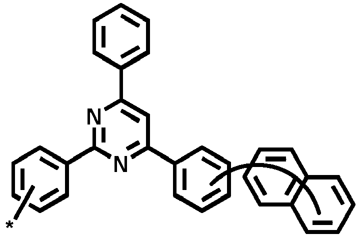

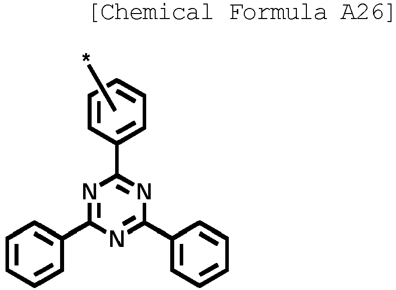

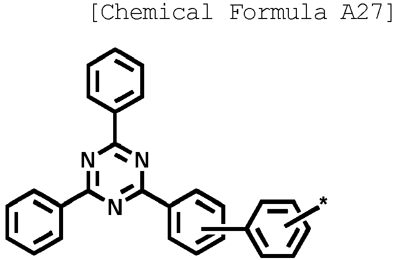

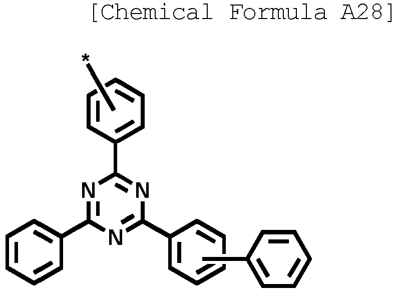

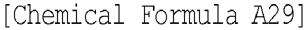

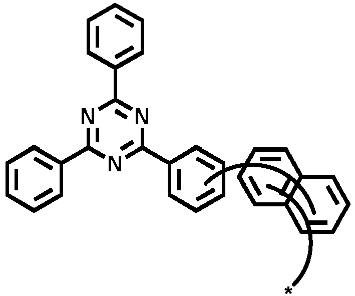

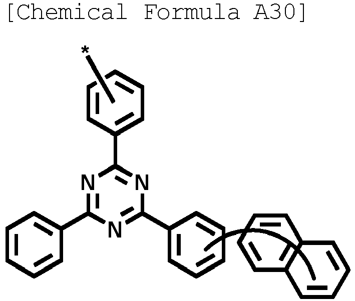

In Chemical Formula A1 to Chemical Formula A30,
* means a position each bonding to L1 or L2.
In one embodiment of the present application, only R1 may comprise a substituted or unsubstituted N-containing heteroaryl group. Specifically, R1 comprises a substituted or unsubstituted N-containing heteroaryl group, and R2 may be a cyano group; a substituted or unsubstituted phenyl group; a substituted or unsubstituted biphenyl group; a substituted or unsubstituted naphthyl group; a substituted or unsubstituted triphenylene group; a substituted or unsubstituted dibenzofuran group; a substituted or unsubstituted dibenzothiophene group; or a substituted or unsubstituted phosphine oxide group.

In one embodiment of the present application, only R2 may comprise a substituted or unsubstituted N-containing heteroaryl group. Specifically, R2 comprises a substituted or unsubstituted N-containing heteroaryl group, and R1 may be a cyano group; a substituted or unsubstituted phenyl group; a substituted or unsubstituted biphenyl group; a substituted or unsubstituted naphthyl group; a substituted or unsubstituted triphenylene group; a substituted or unsubstituted dibenzofuran group; a substituted or unsubstituted dibenzothiophene group; or a substituted or unsubstituted phosphine oxide group.

In one embodiment of the present application, R1 and R2 are the same as or different from each other, and may each independently comprise a substituted or unsubstituted N-containing heteroaryl group.

In the heterocyclic compound provided in one embodiment of the present application, Chemical Formula 1 is represented by any one of the following compounds.

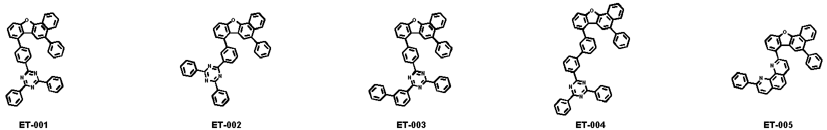

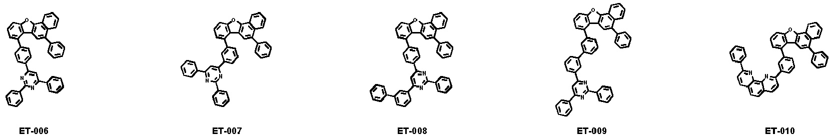

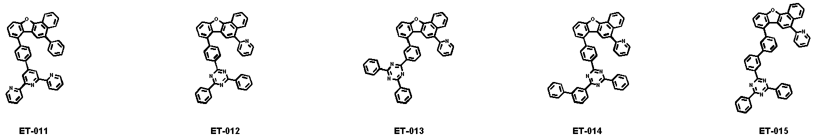

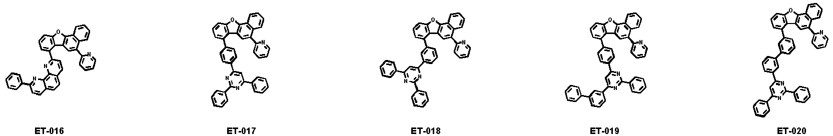

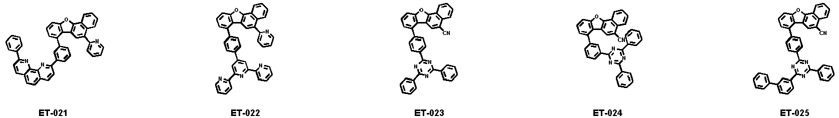

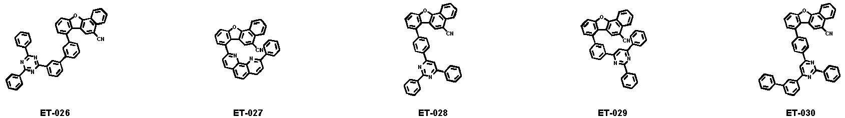

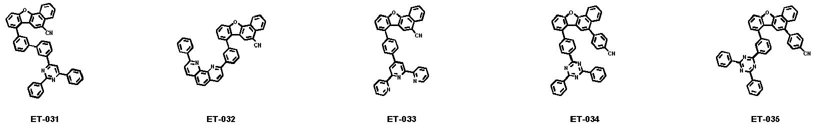

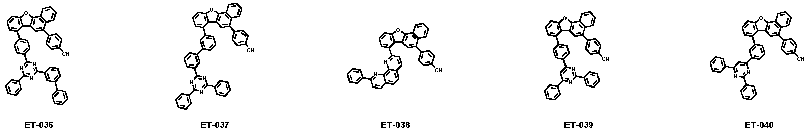

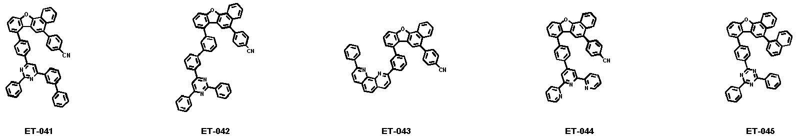

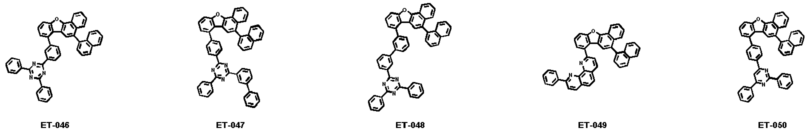

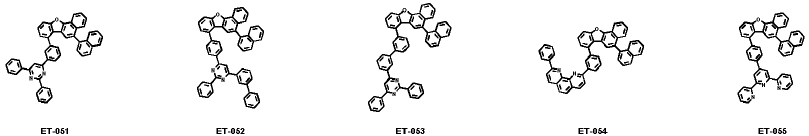

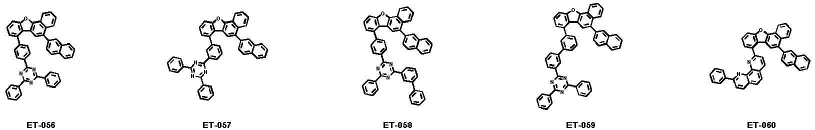

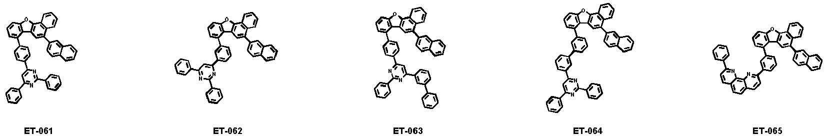

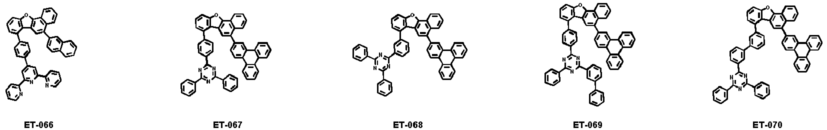

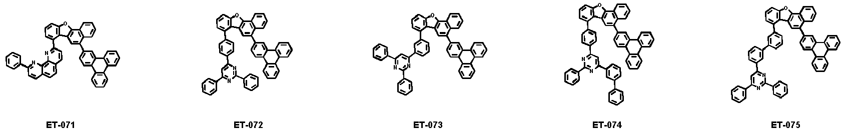

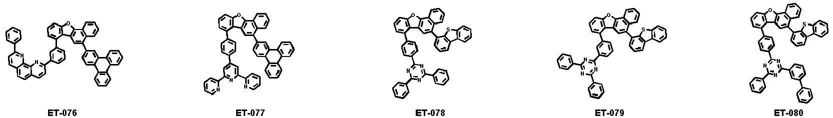

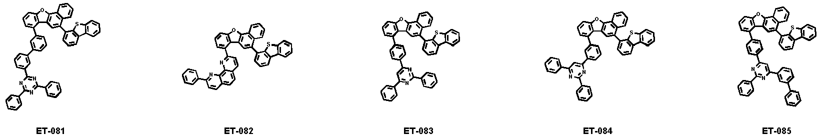

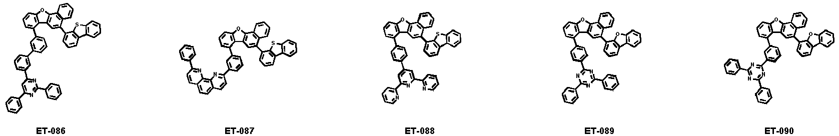

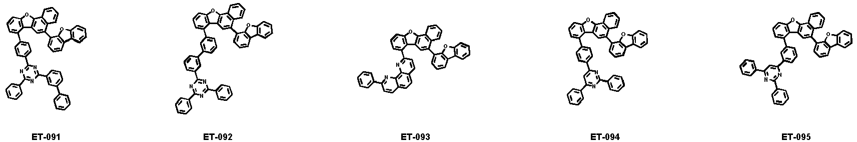

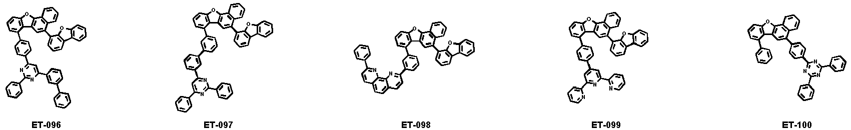

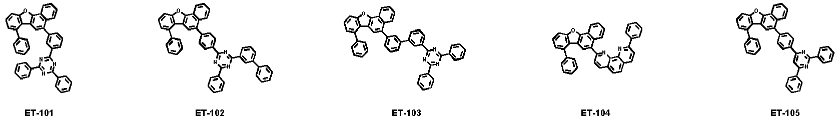

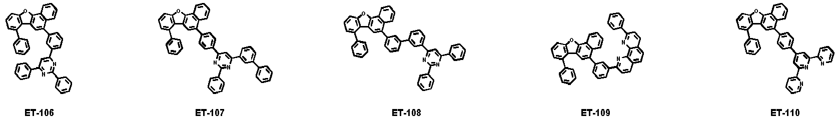

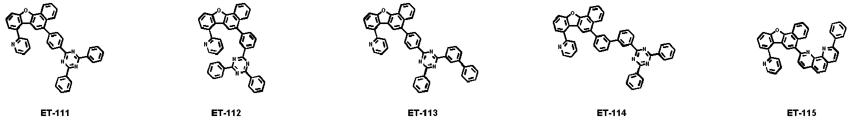

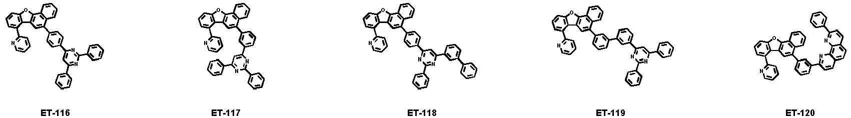

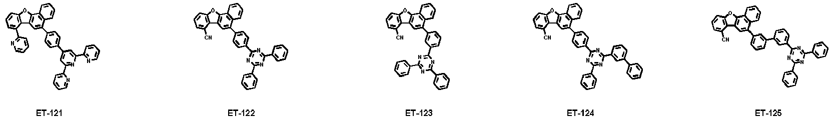

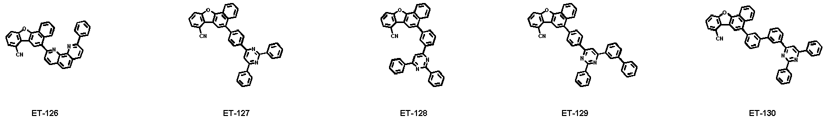

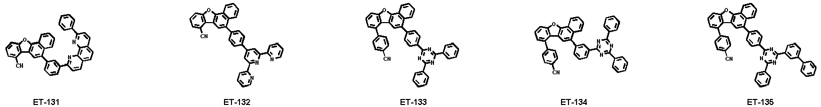

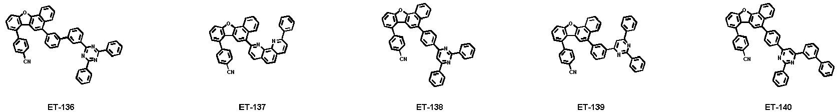

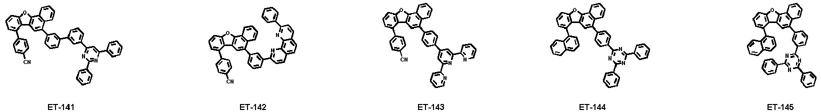

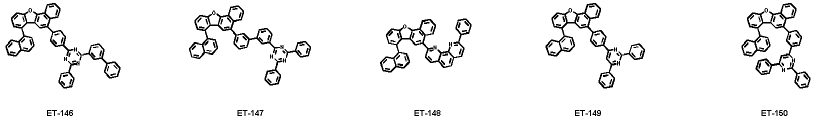

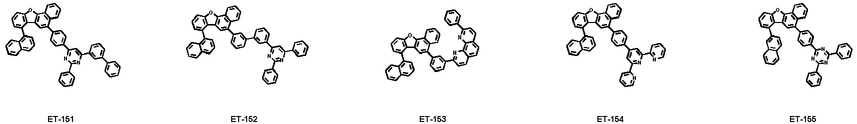

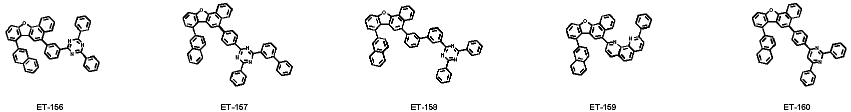

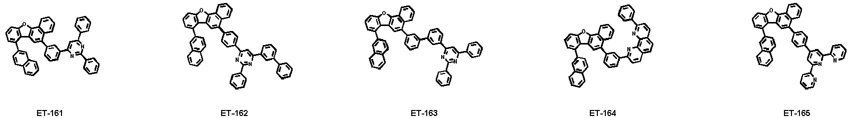

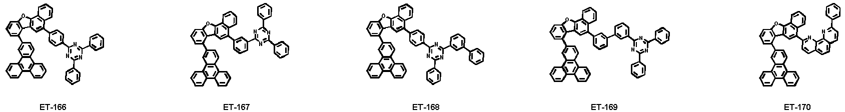

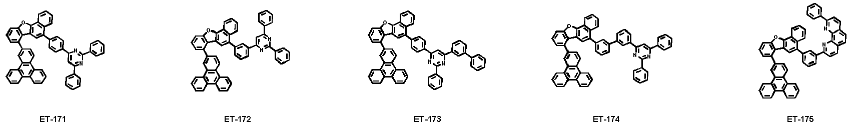

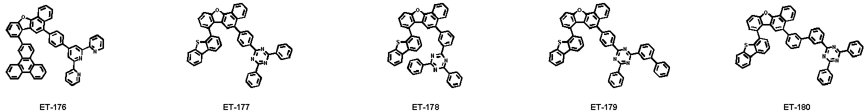

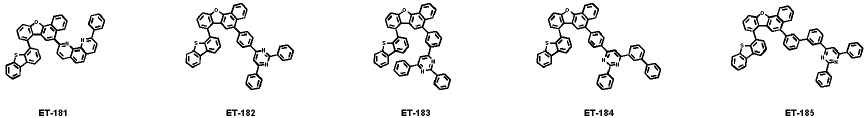

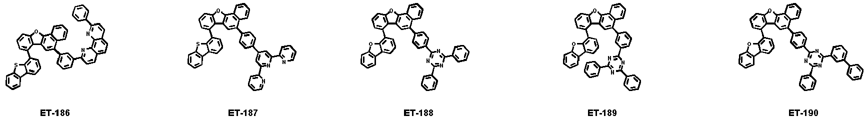

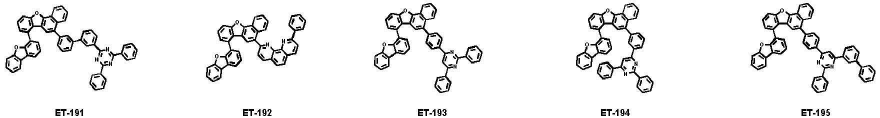

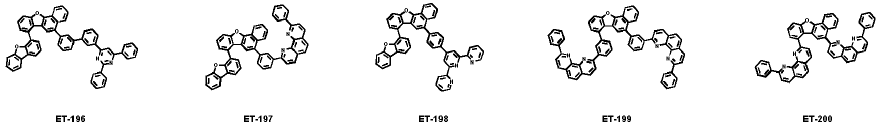

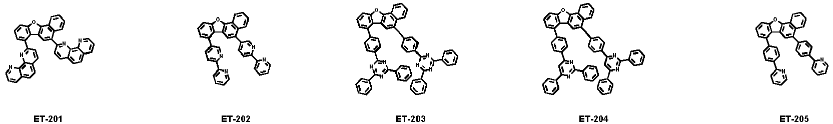

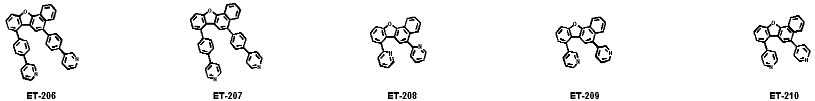

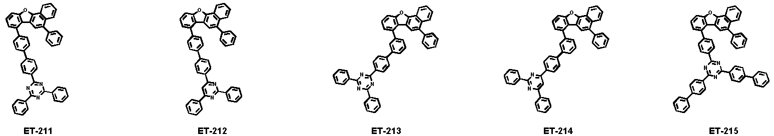

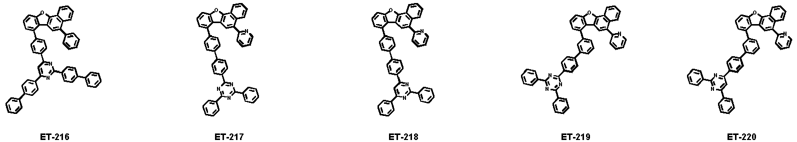

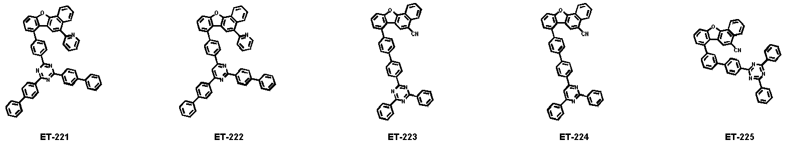

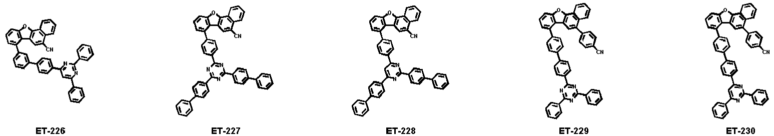

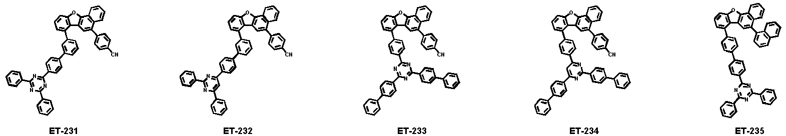

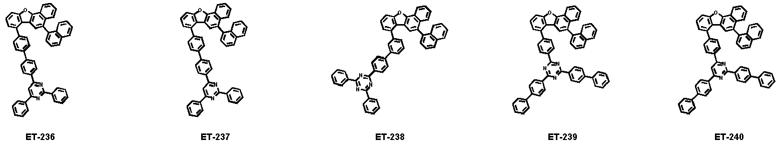

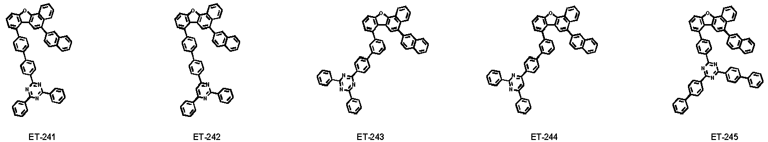

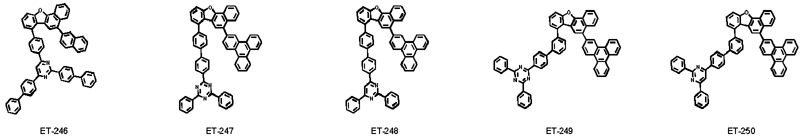

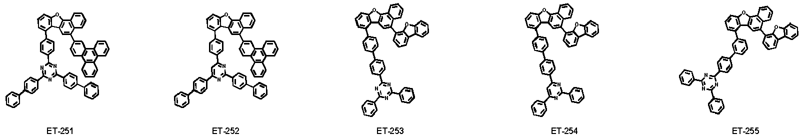

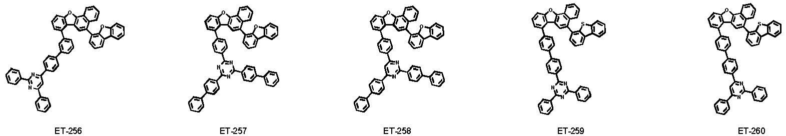

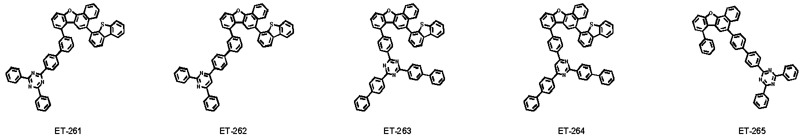

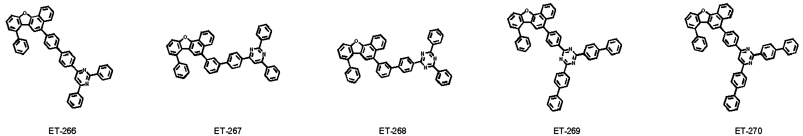

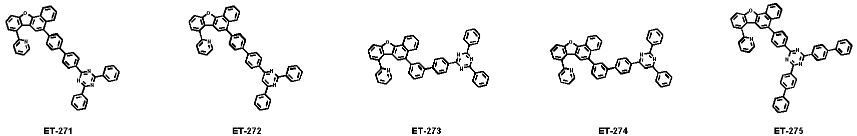

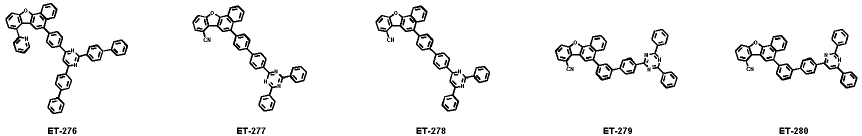

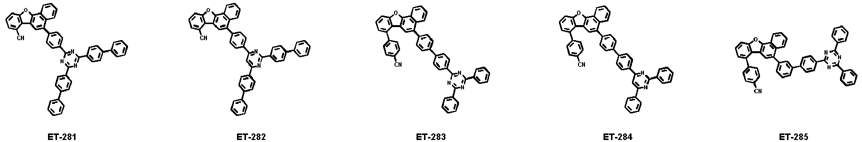

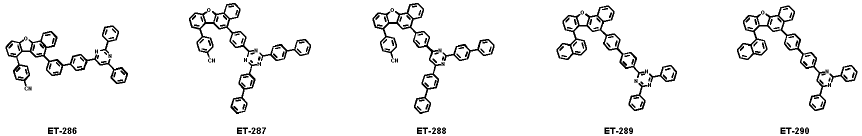

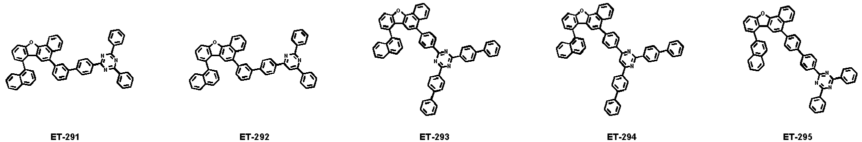

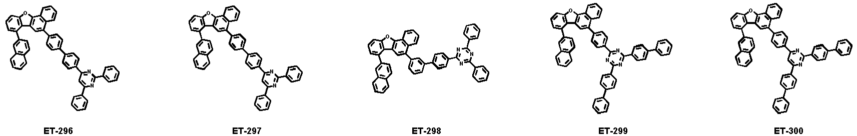

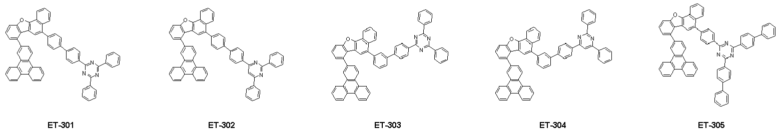

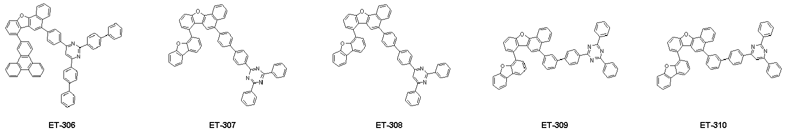

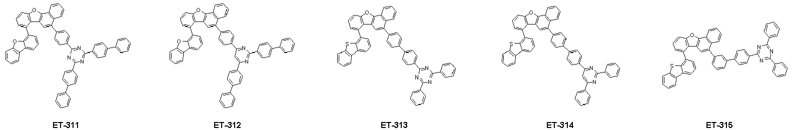

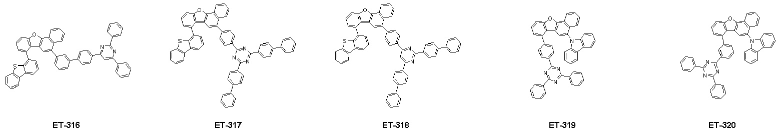

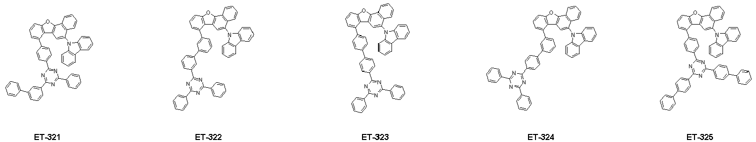

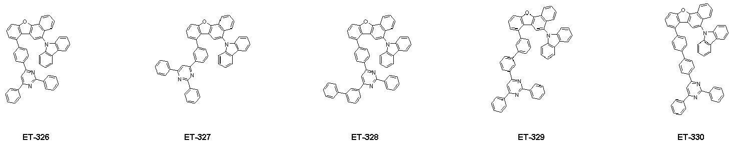

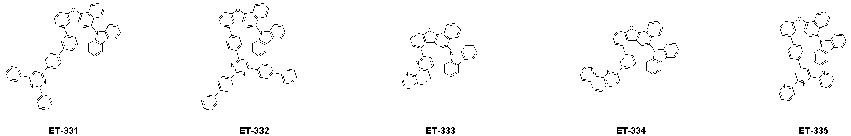

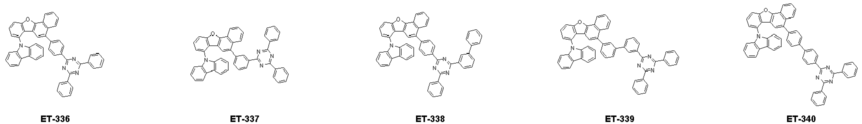

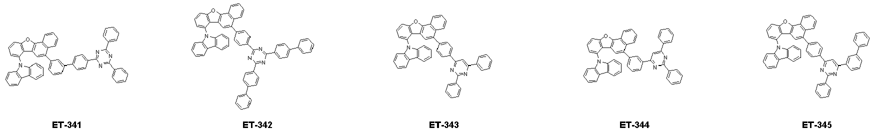

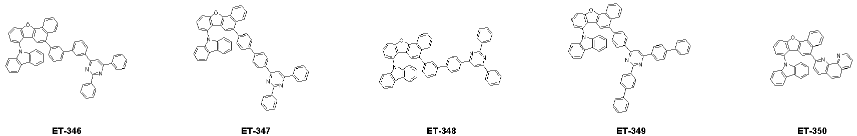

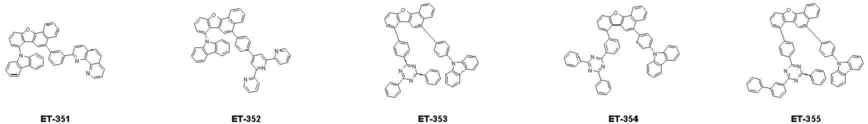

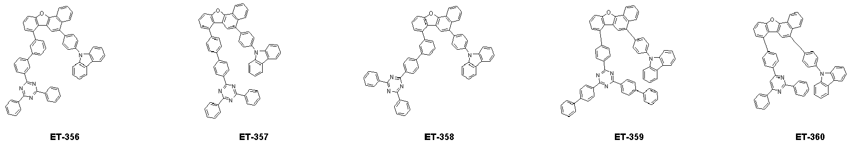

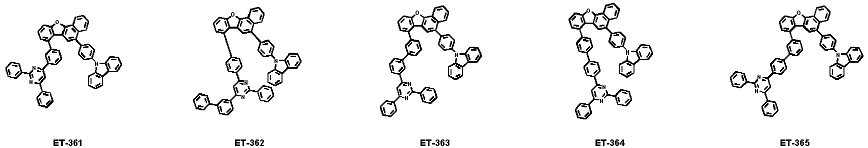

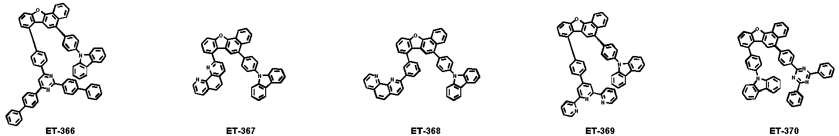

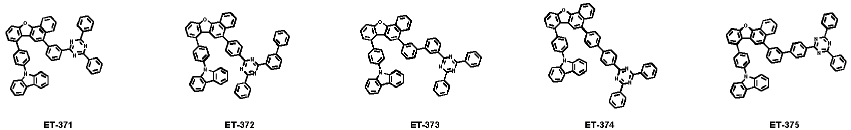

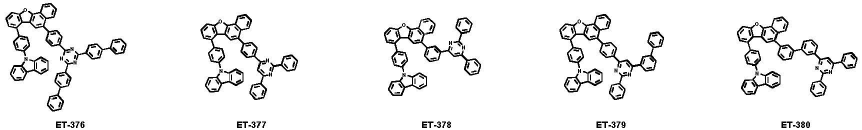

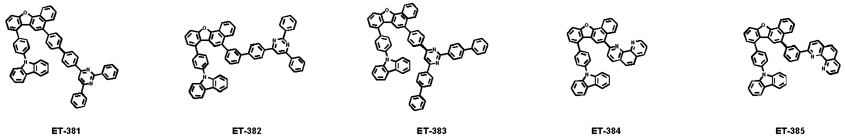

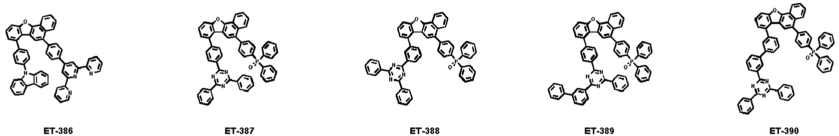

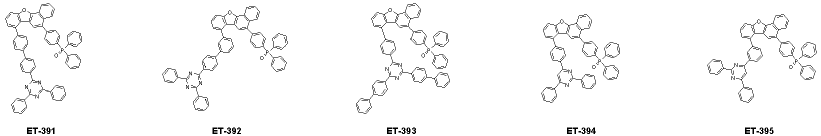

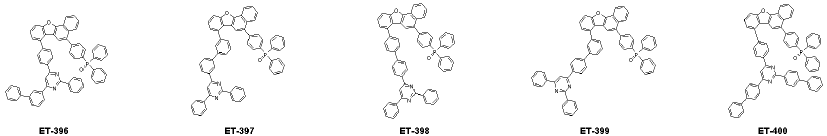

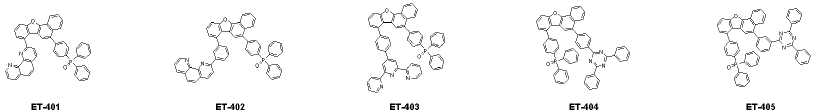

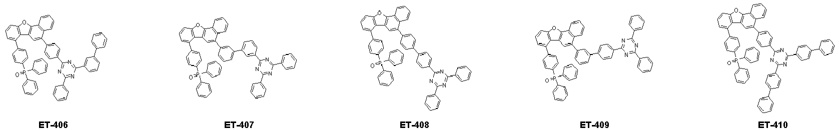

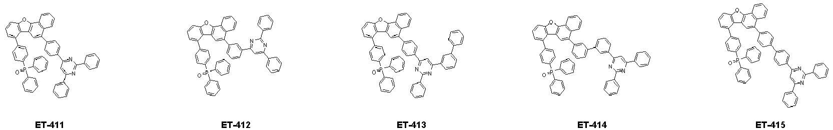

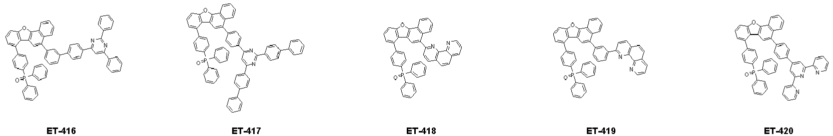

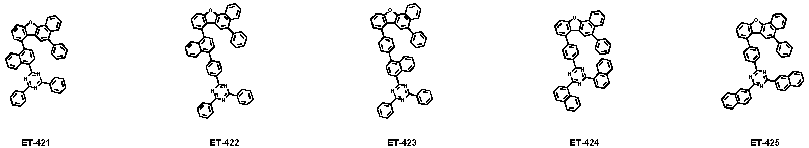

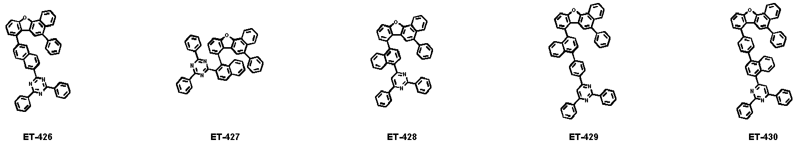

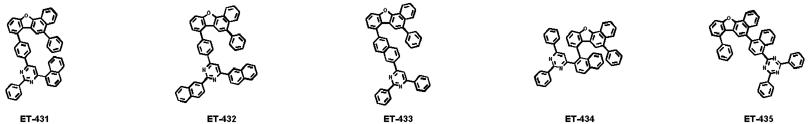

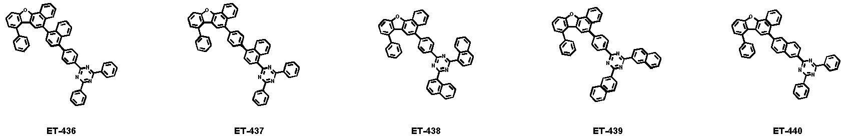

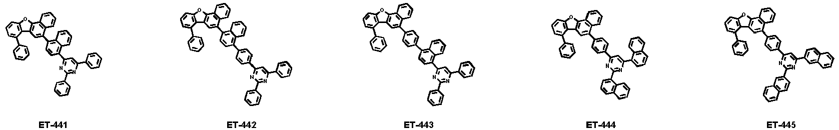

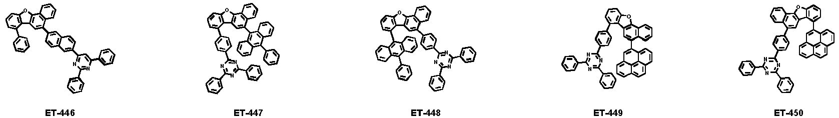

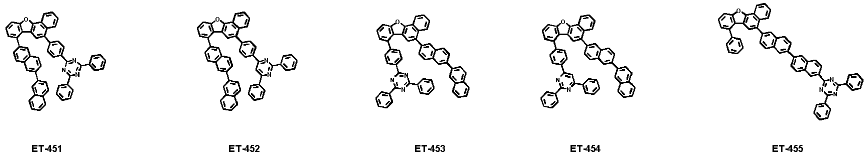

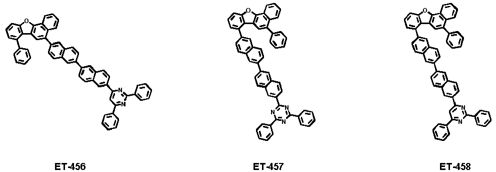

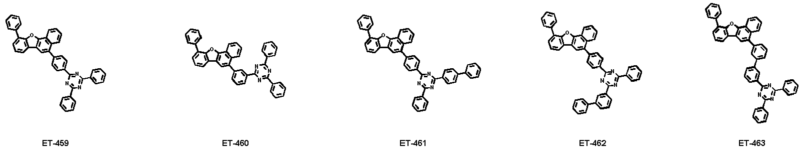

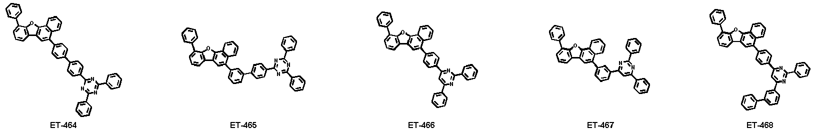

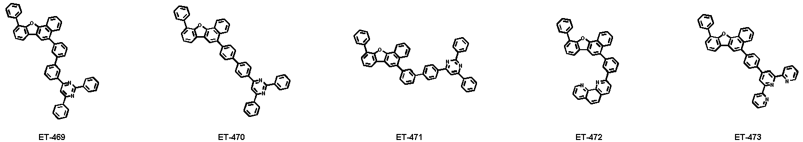

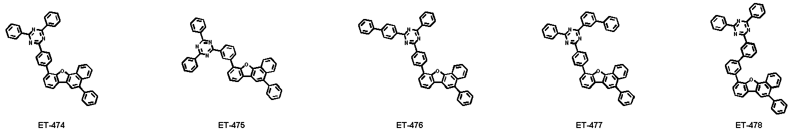

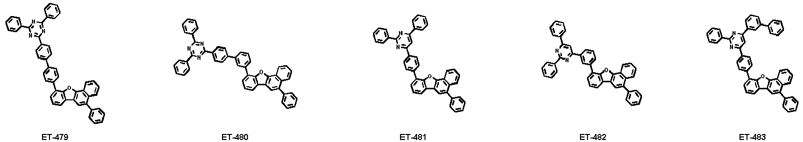

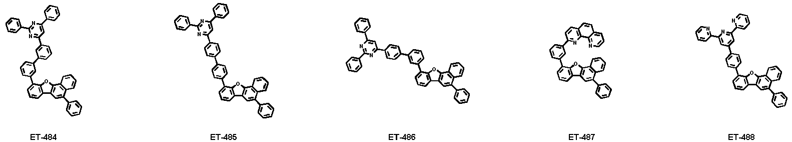

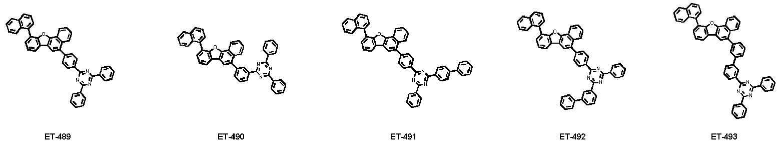

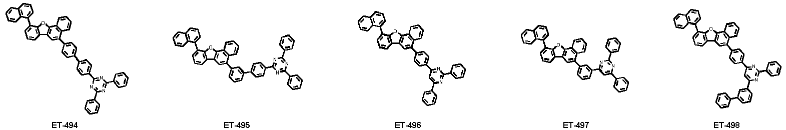

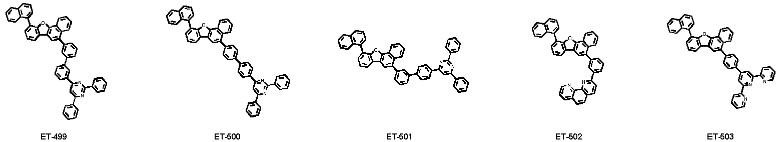

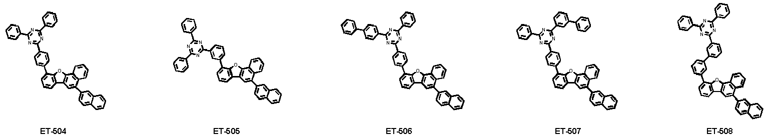

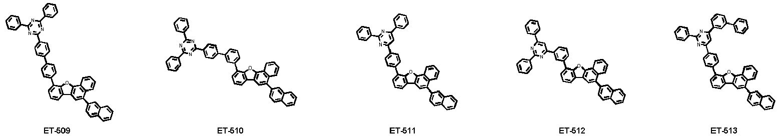

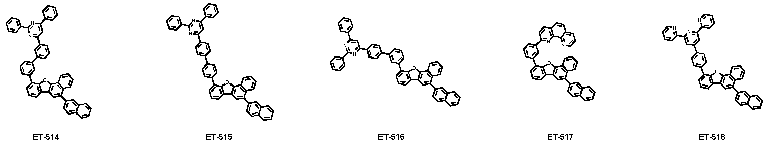

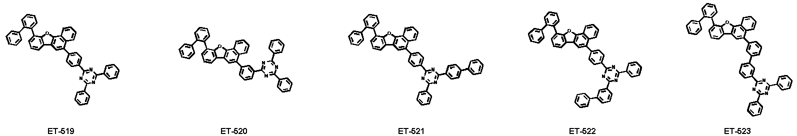

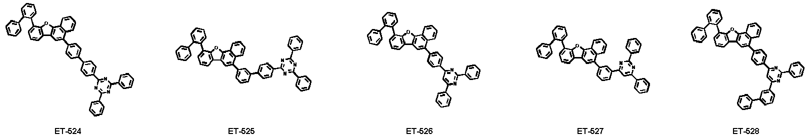

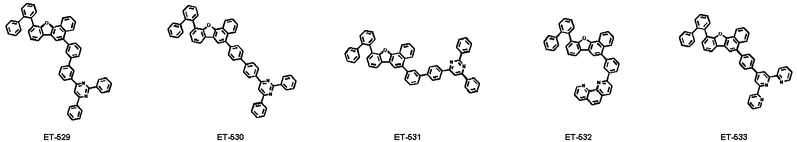

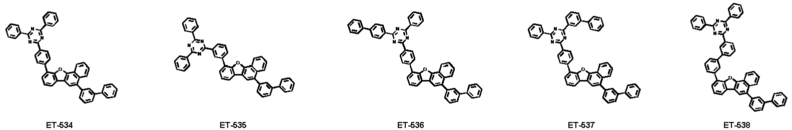

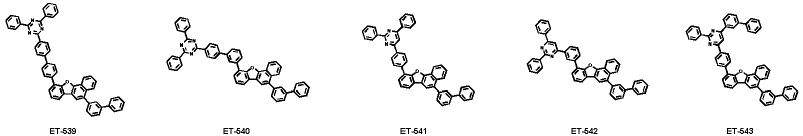

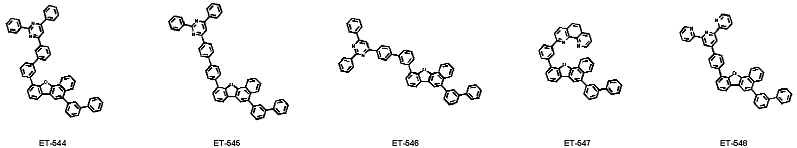

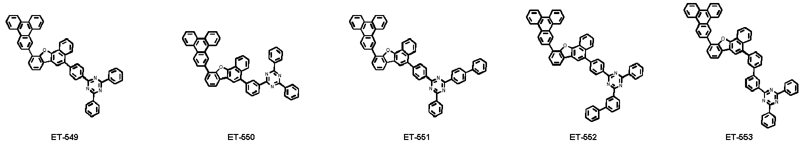

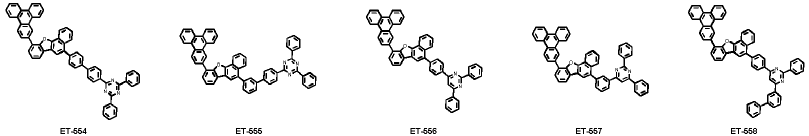

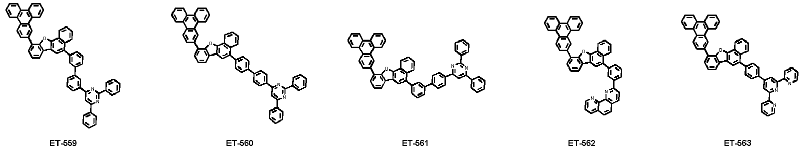

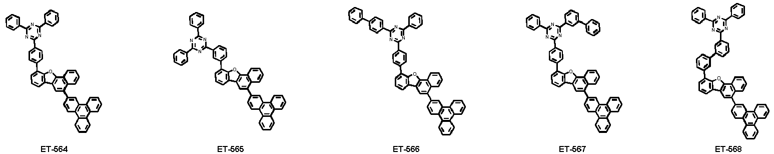

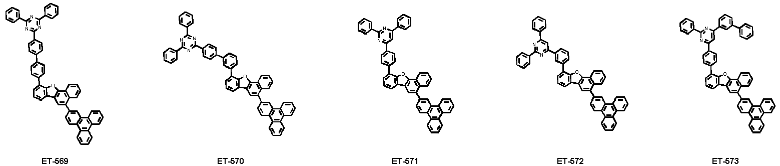

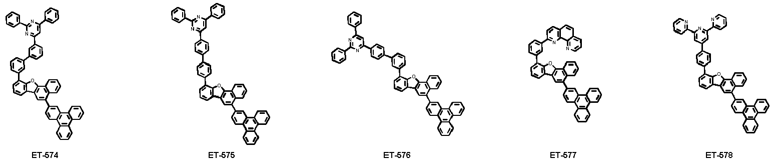

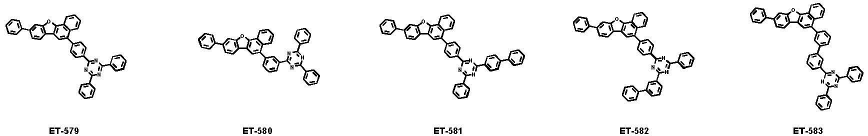

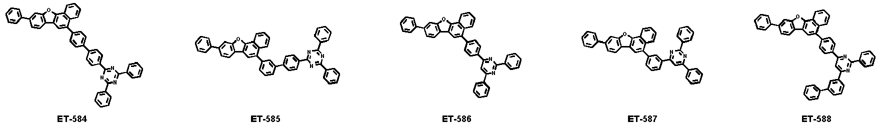

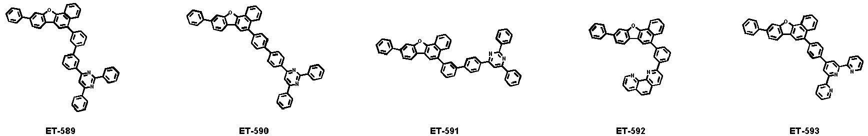

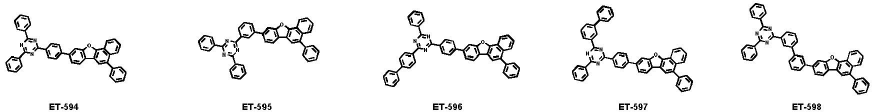

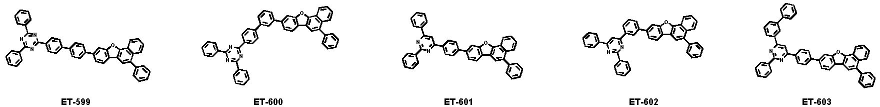

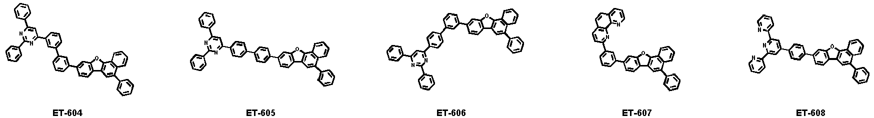

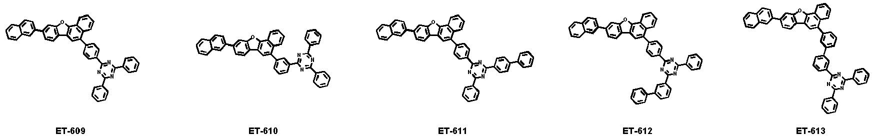

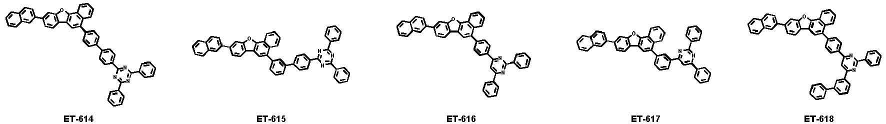

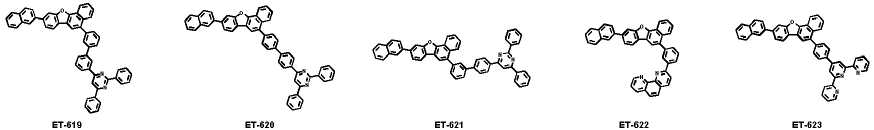

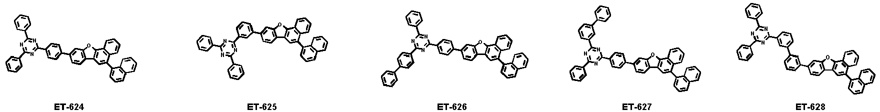

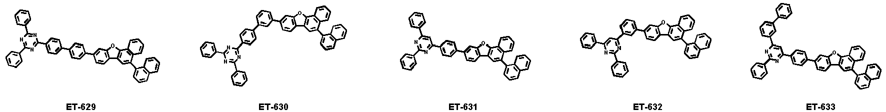

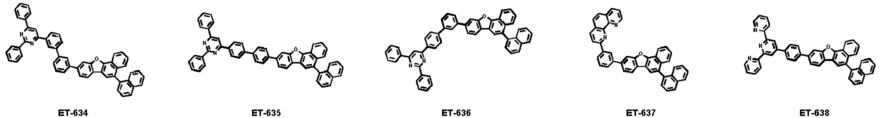

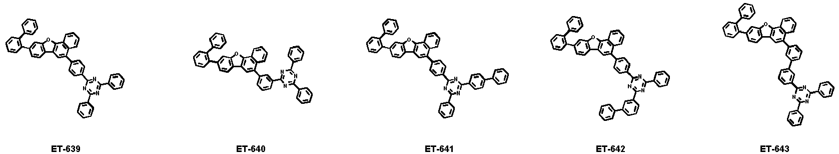

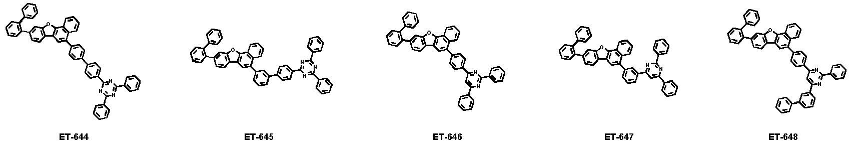

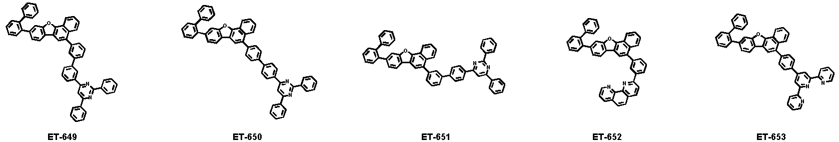

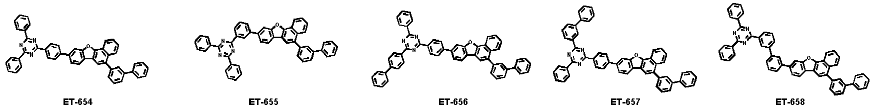

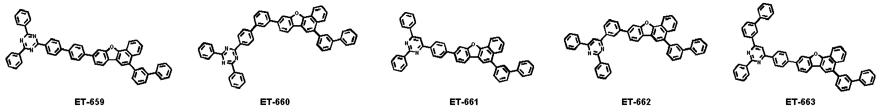

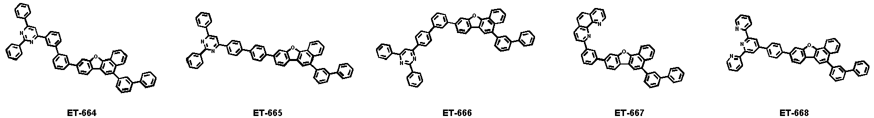

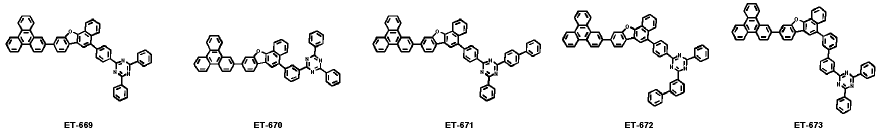

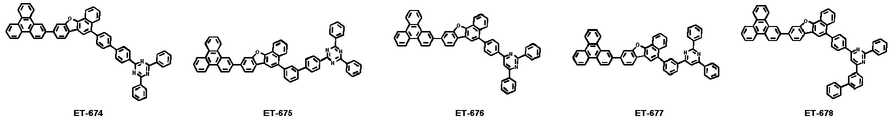

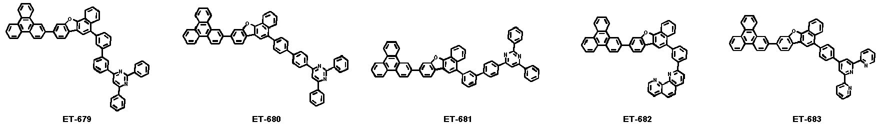

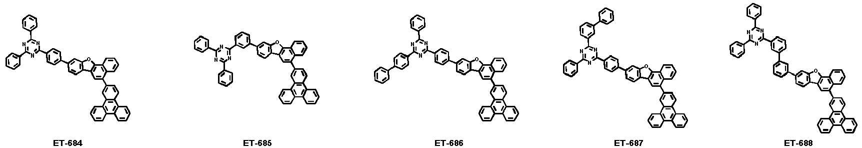

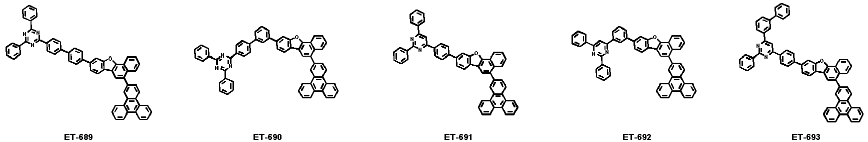

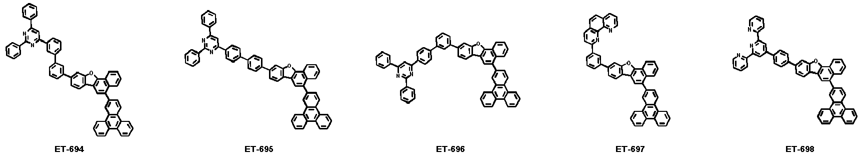

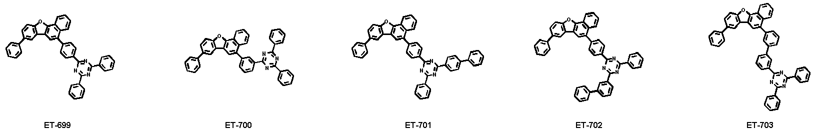

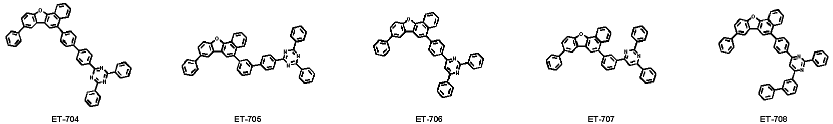

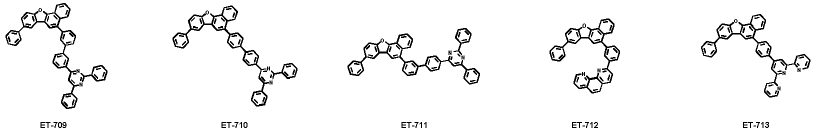

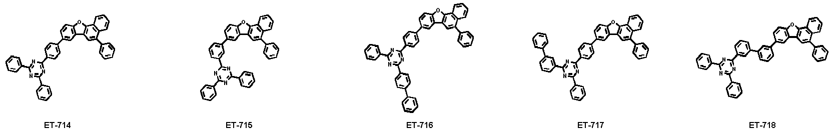

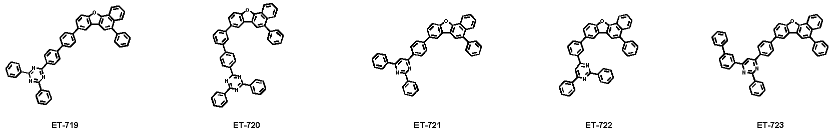

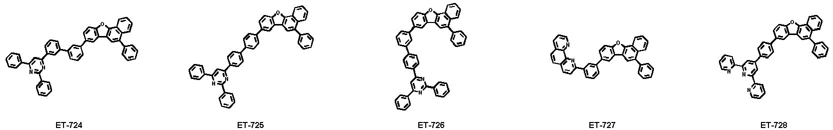

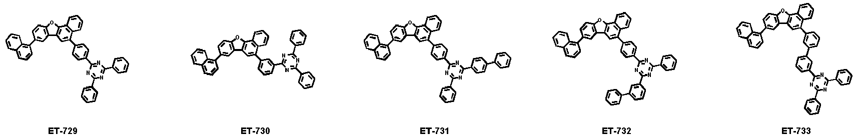

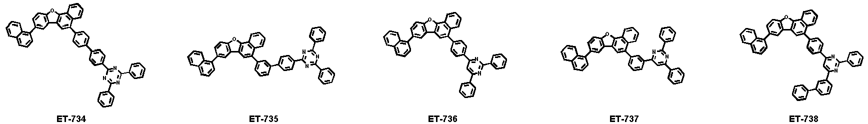

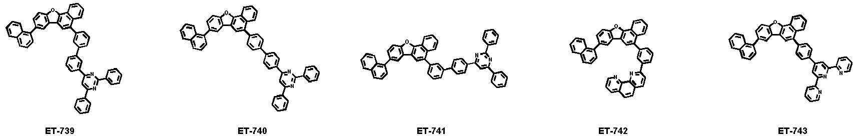

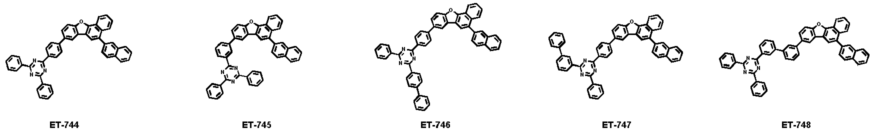

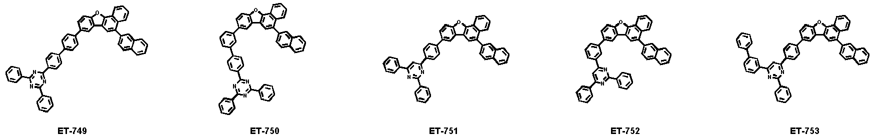

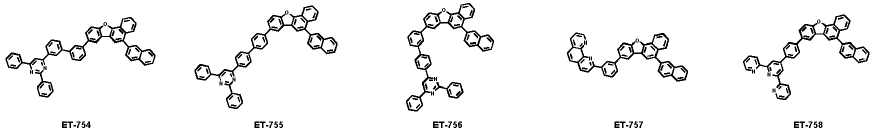

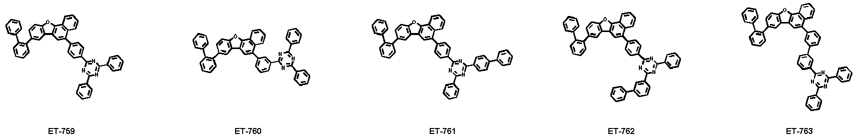

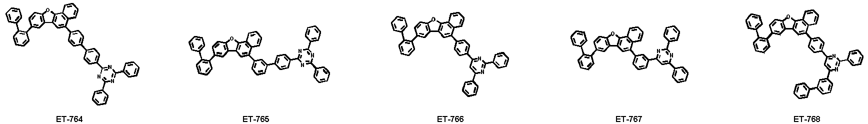

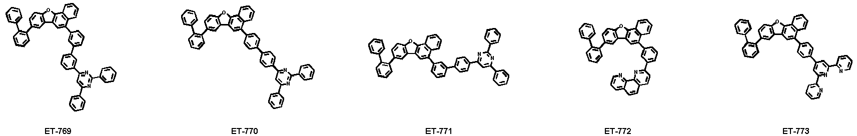

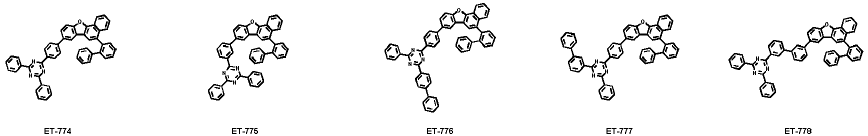

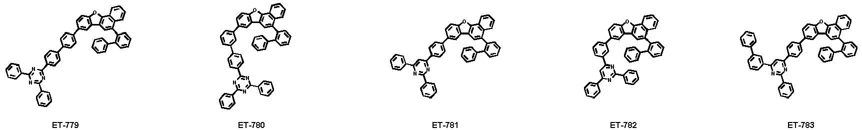

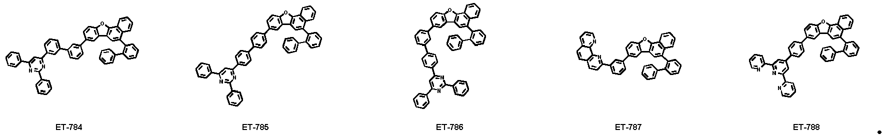

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the heterocyclic compound has a high glass transition temperature (Tg) and thereby has superior thermal stability. Such an increase in the thermal stability becomes an important factor in providing driving stability to a device.

The heterocyclic compound according to one embodiment of the present application may be prepared using a multi-step chemical reaction. Some intermediate compounds are prepared first, and from the intermediate compounds, the compound of Chemical Formula 1 may be prepared. More specifically, the heterocyclic compound according to one embodiment of the present application may be prepared based on preparation examples to describe later.

Another embodiment of the present application provides an organic light emitting device comprising the heterocyclic compound represented by Chemical Formula 1. The "organic light emitting device" may be expressed in terms such as an "organic light emitting diode", an "OLED", an "OLED device" and an "organic electroluminescent device".

One embodiment of the present application provides an organic light emitting device comprising a first electrode; a second electrode; and one or more organic material layers provided between the first electrode and the second electrode, wherein one or more layers of the organic material layers comprise the heterocyclic compound represented by Chemical Formula 1.

In one embodiment of the present application, the first electrode may be an anode, and the second electrode may be a cathode.

In another embodiment of the present application, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device.

In another embodiment of the present application, the organic light emitting device may be a green organic light emitting device, and the heterocyclic compound according to Chemical Formula 1 may be used as a material of the green organic light emitting device.

In another embodiment of the present application, the organic light emitting device may be a red organic light emitting device, and the heterocyclic compound according to Chemical Formula 1 may be used as a material of the red organic light emitting device.

Specific descriptions on the heterocyclic compound represented by Chemical Formula 1 are the same as the descriptions provided above.

The organic light emitting device of the present application may be manufactured using common organic light emitting device manufacturing methods and materials except that one or more of the organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present application may be formed in a single layer structure, but may be formed in a multilayer structure in which two or more organic material layers are laminated. For example, the organic light emitting device of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present application, the organic material layer comprises an electron transfer layer, and the electron transfer layer may comprise the heterocyclic compound. When using the heterocyclic compound in an electron transfer layer, electrons are efficiently transferred without decomposing or destroying the compound, and the organic light emitting device may have superior driving, efficiency and lifetime.

In another organic light emitting device, the organic material layer comprises a hole blocking layer, and the hole blocking layer may comprise the heterocyclic compound. When using the heterocyclic compound in a hole blocking layer, the organic light emitting device may have superior driving, efficiency and lifetime.

The organic light emitting device of the present disclosure may further comprise one, two or more layers selected from the group consisting of a light emitting layer, a hole injection layer, a hole transfer layer. an electron injection layer, an electron transfer layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

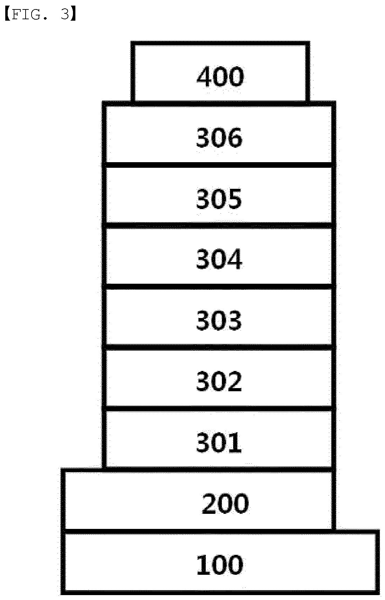

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further included.

The organic material layer comprising the heterocyclic compound represented by Chemical Formula 1 may further comprise other materials as necessary.

In addition, the organic light emitting device according to one embodiment of the present application comprises a first electrode, a first stack provided on the first electrode and comprising a first light emitting layer, a charge generation layer provided on the first stack, a second stack provided on the charge generation layer and comprising a second light emitting layer, and a second electrode provided on the second stack.

In addition, the organic light emitting device according to one embodiment of the present application comprises a first stack provided on the first electrode and comprising a first light emitting layer, a charge generation layer provided on the first stack, a second stack provided on the charge generation layer and comprising a second light emitting layer, and a second electrode provided on the second stack.

Herein, the charge generation layer may comprise the heterocyclic compound represented by Chemical Formula 1. When using the heterocyclic compound in a charge generation layer, the organic light emitting device may have superior driving, efficiency and lifetime.

In addition, the first stack and the second stack may each independently further comprise one or more types of the hole injection layer, the hole transfer layer, the hole blocking layer, the electron transfer layer, the electron injection layer and the like described above.

The charge generation layer may be an N-type charge generation layer, and the charge generation layer may further comprise a dopant known in the art in addition to the heterocyclic compound represented by Chemical Formula 1.

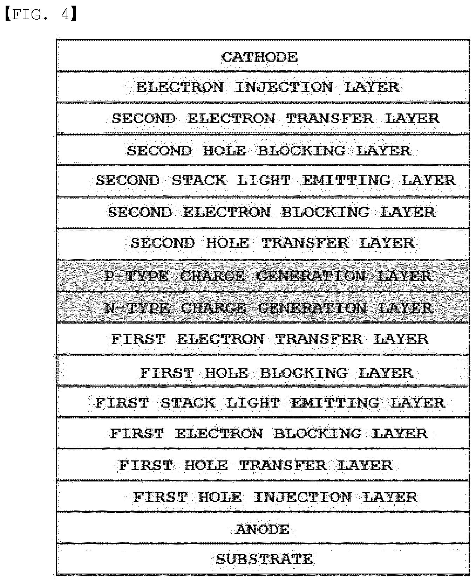

As the organic light emitting device according to one embodiment of the present application, an organic light emitting device having a 2-stack tandem structure is illustrated in FIG. 4.

Herein, the first electron blocking layer, the first hole blocking layer, the second hole blocking layer and the like described in FIG. 4 may not be included in some cases.

In one embodiment of the present application, an alkali metal or an alkaline earth metal may be doped to the compound of Chemical Formula 1. In this case, the organic light emitting device may have a lower driving voltage and more improved efficiency and lifetime due to forming a gap state.

One embodiment of the present application provides a composition comprising an alkali metal or an alkaline earth metal in the compound of Chemical Formula 1. A content of the alkali metal or the alkaline earth metal in the composition may be from 0.01 wt% to 30 wt%, preferably from 0.01 wt% to 25 wt% and more preferably from 0.01 wt% to 20 wt% based on a total weight of the composition.

In one embodiment of the present application, the alkali metal or the alkaline earth metal may be lithium (Li), rubidium (Rb), cesium (Cs) or magnesium (Mg), but is not limited thereto.

In the organic light emitting device according to one embodiment of the present application, materials other than the compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Example>

### <Preparation Example 1> Preparation of Intermediate A

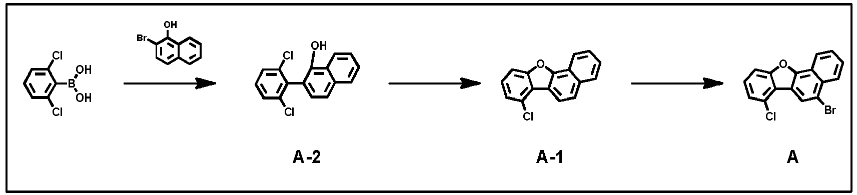

### 1) Preparation of Intermediate A-2

After dissolving (2,6-dichlorophenyl)boronic acid (47.05 g, 246.56 mmol) and 2-bromonaphthalen-1-ol (50 g, 224.14 mmol) in tetrahydrofuran (hereinafter, THF) (500 ml) and H₂O (100 ml), Pd(PPh₃)₄ (7.77 g, 6.72 mmol) and K₂CO₃ (92.94 g, 672.43 mmol) were introduced thereto, and the result was stirred for 17 hours under reflux. After the reaction was completed, methylene chloride (hereinafter MC) was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Intermediate A-2 (49 g, 75%).

### 2) Preparation of Intermediate A-1

After dissolving Intermediate A-2 (49 g, 169.19 mmol) in dimethylacetamide (500 ml), Cs₂CO₃ (165.38 g, 507.58 mmol) was introduced thereto, and the result was stirred for 4 hours under reflux. After the reaction was completed, MC was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Intermediate A-1 (35 g, 82%).

### 3) Preparation of Intermediate A

After dissolving Intermediate A-1 (35 g, 138.50 mmol) in dimethylformamide (hereinafter, DMF) (350 ml), N-bromosuccinimide (hereinafter, NBS) (27.12 g, 152.35 mmol) was introduced thereto, and the result was stirred for 4 hours at room temperature (25°C). After the reaction was completed, MC was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Intermediate A (40 g, 87%).

Target intermediates were prepared in the same manner as in Preparation Example 1 except that Compound A of the following Table 1 was used instead of (2,6-dichlorophenyl)boronic acid.

**[Table 1]**

| Intermediate | Compound A | Target Intermediate | Yield |
|---|---|---|---|
| B | 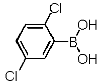 | 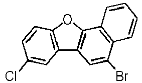 | 80% |
| C | 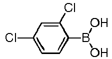 | 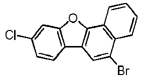 | 78% |
| D | 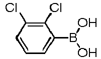 | 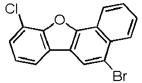 | 81% |

### <Preparation Example 2> Preparation of Compound ET-001

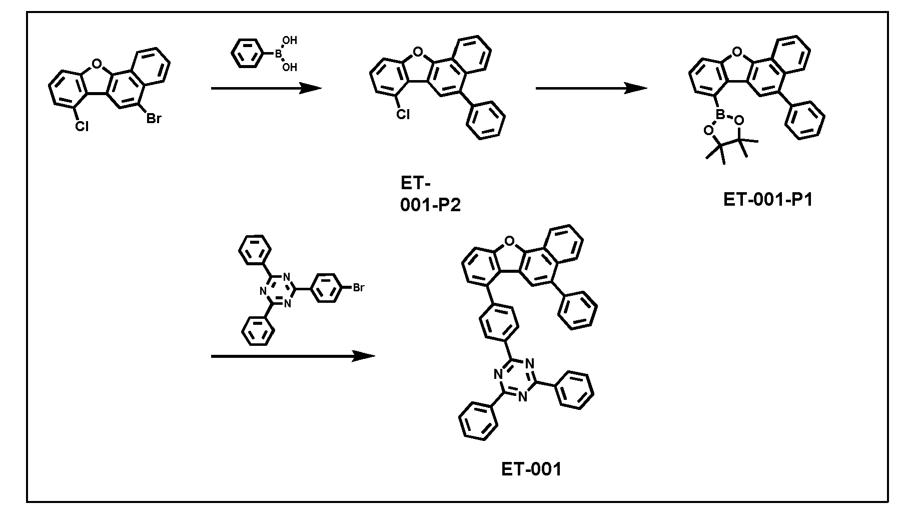

### 1) Preparation of Compound ET-001-P2

After dissolving Intermediate A (20 g, 60.32 mmol) and phenylboronic acid (7.72 g, 63.33 mmol) in toluene (200 ml), ethanol (40 ml) and H₂O (40 ml), Pd(PPh₃)₄ (3.48 g, 3.02 mmol) and K₃PO₄ (38.41 g, 180.95 mmol) were introduced thereto, and the result was stirred for 5 hours under reflux. After the reaction was completed, MC was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Compound ET-001-P2 (17 g, 85%).

### 2) Preparation of Compound ET-001-P1

After dissolving Compound ET-001-P2 (17 g, 51.70 mmol) and bis(pinacolato)diboron (17.07 g, 67.22 mmol) in 1,4-dioxane (170 ml), Pd(dba)₂ (1.49 g, 2.59 mmol), Xphos (2.46 g, 5.17 mmol) and KOAc (15.22 g, 155.11 mmol) were introduced thereto, and the result was stirred for 3 hours under reflux. After the reaction was completed, MC was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Compound ET-001-P1 (18 g, 82%).

### 3) Preparation of Compound ET-001

After dissolving Compound ET-001-P1 (10 g, 23.79 mmol) and 2-(4-bromophenyl)-4,6-diphenyl-1,3,5-triazine (9.24 g, 23.79 mmol) in toluene (100 ml), ethanol (20 ml) and H₂O (20 ml), Pd(PPh₃)₄ (1.37 g, 1.19 mmol) and K₃PO₄ (15.15 g, 71.38 mmol) were introduced thereto, and the result was stirred for 5 hours under reflux. After the reaction was completed, MC was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Compound ET-001 (11 g, 76%).

Target Compounds were prepared in the same manner as in Preparation Example 2 except that Intermediate of the following Table 2 was used instead of Intermediate A, Compound B of the following Table 2 was used instead of phenylboronic acid, and Compound C of the following Table 2 was used instead of 2-(4-bromophenyl)-4,6-diphenyl-1,3,5-triazine.

**[Table 2]**

| **Compo und** | **Intermediat e** | **Compound B** | **Compound C** | **Target Compound** | **Yield** |
|---|---|---|---|---|---|
| **ET-002** | 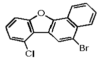 | 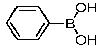 | 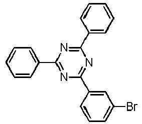 | 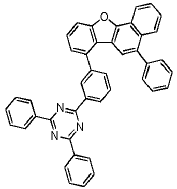 | 74% |
| **ET-003** | 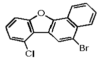 | 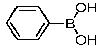 | 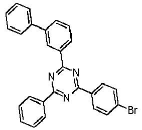 | 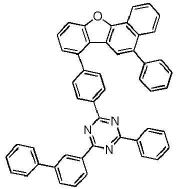 | 72% |
| **ET-004** | 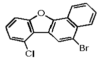 | 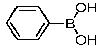 | 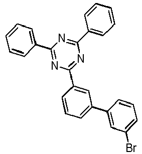 | 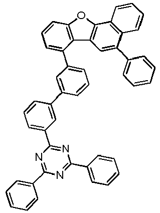 | 81% |
| **ET-005** | 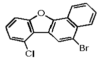 | 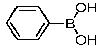 | 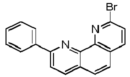 | 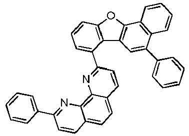 | 77% |
| **ET-006** | 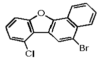 | 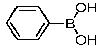 | 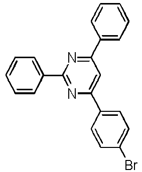 | 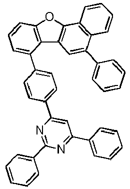 | 70% |
| **ET-007** | 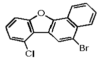 | 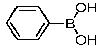 | 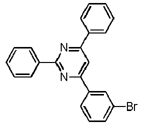 | 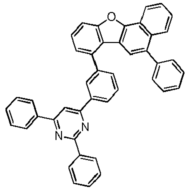 | 67% |
| **ET-008** | 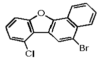 | 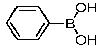 | 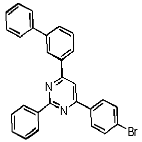 | 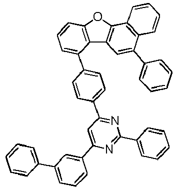 | 71% |
| **ET-009** | 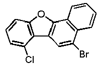 | 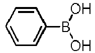 | 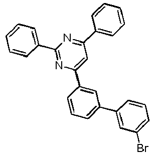 | 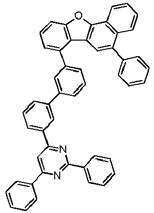 | 84% |
| **ET-010** | 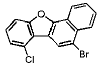 | 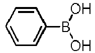 | 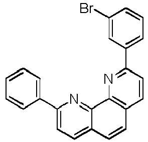 | 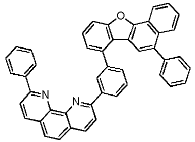 | 79% |
| **ET-011** | 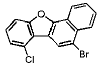 | 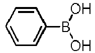 | 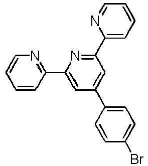 | 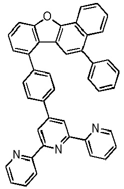 | 69% |
| **ET-012** | 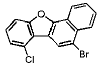 | 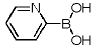 | 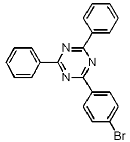 | 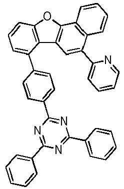 | 66% |
| **ET-013** | 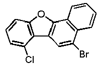 | 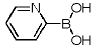 | 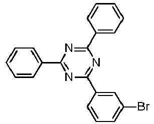 | 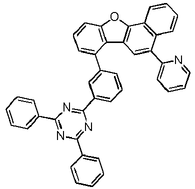 | 79% |
| **ET-016** | 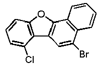 | 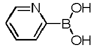 | 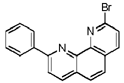 | 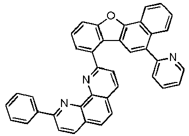 | 85% |
| **ET-019** | 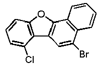 | 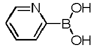 | 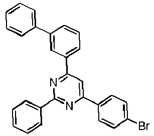 | 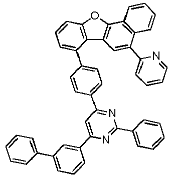 | 76% |
| **ET-022** | 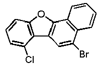 | 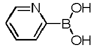 | 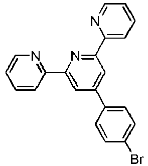 | 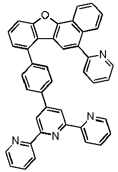 | 71% |
| **ET-034** | 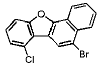 | 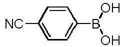 | 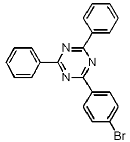 | 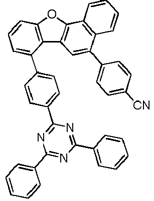 | 71% |
| **ET-036** | 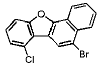 | 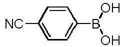 | 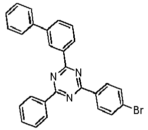 | 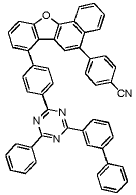 | 75% |
| **ET-041** | 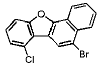 | 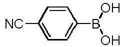 | 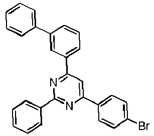 | 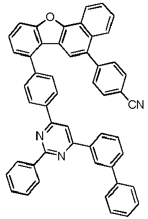 | 69% |
| **ET-042** | 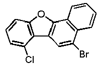 | 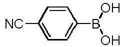 | 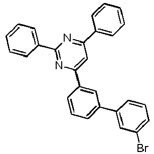 | 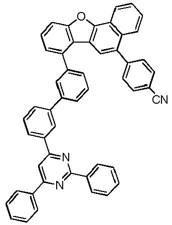 | 66% |
| **ET-047** | 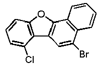 | 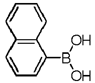 | 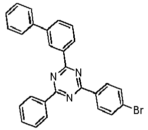 | 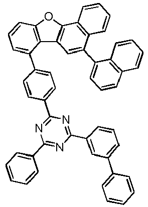 | 79% |
| **ET-048** | 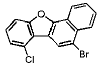 | 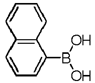 | 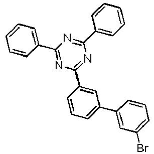 | 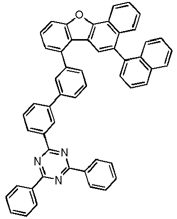 | 85% |
| **ET-052** | 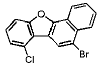 | 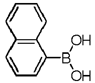 | 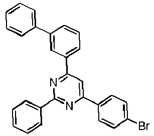 | 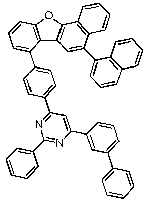 | 66% |
| **ET-053** | 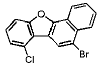 | 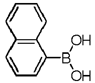 | 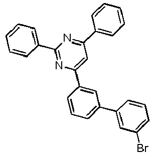 | 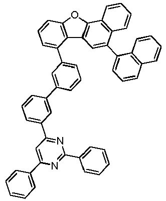 | 79% |
| **ET-056** | 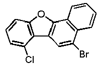 | 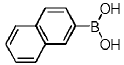 | 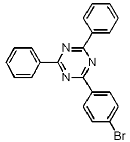 | 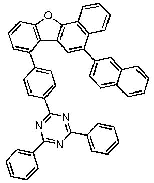 | 85% |
| **ET-057** | 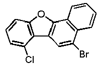 | 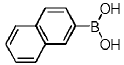 | 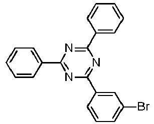 | 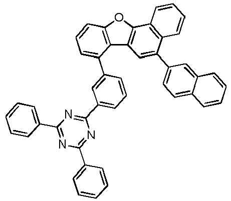 | 76% |
| **ET-058** | 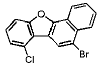 | 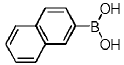 | 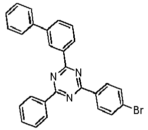 | 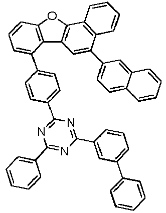 | 67% |
| **ET-059** | 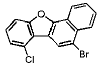 | 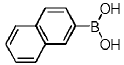 | 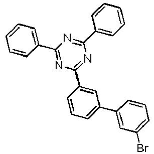 | 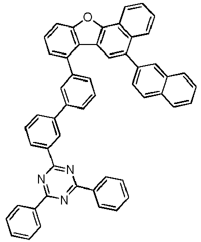 | 74% |
| **ET-060** | 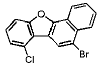 | 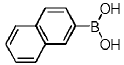 | 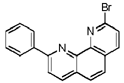 | 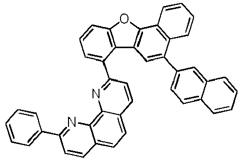 | 72% |
| **ET-062** | 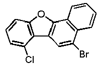 | 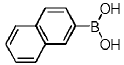 | 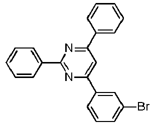 | 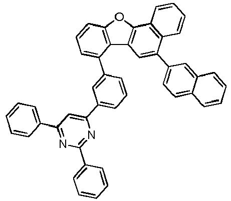 | 81% |
| **ET-067** | 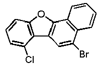 | 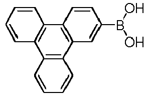 | 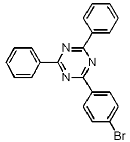 | 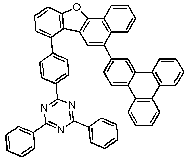 | 77% |
| **ET-068** | 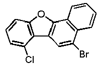 | 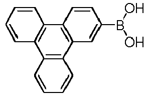 | 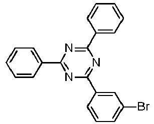 | 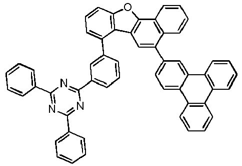 | 70% |
| **ET-069** | 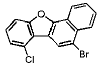 | 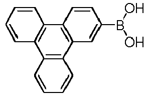 | 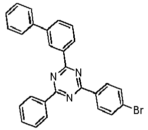 | 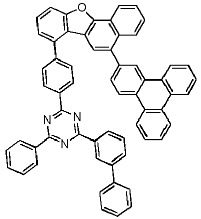 | 67% |
| **ET-070** | 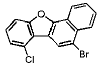 | 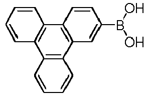 | 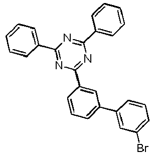 | 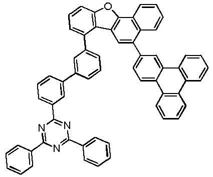 | 81% |
| **ET-072** | 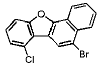 | 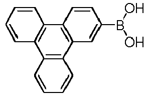 | 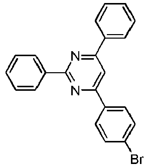 | 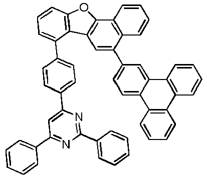 | 77% |
| **ET-080** | 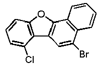 | 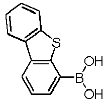 | 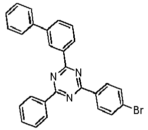 | 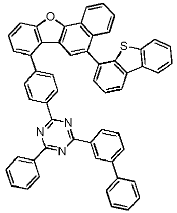 | 72% |
| **ET-083** | 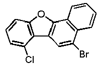 | 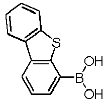 | 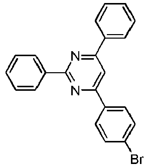 | 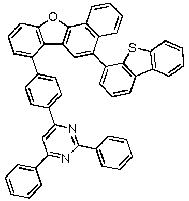 | 67% |
| **ET-091** | 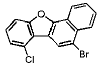 | 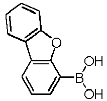 | 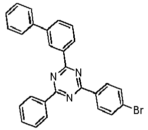 | 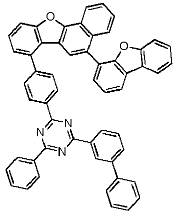 | 72% |
| **ET-092** | 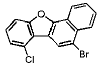 | 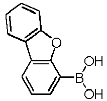 | 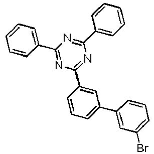 | 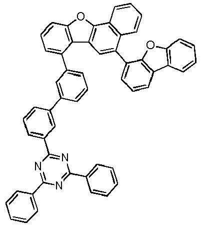 | 81% |
| **ET-098** | 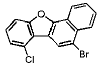 | 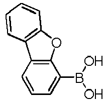 | 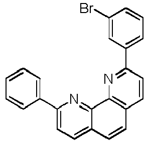 | 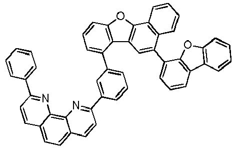 | 77% |
| **ET-100** | 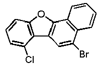 | 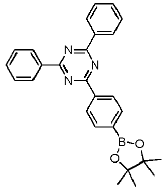 | 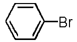 | 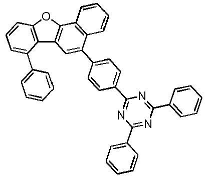 | 81% |
| **ET-101** | 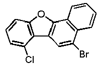 | 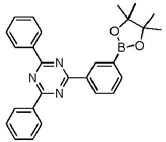 | 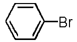 | 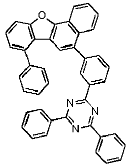 | 77% |
| **ET-102** | 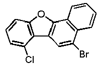 | 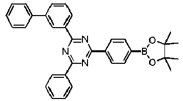 | 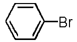 | 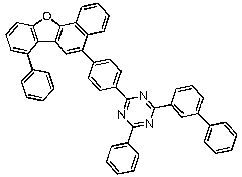 | 70% |
| **ET-103** | 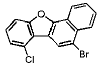 | 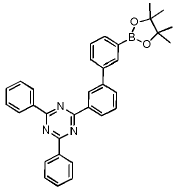 | 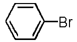 | 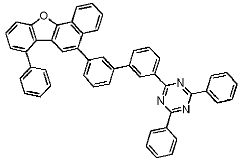 | 67% |
| **ET-105** | 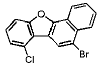 | 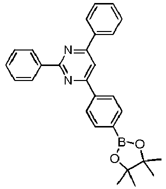 | 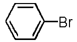 | 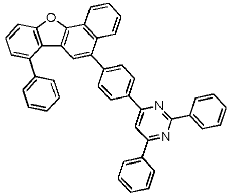 | 85% |
| **ET-106** | 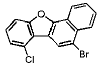 | 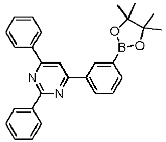 | 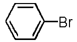 | 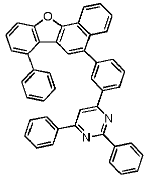 | 76% |
| **ET-107** | 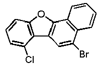 | 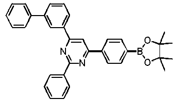 | 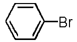 | 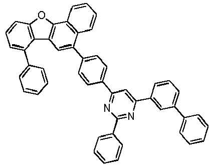 | 67% |
| **ET-108** | 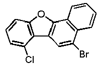 | 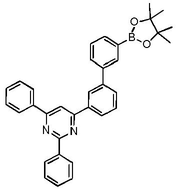 | 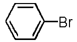 | 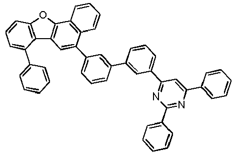 | 74% |
| **ET-109** | 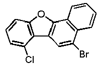 | 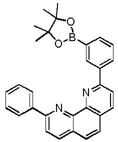 | 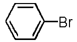 | 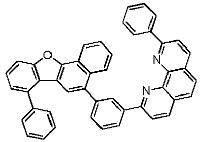 | 72% |
| **ET-110** | 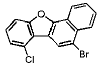 | 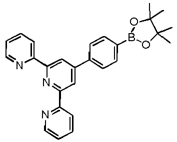 | 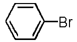 | 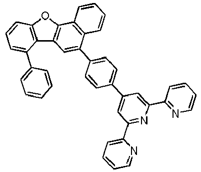 | 81% |
| **ET-114** | 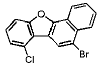 | 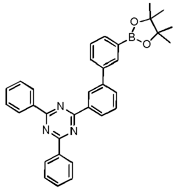 | 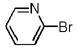 | 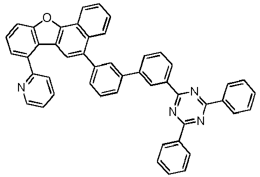 | 77% |
| **ET-119** | 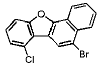 | 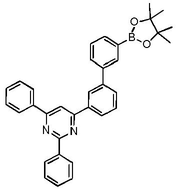 | 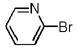 | 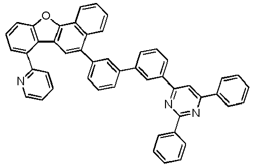 | 70% |
| **ET-133** | 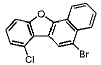 | 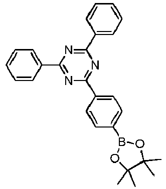 | 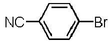 | 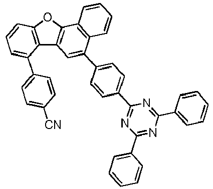 | 76% |
| **ET-134** | 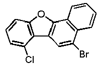 | 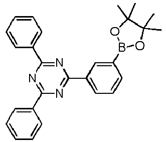 | 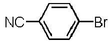 | 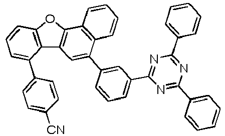 | 67% |
| **ET-149** | 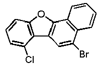 | 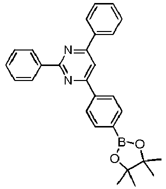 | 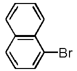 | 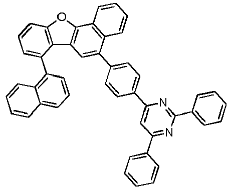 | 74% |
| **ET-150** | 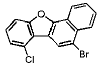 | 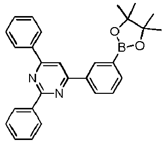 | 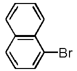 | 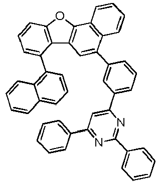 | 72% |
| **ET-157** | 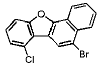 | 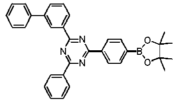 | 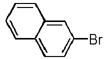 | 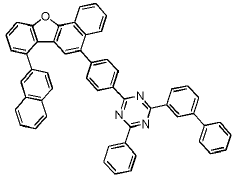 | 81% |
| **ET-166** | 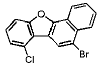 | 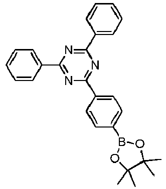 | 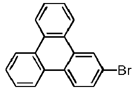 | 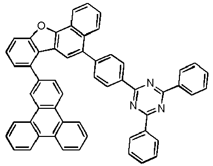 | 77% |
| **ET-167** | 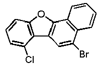 | 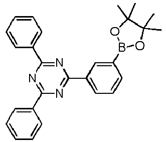 | 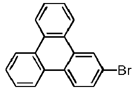 | 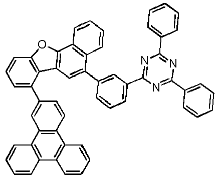 | 70% |
| **ET-168** | 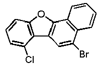 | 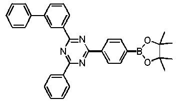 | 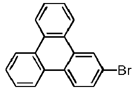 | 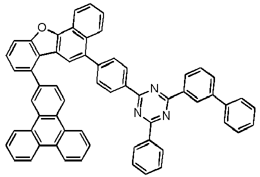 | 67% |
| **ET-169** | 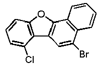 | 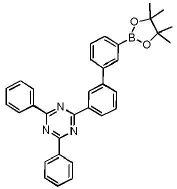 | 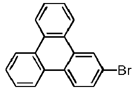 | 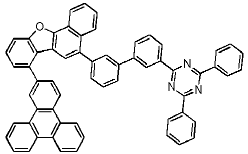 | 76% |
| **ET-171** | 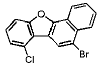 | 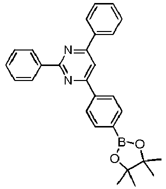 | 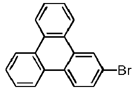 | 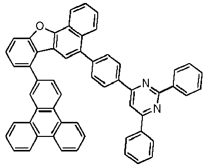 | 74% |
| **ET-175** | 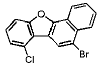 | 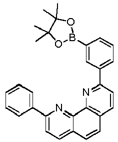 | 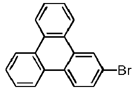 | 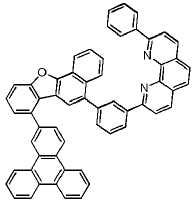 | 72% |
| **ET-182** | 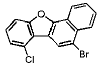 | 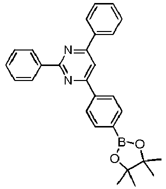 | 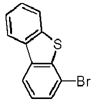 | 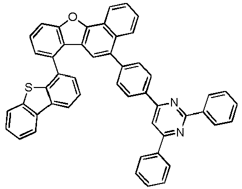 | 70% |
| **ET-190** | 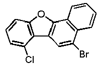 | 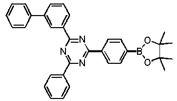 | 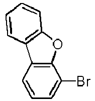 | 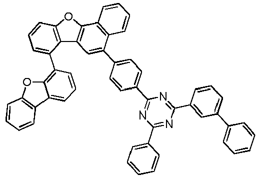 | 81% |
| **ET-211** | 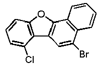 | 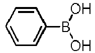 | 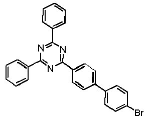 | 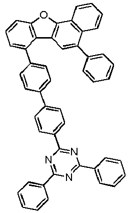 | 73% |
| **ET-212** | 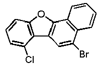 | 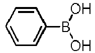 | 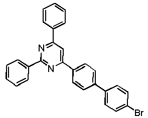 | 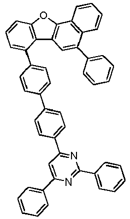 | 72% |
| **ET-243** | 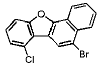 | 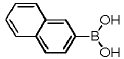 | 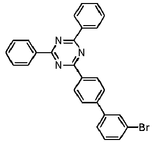 | 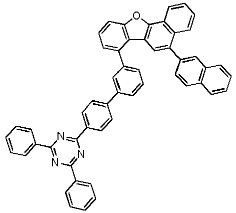 | 67% |
| **ET-244** | 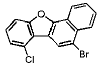 | 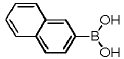 | 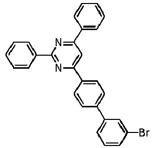 | 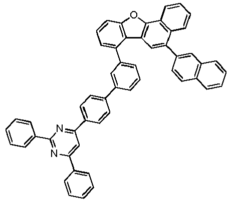 | 72% |
| **ET-299** | 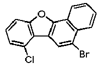 | 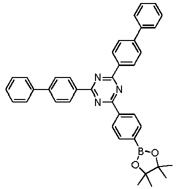 | 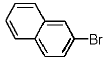 | 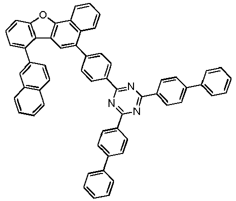 | 83% |
| **ET-300** | 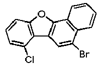 | 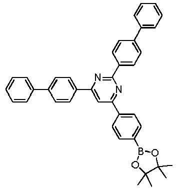 | 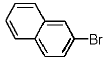 | 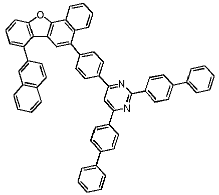 | 71% |
| **ET-303** | 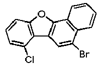 | 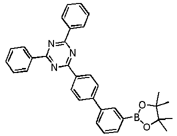 | 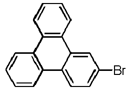 | 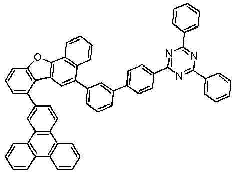 | 72% |
| **ET-354** | 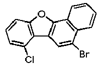 | 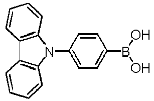 | 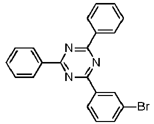 | 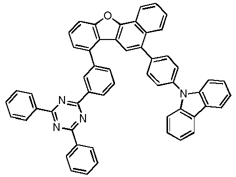 | 63% |
| **ET-357** | 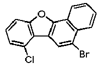 | 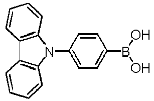 | 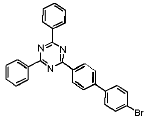 | 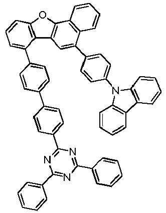 | 75% |
| **ET-358** | 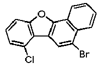 | 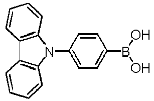 | 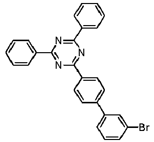 | 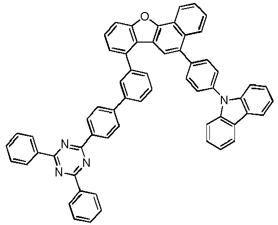 | 68% |
| **ET-361** | 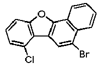 | 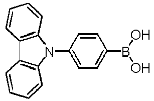 | 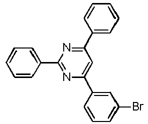 | 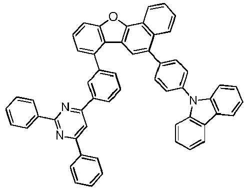 | 72% |
| **ET-388** | 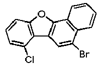 | 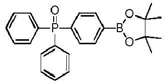 | 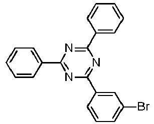 | 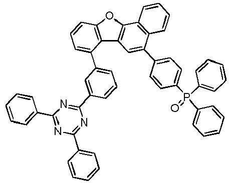 | 81% |
| **ET-389** | 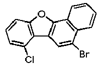 | 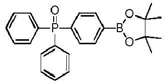 | 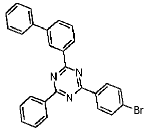 | 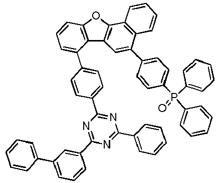 | 74% |
| **ET-392** | 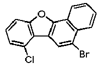 | 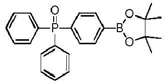 | 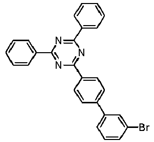 | 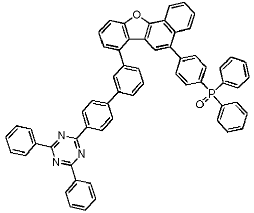 | 70% |
| **ET-401** | 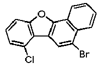 | 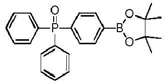 | 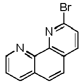 | 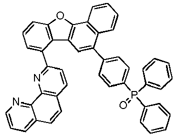 | 67% |
| **ET-402** | 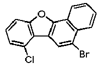 | 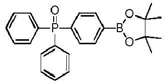 | 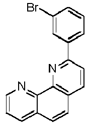 | 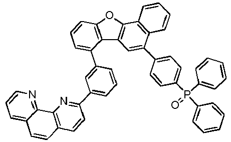 | 72% |
| **ET-408** | 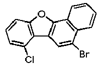 | 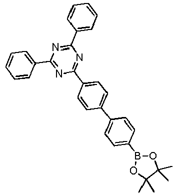 | 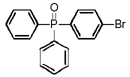 | 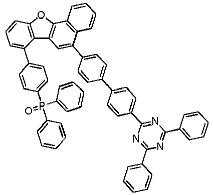 | 80% |
| **ET-411** | 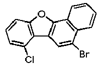 | 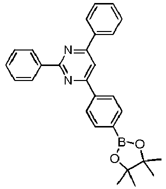 | 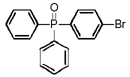 | 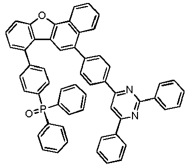 | 73% |
| **ET-419** | 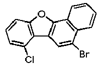 | 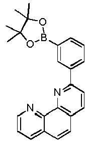 | 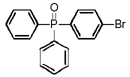 | 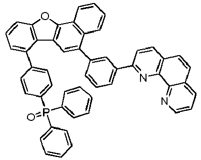 | 73% |
| **ET-459** | 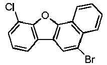 | 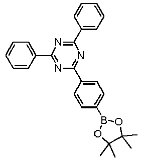 | 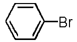 | 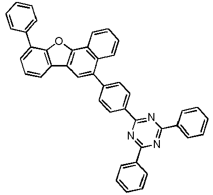 | 82% |
| **ET-463** | 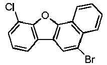 | 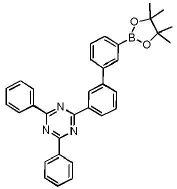 | 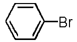 | 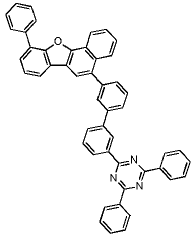 | 74% |
| **ET-474** | 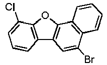 | 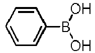 | 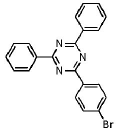 | 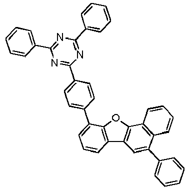 | 72% |
| **ET-487** | 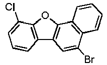 | 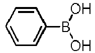 | 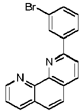 | 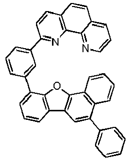 | 73% |
| **ET-489** | 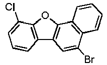 | 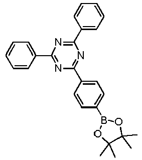 | 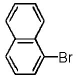 | 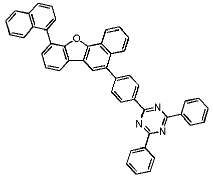 | 77% |
| **ET-496** | 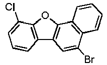 | 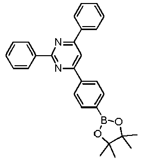 | 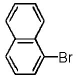 | 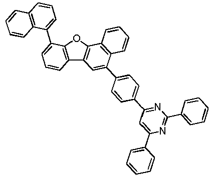 | 75% |
| **ET-511** | 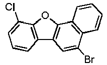 | 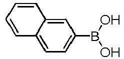 | 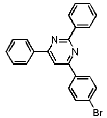 | 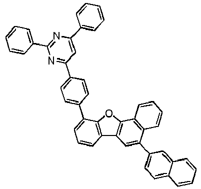 | 81% |
| **ET-517** | 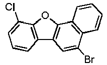 | 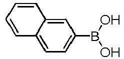 | 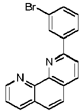 | 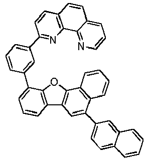 | 77% |
| **ET-535** | 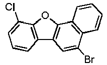 | 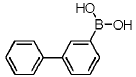 | 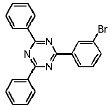 | 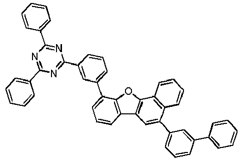 | 72% |
| **ET-549** | 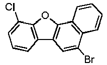 | 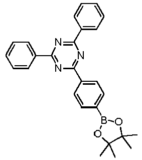 | 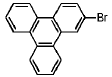 | 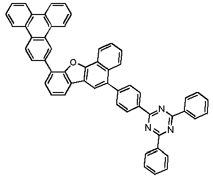 | 80% |
| **ET-550** | 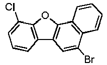 | 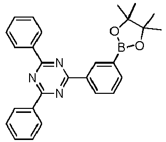 | 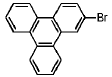 | 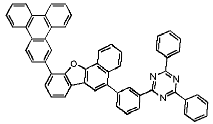 | 75% |
| **ET-571** | 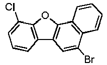 | 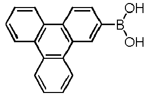 | 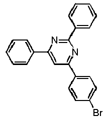 | 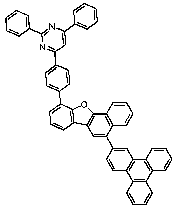 | 72% |
| **ET-579** | 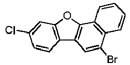 | 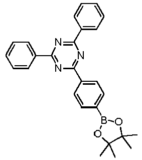 | 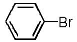 | 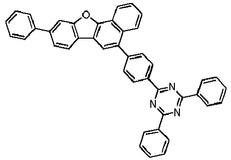 | 73% |
| **ET-582** | 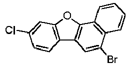 | 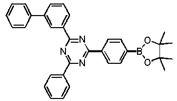 | 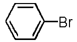 | 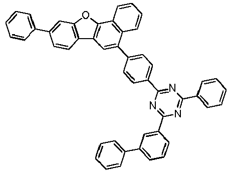 | 71% |
| **ET-603** | 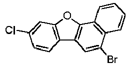 | 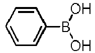 | 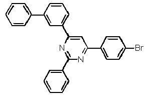 | 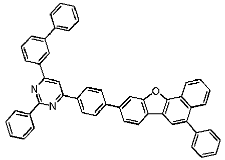 | 72% |
| **ET-615** | 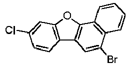 | 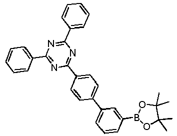 | 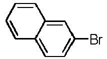 | 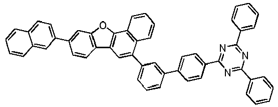 | 81% |
| **ET-639** | 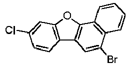 | 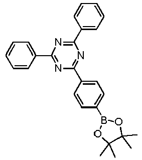 | 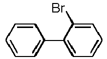 | 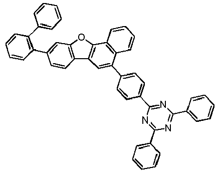 | 77% |
| **ET-640** | 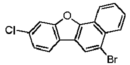 | 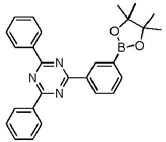 | 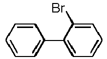 | 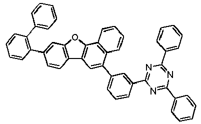 | 72% |
| **ET-652** | 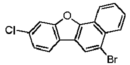 | 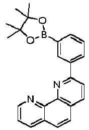 | 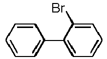 | 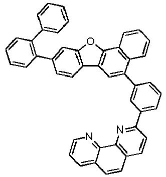 | 81% |
| **ET-661** | 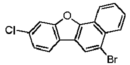 | 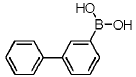 | 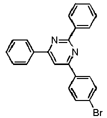 | 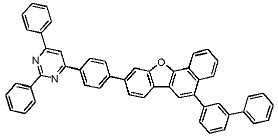 | 75% |
| **ET-699** | 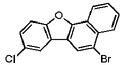 | 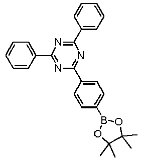 | 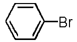 | 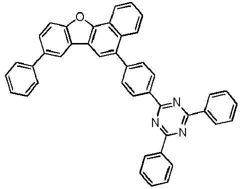 | 79% |
| **ET-712** | 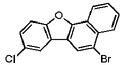 | 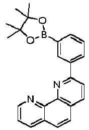 | 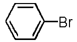 | 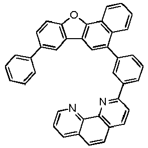 | 82% |
| **ET-729** | 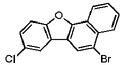 | 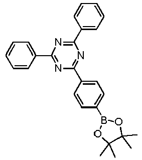 | 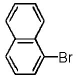 | 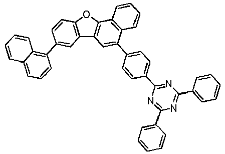 | 77% |
| **ET-744** | 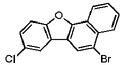 | 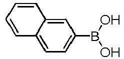 | 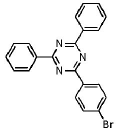 | 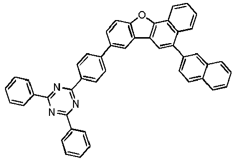 | 75% |
| **ET-752** | 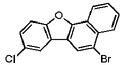 | 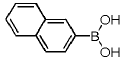 | 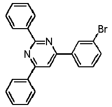 | 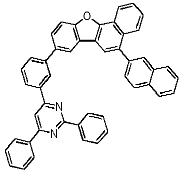 | 81% |
| **ET-765** | 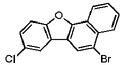 | 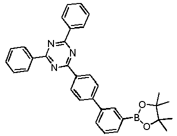 | 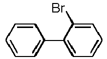 | 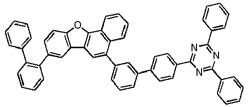 | 71% |
| **ET-774** | 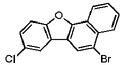 | 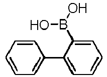 | 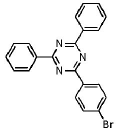 | 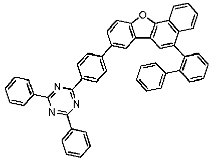 | 72% |
| **ET-781** | 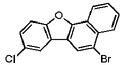 | 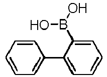 | 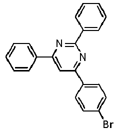 | 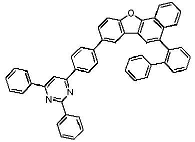 | 74% |

In order to identify synthesis identification results of Compound ET-001 and the target compounds of Table 2, ¹H NMR (CDCl₃, 200 Mz) and FD-mass spectrometry (FD-MS: field desorption mass spectrometry) were measured, and the measurement values are shown in the following Table 3 and Table 4. The following Table 3 shows measurement values of ¹H NMR (CDC1₃, 300 Mz) of Compound ET-001 and the target compounds of Table 2, and the following Table 4 shows measurement values of FD-mass spectrometry (FD-MS: field desorption mass spectrometry) of Compound ET-001 and the target compounds of Table 2.

**[Table 3]**

| NO | ¹H NMR (CDCl₃, 300 Mz) |
|---|---|
| **ET-001** | 8.55 (1H, d), 8.28 (4H, d), 8.18 (1H, d), 7.85-7.71 (6H, m) , 7.62-7.41 (13H, m), 7.25 (2H, d) |
| **ET-002** | 8.55 (1H, d), 8.28-8.18 (6H, m), 7.79-7.70 (5H, m), 7.62-7.41 (15H, m) |
| **ET-003** | 8.55 (1H, d), 8.28-8.18 (4H, m), 7.85-7.70 (7H, m), 7.62-7.41 (17H, m), 7.25 (2H, d) |
| **ET-004** | 8.55 (1H, d), 8.28-8.18 (6H, m), 7.79-7.70 (6H, m), 7.62-7.41 (18H, m) |
| **ET-005** | 8.55 (1H, d), 8.30-8.10 (7H, m), 7.81-7.79 (3H, m), 7.71 (1H, s), 7.68 (1H, d), 7.55-7.35 (11H, m) |
| **ET-006** | 8.55 (1H, d), 8.30-8.18 (6H, m), 7.79-7.71 (6H, m), 7.62-7.41 (13H, m), 7.25 (2H, d) |
| **ET-007** | 8.55 (1H, d), 828-8.18 (4H, m), 7.79-7.70 (8H, m), 7.57-7.41 (15H, m) |
| **ET-008** | 8.55 (1H, d), 8.30-8.18 (6H, m), 7.79-7.70 (6H, m), 7.62-7.41 (17H, m), 725 (2H, d) |
| **ET-009** | 8.55 (1H, d), 8.28-8.18 (4H, m), 7,79-7.70 (9H, m), 7.62-7.41 (18H, m) |
| **ET-010** | 8.55 (1H, d), 8.30-8.06 (8H, m), 7.81-7.71 (5H, m), 7.62-7.35 (14H, m) |
| **ET-011** | 9.30 (2H, d), 9.15 (2H, s), 8.55-8.53 (3H, m), 8.18 (1H, d), 7.75-7.44 (13H, m), 7.25 (4H, d), 7.14 (2H, t) |
| **ET-012** | 8.55-8.50 (2H, m), 8.28-8.18 (6H, m), 7.85 (2H, d), 7.75 (1H, d), 7.62-7.41 (11H, m), 7.26-7.25 (3H, m), 7.00 (1H, t) |
| **ET-013** | 8.55-8.50 (2H, m), 8.28-8.18 (7H, m), 7.75-7.50 (2H, d), 7.62-7.41 (13H, m), 7.26 (1H, d), 7.00 (1H, t) |
| **ET-016** | 8.55-8.50 (2H, m), 8.30-8.06 (8H, m), 7.81 (1H, d), 7.68 (1H, d), 7.55-7.47 (7H, m), 7,35 (2H, d), 7.26 (1H, d), 7.00 (1H, t) |
| **ET-019** | 8.55-8.50 (2H, m), 8.30-8.18 (7H, m), 7.75-7.70 (3H, m), 7.62-7.41 (15H, m), 7.26-7.25 (3H, m), 7.00 (1H, t) |
| **ET-022** | 9.30 (2H, d), 9.15 (2H, s), 8.55-8.50 (4H, m), 8.22-8.18 (2H, m), 7.70-7.44 (8H, m), 7.26-7.25 (5H, m), 7.14 (2H, m), 7.00 (1H, t) |
| **ET-034** | 8.55 (1H, d), 8.28 (4H, d), 8.18 (1H, d), 7.85-7.71 (8H, m), 7.62-7.41 (10H, m), 7.25 (2H, d) |
| **ET-036** | 8.55 (1H, d), 8.28-8.18 (4H, m), 7.85-7.71 (9H, m), 7.62-7.41 (14H, m), 7.25 (2H, d) |
| **ET-041** | 8.55 (1H, d), 8.30-8.18 (6H, m), 7.84-7.70 (8H, m), 7.62-7.41 (14H, m), 7.25 (2H, d) |
| **ET-042** | 8.55 (1H, d), 8.28-8.18 (4H, m), 7.84-7.70 (11H, m), 7.62-7.41 (15H, m) |
| **ET-047** | 8.55 (2H, d), 8.42 (1H, d), 8.28-8.18 (4H, m), 8.08-8.04 (2H, m), 7.85 (2H, d), 7.75-7.70 (3H, d), 7.62-7.41 (17H, m), 7.25 (2H, d) |
| **ET-048** | 8.55 (2H, d), 8.42 (1H, d), 8.28-8.18 (6H, m), 8.08-8.04 (2H, m), 7.75-7.70 (4H, m), 7.62-7.41 (18H, m) |
| **ET-052** | 8.55 (2H, d), 8.42 (1H, d), 8.30-8.18 (6H, m), 8.08-8.04 (2H, m), 7.75-7.70 (4H, m), 7.62-7.41 (17H, m), 7.25 (2H, d) |
| **ET-053** | 8.55 (2H, d), 8.42 (1H, d), 8.28-8.18 (4H, m), 8.08-8.04 (2H, m), 7.79-7.70 (7H, m), 7.61-7.41 (18H, m) |
| **ET-056** | 8.55 (1H, d), 8.28 (4H, d), 8.18 (1H, d), 8.00-7.85 (5H, m), 7.75-7.71 (3H, m), 7.62-7.41 (13H, m), 7.25 (2H, d) |
| **ET-057** | 8.55 (1H, d), 8.28-8.18 (6H, m), 8.00-7.92 (3H, m), 7.75-7.70 (4H, m), 7.62-7.41 (15H, m) |
| **ET-058** | 8.55 (1H, d), 8.28-8.18 (4H, m), 8.00-7.85 (5H, m), 7.75-7.70 (4H, m), 7.62-7.41 (17H, m), 7.25 (2H, d) |
| **ET-059** | 8.55 (1H, d), 8.28-8.18 (6H, m), 8.00-7.92 (3H, m), 7.75-7.70 (5H, m), 7.62-7.41 (18H, m) |
| **ET-060** | 8.55 (1H, d), 8.30-7.92 (10H, m), 7.81 (1H, d), 7.73-7.41 (12H, m), 7.35 (2H, d) |
| **ET-062** | 8.55 (1H, d), 8.28-8.18 (4H, m), 8.00-7.92 (3H, m), 7.79-7.70 (7H, m), 7.62-7.41 (15H, m) |
| **ET-067** | 9.15 (1H, s), 8.93 (2H, d), 8.55 (1H, d), 8.28-8.04 (9H, m), 7.88-7.71 (8H, m), 7.62-7.41 (10H, m), 7.25 (2H, d) |
| **ET-068** | 9.15 (1H, s), 8.93 (2H, d), 8.55 (1H, d), 8.28-8.04 (10H, m), 7.88-7.70 (7H, m), 7.62-7.41 (12H, m) |
| **ET-069** | 9.15 (1H, s), 8.93 (2H, d), 8.55 (1H, d), 8.28-8.12 (7H, m), 8.04 (1H, d), 7.88-7.70 (9H, m), 7.62-7.41 (14H, m), 7.25 (2H, d) |
| **ET-070** | 9.15 (1H, s), 8.93 (2H, d), 8.55 (1H, d), 8.28-8.12 (9H, m), 8.04 (1H, d), 7.88-7.70 (8H, m), 7.62-7.41 (15H, m) |
| **ET-072** | 9.15 (1H, s), 8.93 (2H, d), 8.55 (1H, d), 8.30-8.12 (10H, m), 7.88-7.71 (8H, m), 7.62-7.41 (10H, m), 7.25 (2H, d) |
| **ET-080** | 8.55 (1H, d), 8.45-8.41 (2H, m), 8.28-8.18 (5H, m), 7.98 (1H, d), 7.85 (2H, d), 7.75-7.71 (2H, m), 7.62-7.41 (17H, m), 7.25 (2H, d) |
| **ET-083** | 8.55 (1H, d), 8.45-8.41 (2H, m), 8.30-8.18 (7H, m), 7.98 (1H, d), 7.79-7.71 (4H, m), 7.62-7.41 (13H, m), 7.25 (2H, d) |
| **ET-091** | 8.55 (1H, d), 8.28-8.18 (4H, m), 7.85-7.32 (28H, m) |
| **ET-092** | 8.55 (1H, d), 8.28-8.18 (6H, m), 7.89-7.32 (26H, m) |
| **ET-098** | 8.55 (1H, d), 8.30-8.06 (8H, m), 7.89-7.32 (21H, m) |
| **ET-100** | 8.55 (1H, d), 8.28 (4H, d), 8.18 (1H, d), 7.85-7.71 (6H, m), 7.62-7.41 (13H, m), 7.25 (2H, d) |
| **ET-101** | 8.55 (1H, d), 8.28-8.18 (6H, m), 7.79-7.70 (5H, m), 7.62-7.41 (15H, m) |
| **ET-102** | 8.55 (1H, d), 8.28-8.18 (4H, m), 7.85-7.70 (7H, m), 7.62-7.41 (17H, m), 7.25 (2H, d) |
| **ET-103** | 8.55 (1H, d), 8.28-8.18 (6H, m), 7.79-7.70 (6H, m), 7.62-7.41 (18H, m) |
| **ET-105** | 8.55 (1H, d), 8.30-8.18 (6H, m), 7.79-7.71 (6H, m), 7.62-7.41 (13H, m), 7.25 (2H, d) |
| **ET-106** | 8.55 (1H, d), 8.28-8.18 (4H, m), 7.79-7.71 (8H, m), 7.62-7.41 (15H, m) |
| **ET-107** | 8.55 (1H, d), 8.30-8.18 (6H, m), 7.79-7.70 (6H, m), 7.62-7.41 (17H, m), 7.25 (2H, d) |
| **ET-108** | 8.55 (1H, d), 8.28-8.18 (4H, m), 7.79-7.70 (9H, m), 7.62-7.41 (18H, m) |
| **ET-109** | 8.55 (1H, d), 8.30-8.06 (8H, m), 7.81-7.71 (5H, m), 7.62-7.35 (14H, m) |
| **ET-110** | 8.30 (2H, d), 9.15 (2H, s), 8.55-8.53 (3H, m), 8.18 (1H, d), 7.79-7.41 (13H, m), 7.25 (4H, d), 7.14 (2H, t) |
| **ET-114** | 8.55-8.50 (2H, m), 8.28-8.18 (7H, m), 7.71-7.68 (4H, m), 7.57-7.41 (15H, m), 7.26 (1H, d), 7.00 (1H, t) |
| **ET-119** | 8.55-8.50 (2H, m), 8.28-8.18 (5H, m), 7.79-7.68 (7H, m), 7,57-7.41 (15H, m), 7.26 (1H, d), 7.00 (1H, t) |
| **ET-133** | 8.55 (1H, d) , 8.28 (4H, d), 8.18 (1H, d), 7.85-7.71 (8H, m), 7.62-7.41 (10H, m), 7.25 (2H, d) |
| **ET-134** | 8.55 (1H, d), 8.28-8.18 (6H, m), 7.84-7.70 (7H, m), 7.62-7.41 (12H, m) |
| **ET-149** | 8.55 (2H, d), 8.42 (1H, d), 8.30-8.18 (6H, m), 8.08-8.04 (2H, m), 7.79-7.71 (4H, m), 7.62-7.41 (13H, m), 7.25 (2H, d) |
| **ET-150** | 8.55 (2H, d), 8.42 (1H, d), 8.28-8.18 (4H, m), 8.08-8.04 (2H, m), 7.79-7.70 (6H, m), 7.62-7.41 (15H, m) |
| **ET-157** | 8.55 (1H, d), 8.28-8.18 (4H, m), 8.00-7.85 (5H, m), 7.75-7.70 (4H, m), 7.62-7.41 (17H, m), 7.25 (2H, d) |
| **ET-166** | 9.15 (1H, s), 8.93 (2H, d), 8.55 (1H, d), 8.28-8.8.04 (9H, m), 7.85-7.71 (8H, m), 7.62-7.41 (10H, m), 7.25 (2H, d) |
| **ET-167** | 9.15 (1H, s), 8.93 (2H, d), 8.55 (1H, d), 8.28-8.04 (10H, m), 7.88-7.70 (7H, m), 7.62-7.41 (12H, m) |
| **ET-168** | 9.15 (1H, s), 8.93 (2H, d), 8.55 (1H, d), 8.28-8.12 (7H, m), 8.04 (1H, d), 7.88-7.70 (9H, m), 7.62-7.41 (14H, m), 7.25 (2H, d) |
| **ET-169** | 9.15 (1H, s), 8.93 (2H, d), 8.55 (1H, d), 8.28-8.12 (9H, m), 8.04 (1H, d), 7.88-7.70 (8H, m), 7.62-7.41 (15H, m) |
| **ET-171** | 9.15 (1H, s), 8.93 (2H, d), 8.55 (1H, d), 8.28-8.04 (10H, m), 7.88-7.71 (8H, m), 7.62-7.41 (10H, m), 7.25 (2H, d) |
| **ET-175** | 9.15 (1H, s), 8.93 (2H, d), 8.55 (1H, d), 8.30-8.04 (12H, m), 7.88-7.71 (7H, m), 7.62-7.35 (11H, m) |
| **ET-182** | 8.55 (1H, d), 8.45-8.41 (2H, m), 8.30-8.18 (7H, m), 7.98 (1H, d), 7.79-7.71 (4H, m), 7.62-7.41 (13H, m), 7.25 (2H, d) |
| **ET-190** | 8.55 (1H, d), 8.28-8.18 (4H, m), 7.85-7.32 (28H, m) |
| **ET-211** | 8.55 (1H, d), 8.28 (4H, d), 8.18 (1H, d), 7.85-7.71 (6H, m), 7.62-7.41 (13H, m), 7.25 (6H, d) |
| **ET-212** | 8.55 (1H, d), 8.30-8.18 (6H, m), 7.85-7.71 (8H, m), 7.62-7.41 (13H, m), 7.25 (4H, d) |
| **ET-243** | 8.55 (1H, d), 8.28 (4H, d), 8.18 (1H, d), 8.00-7.85 (5H, m), 7.75-7.70 (4H, m), 7.59-7.41 (16H, m), 7.25 (2H, d) |
| **ET-244** | 8.55 (1H, d), 8.30-8.18 (6H, m), 8.00-7.70 (11H, m), 7.62-7.41 (16H, m) |
| **ET-299** | 8.55 (1H, d), 8.18 (1H, d), 8.00 (2H, d), 7.92-7.85 (7H, m), 7.75-7.71 (3H, m), 7.62-7.41 (17H, m), 7.25 (6H, d) |
| **ET-300** | 8.55 (1H, d), 8.30 (4H, d), 8.23-8.18 (2H, m), 8.00 (2H, d), 7.92-7.85 (5H, m), 7.75-7.71 (3H, m), 7.62-7.41 (17H, m), 7.25 (4H, d) |
| **ET-303** | 9.15 (1H, s), 8.93 (2H, d), 8.55 (1H, d), 8.28 (4H, d), 8.18-8.04 (5H, m), 7.88-7.70 (9H, m), 7.62-7.41 (13H, m), 7.25 (2H, d) |
| **ET-354** | 8.55 (2H, d), 8.28-8.12 (7H, m), 7.94 (1H, d), 7.75-7.25 (24H, m) |
| **ET-357** | 8.55 (2H, d), 8.28 (4H, d), 8.18-8.12 (2H, m), 7.94 (1H, d), 7.79-7.25 (29H, m) |
| **ET-358** | 8.55 (2H, d), 8.28 (4H, d), 8.18-8.12 (2H, m), 7.94 (1H, d), 7.85-7.25 (29H, m) |
| **ET-361** | 8.55 (2H, d), 8.28-8.12 (5H, m), 7.94 (1H, d), 7.79-7.25 (27H, m) |
| **ET-388** | 8.55 (1H, d), 8.28-8.18 (6H, m), 7.83-7.70 (11H, m), 7.62-7.41 (18H, m) |
| **ET-389** | 8.55 (1H, d), 8.28-8.18 (4H, m), 7.85-7.70 (13H, m), 7.62-7.41 (20H, m) |
| **ET-392** | 8.55 (1H, d), 8.28 (4H, d), 8.18 (1H, d), 7.85-7.70 (13H, m), 7.62-7.41 (19H, m), 7.25 (2H, d) |
| **ET-401** | 8.83 (1H, d), 8.55 (1H, d), 8.38 (1H, d), 8.26 (1H, d), 8.18 (1H, d), 8.10-8.06 (2H, m), 7.83-7.68 (11H, m), 7.58-7.45 (10H, m), 7.35 (1H, d) |
| **ET-402** | 8.83 (1H, d), 8.55 (1H, d), 8.38 (1H, d), 8.26-8.18 (3H, m), 8.10-8.06 (2H, m), 7.83-7.71 (11H, m), 7.62-7.44 (13H, m), 7.35 (1H, d) |
| **ET-408** | 8.55 (1H, d), 8.28 (4H, d), 8.18 (1H, d), 7.85-7.71 (12H, m), 7.62-7.41 (16H, m), 7.25 (6H, d) |
| **ET-411** | 8.55 (1H, d), 8.30-8.18 (6H, m), 7.83-7.71 (12H, m), 7.62-7.41 (16H, m), 7.25 (2H, d) |
| **ET-419** | 8.83 (1H, d), 8.55 (1H, d), 8.38 (1H, d), 8.26-8.18 (3H, m), 8.10-8.06 (2H, m), 7.83-7.71 (11H, m), 7.62-7.44 (13H, m), 7.35 (1H, d) |
| **ET-459** | 8.55 (1H, d), 8.28 (4H, d), 8.18 (1H, d), 7.85-7.81 (4H, m), 7.71 (1H, s), 7.55-7.38 (14H, m), 7.25 (2H, d) |
| **ET-463** | 8.55 (1H, d), 8.28-8.18 (6H, m), 7.85-7.81 (2H, m), 7.71-7.70 (3H, s), 7.57-7.38 (19H, m) |
| **ET-474** | 8.55 (1H, d), 8.28 (4H, d), 8.18 (1H, d), 7.85-7.79 (6H, m), 7.71 (1H, s), 7.55-7.38 (12H, m), 7.25 (2H, d) |
| **ET-487** | 8.83 (1H, d), 8.55 (1H, d), 8.38 (1H, d), 8.26-8.06 (5H, m), 7.85-7.79 (5H, m), 7.71 (1H, s), 7.58-7.35 (10H, m) |
| **ET-489** | 8.55 (2H, d), 8.42 (1H, d, 8.28 (4H, d), 8.18 (1H, d), 8.08-8.04 (2H, m), 7.85-7.81 (4H, m), 7.71 (1H, s), 7.61-7.38 (12H, m), 7.25 (2H, d) |
| **ET-496** | 8.55 (2H, d), 8.42 (1H, d), 8.30-8.18 (6H, m), 8.08-8.04 (2H, m), 7.85-7.79 (4H, m), 7.71 (1H, s), 7.61-7.38 (12H, m), 7.25 (2H, d) |
| **ET-511** | 8.55 (1H, d), 8.30-8.18 (6H, m), 8.00-7.71 (9H, m), 7.59-7.38 (12H, m), 7.25 (2H, d) |
| **ET-517** | 8.83 (1H, d), 8.55 (1H, d), 8.38 (1H, d), 8.26-7.71 (13H, m), 7.60-7.55 (8H, m), 7.38-7.35 (2H, m) |
| **ET-535** | 8.55 (1H, d), 8.28-8.18 (6H, m), 7.85-7.81 (2H, m), 7.71-7.70 (3H, m), 7.57-7.38 (19H, m) |
| **ET-549** | 9.15 (1H, s), 8.93 (2H, d), 8.55 (1H, d), 8.28 (4H, d), 8.18-8.04 (5H, m), 7.88-7.81 (8H, m), 7.71 (1H, s), 7.55-7.38 (9H, m), 7.25 (2H, d) |
| **ET-550** | 9.15 (1H, s), 8.93 (2H, d), 8.55 (1H, d), 8.28-8.04 (10H, m), 7.88-7.81 (6H, m), 7.71-7.70 (2H, s), 7.57-7.38 (11H, m) |
| **ET-571** | 9.15 (1H, s), 8.93 (2H, d), 8.55 (1H, d), 8.30-8.12 (10H, m), 7.88-7.79 (8H, m), 7.71 (1H, s), 7.55-7.38 (9H, m), 7.25 (2H, d) |
| **ET-579** | 8.55 (1H, d), 8.28 (4H, d), 8.18 (1H, d), 7.95 (1H, d), 7.85 (2H, d), 7.75-7.71 (2H, m), 7.64 (1H, s), 7.55-7.41 (13H, m), 7.25 (2H, d) |
| **ET-582** | 8.55 (1H, d), 8.28-8.18 (4H, m), 7.95 (1H, d), 7.85 (2H, d), 7.75-7.70 (3H, m), 7.4-7.41 (18H, m), 7.25 (2H, d) |
| **ET-603** | 8.55 (1H, d), 8.30-8.18 (6H, m), 7.95 (1H, d), 7.85-7.70 (8H, m), 7.64-7.41 (16H, m) |
| **ET-615** | 8.55 (1H, d), 8.28 (4H, d), 8.18 (1H, d), 8.00-7.85 (6H, m), 7.71-7.41 (19H, m), 7.25 (2H, d) |
| **ET-639** | 8.55 (1H, d), 8.28 (4H, d), 8.18 (1H, d), 7.98 (1H, d), 7.85-7.71 (8H, m), 7.64 (1H, s), 7.55-7.41 (13H, m), 7.25 (2H, d) |
| **ET-640** | 8.55 (1H, d), 8.28-8.18 (6H, m), 7.95 (1H, d), 7.85-7.70 (7H, m), 7.57 (16H, m) |
| **ET-652** | 8.83 (1H, d), 8.55 (1H, d), 8.38 (1H, d), 8.26-8.06 (5H, m), 7.95 (1H, d), 7.85-7.71 (7H, m), 7.60-7.35 (12H, m) |
| **ET-661** | 8.55 (1H, d), 8.30-8.18 (6H, m), 7.95 (1H, d), 7.85-7.70 (7H, m), 7.57-7.41 (17H, m) |
| **ET-699** | 8.55 (1H, d), 8.28 (4H, d), 8.18 (1H, d), 7.85-7.81 (3H, m), 7.72-7.71 (3H, m), 7.55-7.41 (13H, m), 7.25 (2H, d) |
| **ET-712** | 8.83 (1H, d), 8.55 (1H, d), 8.38 (1H, d), 8.26-8.06 (5H, m), 7.81 (2H, d), 7.72-7.71 (3H, m), 7.60-7.35 (13H, m) |
| **ET-729** | 8.55 (2H, d), 8.42 (1H, d), 8.28 (4H, d), 8.18 (1H, d), 8.08-8.04 (2H, m), 7.85-7.81 (3H, m), 7.72-7.71 (3H, m), 7.61-7.41 (11H, m), 7.25 (2H, d) |
| **ET-744** | 8.55 (1H, d), 8.28 (4H, d), 8.18 (1H, d), 8.00-7.71 (10H, m), 7.59-7.41 (11H, m), 7.25 (2H, d) |
| **ET-752** | 8.55 (1H, d), 8.28-8.18 (4H, m), 8.00-7.92 (3H, m), 7.81-7.71 (9H, m), 7.59-7.41 (13H, m) |
| **ET-765** | 8.55 (1H, d), 8.28 (4H, d), 8.18 (1H, d), 7.85-7.70 (11H, m), 7.57-7.41 (16H, m), 7.25 (2H, d) |
| **ET-774** | 8.55 (1H, d), 8.28 (4H, d), 8.18 (1H, d), 7.85-7.71 (10H, m), 7.55-7.41 (13H, m), 7.25 (2H, d) |
| **ET-781** | 8.55 (1H, d), 8.30-8.18 (6H, m), 7.85-7.71 (12H, m), 7.55-7.41 (13H, m) |

**[Table 4]**

| **Compound** | FD-MS | **Compound** | FD-MS |
|---|---|---|---|
| **ET-001** | m/z=601.69 (C₄₃H₂₇N₃O=601.22) | **ET-002** | m/z=601.69 (C₄₃H₂₇N₃O=601.22) |
| **ET-003** | m/z=677. 79 (C₄₉H₃₁N₃O=677.25) | **ET-004** | m/z=677.79 (C₄₉H₃₁N₃O=677.25) |
| **ET-005** | m/z=548.63 (C₄H₂₄N₂O=548.19) | **ET-006** | m/z=600.71 (C₄₄H₂₈N₂O=600.22) |
| **ET-007** | m/z=600.71 (C₄₄H₂₈N₂O=600.22) | **ET-008** | m/z=676.80 (C₅₀H₃₂N₂O=676.25) |
| **ET-009** | m/z=676.80 (C₅₀H₃₂N₂O=676.25) | **ET-010** | m/z=624.73 (C₄₆H₂₈N₂O=624.22) |
| **ET-011** | m/z=601.69 (C₄₃H₂₇N₃O=601.22) | **ET-012** | m/z=602 . 68 (C₄₂H₂₆N₄O=602.21) |
| **ET-013** | m/z=602 . 68 (C₄₂H_{z6}N₄O=602.21) | **ET-016** | m/z=549.62 (C₃₉H₂₃N₃O=549.18) |
| **ET-019** | m/z=677.79 (C₄₉H₃₁N₃O=677.25) | **ET-022** | m/z=602 . 68 (C₄₂H₂₆N₄O=602.21) |
| **ET-034** | m/z=626.70 (C₄₄H₂₆N₄O=626.21) | **ET-036** | m/z=702. 80 (C₅₀H₃₀N₄O=702.24) |
| **ET-041** | m/z=701. 81 (C₅₁H₃₁N₃O=701.25) | **ET-042** | m/z=701.81 (C₅₁H₃₁N₃O=701.25) |
| **ET-047** | m/z=727. 85 (C₅₃H₃₃N₃O=727.26) | **ET-048** | m/z=727.85 (C₅₃H₃₃N₃O=727.26) |
| **ET-052** | m/z=726.86 (C₅₄H₃₄N₂O=726.27) | **ET-053** | m/z=726.86 (C₅₄H₃₄N₂O=726.27) |
| **ET-056** | m/z=651.75 (C₄₇H₂₉N₃O=651.23) | **ET-057** | m/z=651.75 (C₄₇H₂₉N₃O=651.23) |
| **ET-058** | m/z=727.85 (C₅₃H₃₃N₃O=727.26) | **ET-059** | m/z=727.85 (C₅₃H₃₃N₃O=727.26) |
| **ET-060** | m/z=598.69 (C₄₄H₂₆N₂O=598.20) | **ET-062** | m/z=650.76 (C₄₈H₃()N₂O=650.24) |
| **ET-067** | m/z=751. 87 (C₅₅H₃₃N₃O=751. 26) | **ET-068** | m/z=751.87 (C₅₅H₃₃N₃O=751.26) |
| **ET-069** | m/z=827.97 (C₆₁H₃₇N₃O=827.29) | **ET-070** | m/z=827.97 (C₆₁H₃₇N₃O=827.29) |
| **ET-072** | m/z=750.88 (C₅₆H₃₄N₂O=750.27) | **ET-080** | m/z=783.94 (C₅₅H₃₃N₃OS=783.23) |
| **ET-083** | m/z=706.85 (C₅()H₃()N₂OS=706.21) | **ET-091** | m/z=767.87 (C₅₅H₃₃N₃O₂=767.26) |
| **ET-092** | m/z=767. 87 (C₅₅H₃₃N₃O₂=767.26) | **ET-098** | m/z=714. 81 (C₅₂H₃₀N₂O₂=714. 23) |
| **ET-100** | m/z=601. 69 (C₄₃H₂N₃O=601.22) | **ET-101** | m/z=601.69 (C₄₃H₂₇N₃O=601.22) |
| **ET-102** | m/z=677. 79 (C₄₉H₃₁N₃O=677.25) | **ET-103** | m/z=677.79 (C₄₉H₃₁N₃O=677.25) |
| **ET-105** | m/z=600.71 (C₄₄H₂₈N₂O=600.22) | **ET-106** | m/z=600.71 (C₄₄H₂₈N₂O=600.22) |
| **ET-107** | m/z=676.80 (C₅₀H₃₂N₂O=676.25) | **ET-108** | m/z=676.80 (C₅₀H₃₂N₂O=676.25) |
| **ET-109** | m/z=624.73 (C₄₆H₂₈N₂O=624.22) | **ET-110** | m/z=601.69 (C₄₃H₂₇N₃O=601.22) |
| **ET-114** | m/z=678.78 (C₄₈H₃₀N₄O=678.24) | **ET-119** | m/z=677.79 (C₄₉H₃₁N₃O=677.25) |
| **ET-133** | m/z=626. 70 (C₄₄H₂₆N₄O=626.21) | **ET-134** | m/z=626. 70 (C₄₄H₂₆N₄O=626.21) |
| **ET-149** | m/z=650.76 (C₄₈H₃()N₂O=650.24) | **ET-150** | m/z=650.76 (C₄₈H₃()N₂O=650.24) |
| **ET-157** | m/z=727. 85 (C₅₃H₃₃N₃O=727.26) | **ET-166** | m/z=751.87 (C₅₅H₃₃N₃O=751.26) |
| **ET-167** | m/z=751.87 (C₅₅H₃₃N₃O=751.26) | **ET-168** | m/z=827.97 (C₆₁H₃₇N₃O=827.29) |
| **ET-169** | m/z=827.97 (C₆₁H₃₇N₃O=827.29) | **ET-171** | m/z=750.88 (C₅₆H₃₄N₂O=750.27) |
| **ET-175** | m/z=774.90 (C₅₈H₃₄N₂O=774.27) | **ET-182** | m/z=706.85 (C₅₀H₃₀N₂OS=706.21) |
| **ET-190** | m/z=767.87 (C₅₅H₃₃N₃O₂=767.26) | **ET-211** | m/z=677.79 (C₄₉H₃₁N₃O=677.25) |
| **ET-212** | m/z=676.80 (C₅₀H₃₂N₂O=676.25) | **ET-243** | m/z=727.85 (C₅₃H₃₃N₃O=727.26) |
| **ET-244** | m/z=726.86 (C₅₄H₃₄N₂O=726.27) | **ET-299** | m/z=803.94 (C₅₉H₃₇N₃O=803.29) |
| **ET-300** | m/z=802. 96 (C₆₀H₃₈N₂O=802.30) | **ET-303** | m/z=827.97 (C₆₁H₃₇N₃O=827.29) |
| **ET-354** | m/z=766.88 (C₅₅H₃₄N₄O=766.27) | **ET-357** | m/z=842.98 (C₆₁H₃₈N₄O=842.30) |
| **ET-358** | m/z=842.98 (C₆₁H₃₈N₄O=842.30) | **ET-361** | m/z=765.90 (C₅₆H₃₅N₃₀=765.28) |
| **ET-388** | m/z=801.87 (C₅₅H₃₆N₃O₂P=801.25) | **ET-389** | m/z=877.96 (C₆₁H₄N₃O₂P=877. 29) |
| **ET-392** | m/z=877.96 (C₆₁H₄₀N₃O₂P=877.29) | **ET-401** | m/z=672.71 (C₄₆H₂₉N₂O₂P=672.20) |
| **ET-402** | m/z=748. 80 (C₅₂H₃₃N₂O₂P=748. 23) | **ET-408** | m/z=877.96 (C₆ₗH₄()N_{302P}=877.29) |
| **ET-411** | m/z=800.88 (C₅₆H₃₇N₂O₂P=800.26) | **ET-419** | m/z=748.80 (C₅₂H₃₃N₂O₂P=748.23) |
| **ET-459** | m/z=601.69 (C₄₃H₂₇N₃O=601.22) | **ET-463** | m/z=677.79 (C₄₉H₃₁N₃O=677.25) |
| **ET-474** | m/z=601.69 (C₄₃H₂₇N₃O=601.22) | **ET-487** | m/z=548.63 (C₄₀H₂₄N₂O=548.19) |
| **ET-489** | m/z=651.75 (C₄₇H₂₉N₃O=651.23) | **ET-496** | m/z=650.76 (C₄₈H₃()N₂O=650.24) |
| **ET-511** | m/z=650.76 (C₄₈H₃₀N₂O=650.24) | **ET-517** | m/z=598.69 (C₄₄H₂₆N₂O=598.20) |
| **ET-535** | m/z=677. 79 (C₄₉H₃₁N₃O=677.25) | **ET-549** | m/z=751.87 (C₅₅H₃₃N₃O=751.26) |
| **ET-550** | m/z=751.87 (C₅₅H₃₃N₃O=751.26) | **ET-571** | m/z=750.88 (C₅₆H₃₄N₂O=750.27) |
| **ET-579** | m/z=601.69 (C₄₃H₂₇N₃O=601.22) | **ET-582** | m/z=677.79 (C₄₉H₃₁N₃O=677.25) |
| **ET-603** | m/z=676.80 (C₅₀H₃₂N₂O=676.25) | **ET-615** | m/z=727.85 (C₅₃H₃₃N₃O=727.26) |
| **ET-639** | m/z=677. 79 (C₄₉H₃₁N₃O=677.25) | **ET-640** | m/z=677.79 (C₄₉H₃₁N₃O=677.25) |
| **ET-652** | m/z=624.73 (C₄₆H₂₈N₂O=624.22) | **ET-661** | m/z=676.80 (C₅₀H₃₂N₂O=676.25) |
| **ET-699** | m/z=601.69 (C₄₃H₂₇N₃O=601.22) | **ET-712** | m/z=548.63 (C₄₀H₂₄N₂O=548.19) |
| **ET-729** | m/z=651.75 (C₄₇H₂₉N₃O=651.23) | **ET-744** | m/z=651.75 (C₄₇H₂₉N₃O=651. 23) |
| **ET-752** | m/z=650.76 (C₄₈H₃()N₂O=650.24) | **ET-765** | m/z=753.89 (C₅₅H₃₅N₃O=753.28) |
| **ET-774** | m/z=677.79 (C₄₉H₃₁N₃O=677.25) | **ET-781** | m/z=676.80 (C₅₀H₃₂N₂O=676.25) |

### <Experimental Example 1> Manufacture of Organic Light Emitting Device

### <Comparative Examples 1-1 and 1-2 and Examples 1 to 112>

### 1) Manufacture of Organic Light Emitting Device

A transparent ITO electrode thin film obtained from glass for an organic light emitting device (manufactured by Samsung-Corning Co., Ltd.) was ultrasonic cleaned using trichloroethylene, acetone, ethanol and distilled water consecutively for 5 minutes each, stored in isopropanol, and used.

Next, an ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

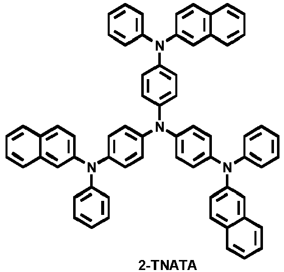

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate.

To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

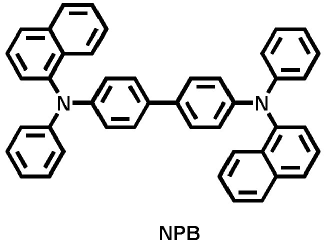

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

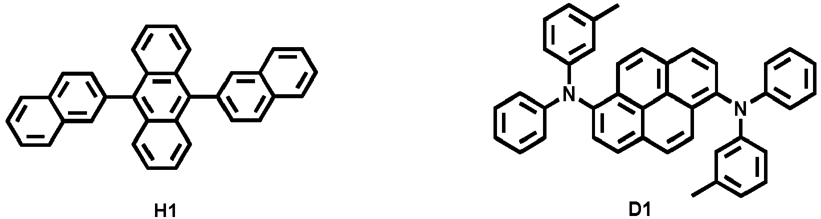

Subsequently, the following Compound E1 and LiQ were vacuum deposited in a weight ratio of 2:1 to a thickness of 300 Å to form an electron transfer layer.

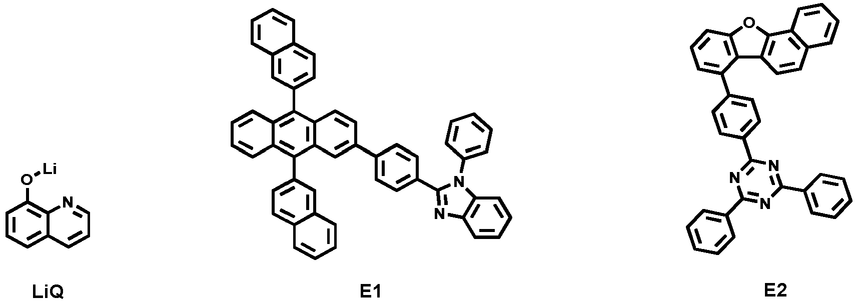

As an electron injection layer, lithium fluoride (LiF) was deposited to a thickness of 10 Å, and an A1 cathode was employed to a thickness of 1,000 Å, and as a result, an organic light emitting device (Comparative Example 1-1) was manufactured.

An organic light emitting device (Comparative Example 1-2) was manufactured in the same manner as in the above-described manufacturing process except that Compound E2 was used instead of Compound E1.

In addition, organic light emitting devices (Examples 1 to 112) were manufactured in the same manner as in the above-described manufacturing process except that one of Compound ET-001 and the target compounds of Table 2 was selected and used instead of Compound E1. The compound selected from among Compound ET-001 and the target compounds of Table 2 is shown in the following Table 5.

Meanwhile, all the organic compounds required to manufacture the organic light emitting device were vacuum sublimation purified under 10⁻⁶ torr to 10⁻⁸ torr by each material to be used in the organic light emitting device manufacture.

### 2) Driving Voltage and Light Emission Efficiency of Organic Light Emitting Device

For each of the organic light emitting devices of Comparative Example 1-1, Comparative Example 1-2 and Examples 1 to 112 manufactured as above, electroluminescent (EL) properties were measured using M7000 manufactured by McScience Inc., and with the measurement results, a lifetime T₉₅ (unit: h, time) that is a time taken to become 95% with respect to initial luminance was measured when standard luminance was 700 cd/m² through a lifetime measurement system (M6000) manufactured by McScience Inc..

Results of measuring the electroluminescent properties, color coordinate and lifetime of Comparative Example 1-1, Comparative Example 1-2 and Examples 1 to 112 are as shown in the following Table 5.

**[Table 5]**

| | **Compound** | **Driving Voltage (V)** | **Light Emission Efficiency (cd/A)** | **CIE (x, y)** | **Lifetime (T₉₅)** |
|---|---|---|---|---|---|
| **Example 1** | ET-001 | 4.92 | 7.11 | (0.134, 0.101) | 73 |
| **Example 2** | ET-002 | 4.89 | 7.26 | (0.134, 0.102) | 52 |
| **Example 3** | ET-003 | 5.14 | 6.79 | (0.134, 0.101) | 61 |
| **Example 4** | ET-004 | 4.99 | 7.25 | (0.134, 0.103) | 71 |
| **Example 5** | ET-005 | 5.72 | 6.12 | (0.134, 0.102) | 50 |
| **Example 6** | ET-006 | 5.43 | 6.49 | (0.134, 0.101) | 61 |
| **Example 7** | ET-007 | 5.72 | 6.92 | (0.134, 0.102) | 59 |
| **Example 8** | ET-008 | 5.32 | 6.33 | (0.134, 0.101) | 59 |
| **Example 9** | ET-009 | 5.40 | 6.13 | (0.134, 0.101) | 62 |
| **Example 10** | ET-010 | 5.30 | 6.72 | (0.134, 0.100) | 67 |
| **Example 11** | ET-011 | 5.37 | 6.35 | (0.134, 0.101) | 54 |
| **Example 12** | ET-012 | 5.38 | 6.41 | (0.134, 0.100) | 55 |
| **Example 13** | ET-013 | 5.27 | 6.42 | (0.134, 0.100) | 60 |
| **Example 14** | ET-016 | 5.48 | 6.21 | (0.134, 0.100) | 59 |
| **Example 15** | ET-019 | 5.36 | 6.11 | (0.134, 0.100) | 47 |
| **Example 16** | ET-022 | 5.45 | 6.68 | (0.134, 0.100) | 61 |
| **Example 17** | ET-034 | 5.22 | 6.28 | (0.134, 0.102) | 44 |
| **Example 18** | ET-036 | 5.12 | 6.20 | (0.134, 0.101) | 58 |
| **Example 19** | ET-041 | 5.09 | 6.77 | (0.134, 0.102) | 61 |
| **Example 20** | ET-042 | 5.42 | 6.88 | (0.134, 0.100) | 63 |
| **Example 21** | ET-047 | 5.21 | 5.45 | (0.134, 0.103) | 59 |
| **Example 22** | ET-048 | 5.38 | 6.66 | (0.134, 0.100) | 62 |
| **Example 23** | ET-052 | 5.40 | 6.36 | (0.134, 0.100) | 63 |
| **Example 24** | ET-053 | 5.56 | 5.91 | (0.134, 0.100) | 58 |
| **Example 25** | ET-056 | 4.78 | 7.64 | (0.134, 0.100) | 80 |
| **Example 26** | ET-057 | 5.19 | 7.15 | (0.134, 0.100) | 82 |
| **Example 27** | ET-058 | 5.42 | 6.88 | (0.134, 0.100) | 62 |
| **Example 28** | ET-059 | 5.42 | 6.56 | (0.134, 0.102) | 65 |
| **Example 29** | ET-060 | 5.46 | 6.81 | (0.134, 0.101) | 62 |
| **Example 30** | ET-062 | 5.30 | 6.72 | (0.134, 0.102) | 63 |
| **Example 31** | ET-067 | 5.37 | 6.35 | (0.134, 0.100) | 58 |
| **Example 32** | ET-068 | 5.42 | 6.34 | (0.134, 0.100) | 59 |
| **Example 33** | ET-069 | 5.49 | 6.68 | (0.134, 0.100) | 63 |
| **Example 34** | ET-070 | 5.42 | 6.77 | (0.134, 0.100) | 62 |
| **Example 35** | ET-072 | 5.56 | 6.18 | (0.134, 0.100) | 45 |
| **Example 36** | ET-080 | 5.12 | 6.20 | (0.134, 0.100) | 59 |
| **Example 37** | ET-083 | 5.09 | 6.97 | (0.134, 0.100) | 64 |
| **Example 38** | ET-091 | 5.41 | 6.76 | (0.134, 0.100) | 60 |
| **Example 39** | ET-092 | 5.21 | 5.45 | (0.134, 0.100) | 56 |
| **Example 40** | ET-098 | 5.42 | 6.88 | (0.134, 0.102) | 56 |
| **Example 41** | ET-100 | 4.86 | 7.84 | (0.134, 0.101) | 78 |
| **Example 42** | ET-101 | 5.14 | 6.79 | (0.134, 0.102) | 57 |
| **Example 43** | ET-102 | 5.45 | 6.12 | (0.134, 0.102) | 65 |
| **Example 44** | ET-103 | 4.73 | 7.49 | (0.134, 0.101) | 69 |
| **Example 45** | ET-105 | 5.45 | 6.12 | (0.134, 0.102) | 65 |
| **Example 46** | ET-106 | 5.43 | 6.49 | (0.134, 0.101) | 61 |
| **Example 47** | ET-107 | 5.72 | 6.92 | (0.134, 0.102) | 59 |
| **Example 48** | ET-108 | 5.32 | 6.33 | (0.134, 0.101) | 59 |
| **Example 49** | ET-109 | 5.40 | 6.13 | (0.134, 0.101) | 62 |
| **Example 50** | ET-110 | 5.30 | 6.72 | (0.134, 0.100) | 67 |
| **Example 51** | ET-114 | 5.37 | 6.35 | (0.134, 0.101) | 54 |
| **Example 52** | ET-119 | 5.38 | 6.41 | (0.134, 0.100) | 55 |
| **Example 53** | ET-133 | 5.27 | 6.42 | (0.134, 0.100) | 60 |
| **Example 54** | ET-134 | 5.71 | 5.42 | (0.134, 0.100) | 42 |
| **Example 55** | ET-149 | 4.72 | 7.39 | (0.134, 0.100) | 68 |
| **Example 56** | ET-150 | 5.45 | 6.68 | (0.134, 0.100) | 61 |
| **Example 57** | ET-157 | 5.22 | 6.28 | (0.134, 0.102) | 44 |
| **Example 58** | ET-166 | 5.12 | 6.20 | (0.134, 0.101) | 58 |
| **Example 59** | ET-167 | 5.09 | 6.77 | (0.134, 0.102) | 61 |
| **Example 60** | ET-168 | 5.42 | 6.88 | (0.134, 0.100) | 63 |
| **Example 61** | ET-169 | 5.21 | 5.45 | (0.134, 0.103) | 59 |
| **Example 62** | ET-171 | 5.38 | 6.66 | (0.134, 0.100) | 62 |
| **Example 63** | ET-175 | 5.40 | 6.36 | (0.134, 0.100) | 63 |
| **Example 64** | ET-182 | 5.56 | 5.91 | (0.134, 0.100) | 58 |
| **Example 65** | ET-190 | 5.14 | 6.99 | (0.134, 0.100) | 80 |
| **Example 66** | ET-211 | 5.19 | 7.15 | (0.134, 0.100) | 82 |
| **Example 67** | ET-212 | 5.42 | 6.88 | (0.134, 0.100) | 62 |
| **Example 68** | ET-243 | 5.42 | 6.56 | (0.134, 0.102) | 65 |
| **Example 69** | ET-244 | 5.46 | 6.81 | (0.134, 0.101) | 62 |
| **Example 70** | ET-299 | 5.30 | 6.72 | (0.134, 0.102) | 63 |
| **Example 71** | ET-300 | 5.37 | 6.35 | (0.134, 0.100) | 58 |
| **Example 72** | ET-303 | 5.42 | 6.34 | (0.134, 0.100) | 59 |
| **Example 73** | ET-354 | 5.49 | 6.68 | (0.134, 0.100) | 63 |
| **Example 74** | ET-357 | 5.42 | 6.77 | (0.134, 0.100) | 62 |
| **Example 75** | ET-358 | 5.56 | 6.18 | (0.134, 0.100) | 45 |
| **Example 76** | ET-361 | 5.12 | 6.20 | (0.134, 0.100) | 59 |
| **Example 77** | ET-388 | 5.99 | 5.57 | (0.134, 0.100) | 94 |
| **Example 78** | ET-389 | 6.03 | 5.34 | (0.134, 0.100) | 89 |
| **Example 79** | ET-392 | 5.91 | 5.25 | (0.134, 0.100) | 87 |
| **Example 80** | ET-401 | 5.42 | 5.88 | (0.134, 0.102) | 56 |
| **Example 81** | ET-402 | 5.96 | 5.84 | (0.134, 0.101) | 57 |
| **Example 82** | ET-408 | 5.94 | 5.19 | (0.134, 0.102) | 82 |
| **Example 83** | ET-411 | 5.91 | 4.96 | (0.134, 0.100) | 91 |
| **Example 84** | ET-419 | 5.71 | 5.45 | (0.134, 0.100) | 60 |
| **Example 85** | ET-459 | 5.21 | 7.14 | (0.134, 0.100) | 83 |
| **Example 86** | ET-463 | 5.86 | 5.84 | (0.134, 0.101) | 57 |
| **Example 87** | ET-474 | 5.73 | 6.03 | (0.134, 0.102) | 59 |
| **Example 88** | ET-487 | 5.66 | 5.98 | (0.134, 0.101) | 55 |
| **Example 89** | ET-489 | 5.22 | 6.23 | (0.134, 0.102) | 60 |
| **Example 90** | ET-496 | 5.11 | 6.33 | (0.134, 0.100) | 54 |
| **Example 91** | ET-511 | 5.32 | 6.43 | (0.134, 0.100) | 52 |
| **Example 92** | ET-517 | 5.53 | 5.89 | (0.134, 0.100) | 59 |
| **Example 93** | ET-535 | 5.42 | 6.23 | (0.134, 0.100) | 63 |
| **Example 94** | ET-549 | 5.32 | 6.42 | (0.134, 0.100) | 55 |
| **Example 95** | ET-550 | 5.30 | 6.52 | (0.134, 0.100) | 49 |
| **Example 96** | ET-571 | 5.33 | 6.43 | (0.134, 0.101) | 50 |
| **Example 97** | ET-579 | 5.33 | 6.32 | (0.134, 0.102) | 55 |
| **Example 98** | ET-582 | 5.35 | 6.43 | (0.134, 0.102) | 57 |
| **Example 99** | ET-603 | 5.38 | 6.54 | (0.134, 0.100) | 51 |
| **Example 100** | ET-615 | 5.33 | 6.34 | (0.134, 0.100) | 62 |
| **Example 101** | ET-639 | 5.44 | 6.25 | (0.134, 0.100) | 60 |
| **Example 102** | ET-640 | 5.43 | 6.32 | (0.134, 0.100) | 59 |
| **Example 103** | ET-652 | 5.87 | 5.78 | (0.134, 0.100) | 49 |
| **Example 104** | ET-661 | 5.32 | 6.23 | (0.134, 0.101) | 63 |
| **Example 105** | ET-699 | 5.22 | 6.89 | (0.134, 0.102) | 79 |
| **Example 106** | ET-712 | 5.45 | 6.11 | (0.134, 0.102) | 58 |
| **Example 107** | ET-729 | 5.38 | 6.54 | (0.134, 0.100) | 51 |
| **Example 108** | ET-744 | 5.38 | 6.66 | (0.134, 0.100) | 62 |
| **Example 109** | ET-752 | 5.40 | 6.36 | (0.134, 0.100) | 63 |
| **Example 110** | ET-765 | 5.56 | 5.91 | (0.134, 0.100) | 58 |
| **Example 111** | ET-774 | 5.45 | 6.68 | (0.134, 0.100) | 61 |
| **Example 112** | ET-781 | 5.22 | 6.28 | (0.134, 0.102) | 44 |
| **Comparative Example 1-1** | E1 | 5.55 | 6.12 | (0.134, 0.100) | 54 |
| **Comparative Example 1-2** | E2 | 5.49 | 6.11 | (0.134, 0.101) | 56 |

From the results of Table 5, it was identified that the organic light emitting device using the electron transfer layer material of the blue organic light emitting device of the present disclosure had lower driving voltage and significantly improved light emission efficiency and lifetime compared to Comparative Example 1.

Particularly, it was identified that the organic light emitting devices of Examples 1, 5, 25, 41, 44, 55, 65, 66, 85 and 105 using Compound ET-001. ET-005, ET-056, ET-100, ET-103, ET-149, ET-190, ET-211, ET-459 and ET-699 in the electron transfer layer had more improved driving, efficiency and lifetime, and were superior in all aspects. This is considered to be due to the fact that, unlike Comparative Example 1-2, HOMO, LUMO, electron mobility and the like may be adjusted by introducing two substituents, and improvements in terms of driving, efficiency and lifetime are obtained by improving a balance between electrons and holes in the light emitting layer when manufacturing the device.

Such a result is considered to be due to the fact that, when using the disclosed compound having proper length and strength, and flatness as an electron transfer layer, a compound in an excited state is made by receiving electrons under a specific condition, and particularly when an excited state is formed in the hetero-skeleton site of the compound, excited energy moves to a stable state before the excited hetero-skeleton site goes through other reactions, and as a result, the relatively stabilized compound is capable of efficiently transferring electrons without the compound being decomposed or destroyed. For reference, those that are stable when excited are considered to be aryl or acene-based compounds or polycyclic hetero-compounds. Accordingly, it is considered that excellent results in all aspects of driving, efficiency and lifetime were obtained by the compound of the present disclosure enhancing enhanced electron-transfer properties or improved stability.

### <Experimental Example 2> Manufacture of Organic Light Emitting Device

### <Comparative Example 2>

### 1) Manufacture of Organic Light Emitting Device

A transparent ITO electrode thin film obtained from glass for an organic light emitting device (manufactured by Samsung-Corning Co., Ltd.) was ultrasonic cleaned using trichloroethylene, acetone, ethanol and distilled water consecutively for 5 minutes each, stored in isopropanol, and used.

Next, an ITO substrate was installed in a substrate folder of a vacuum deposition apparatus, and the following 4,4',4"-tris(N,N-(2-naphthyl)-phenylamino)triphenylamine (2-TNATA) was deposited on a cell in the vacuum deposition apparatus.

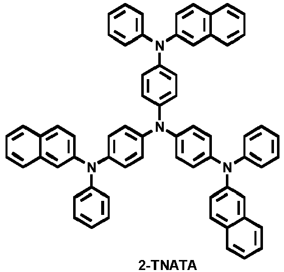

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate.

To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

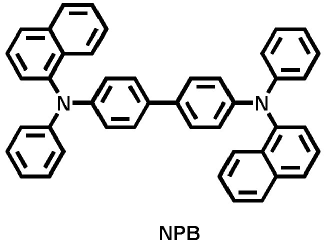

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

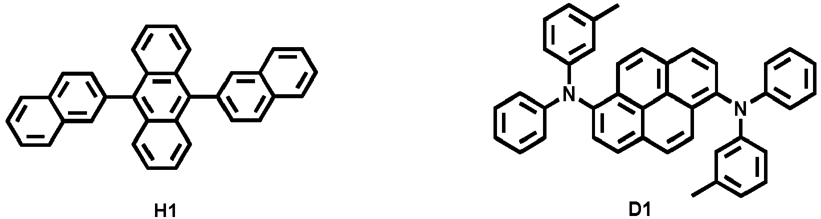

Subsequently, the following Structural Formula E1 and LiQ were vacuum deposited in a weight ratio of 2:1 to a thickness of 300 Å to form an electron transfer layer.

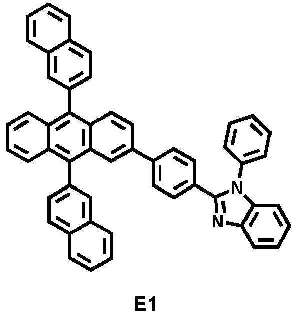

As an electron injection layer, lithium fluoride (LiF) was deposited to a thickness of 10 Å, and an A1 cathode was employed to a thickness of 1,000 Å, and as a result, an organic light emitting device (Comparative Example 2) was manufactured.

In addition, organic light emitting devices (Examples 113 to 133) were manufactured in the same manner as in the above-described manufacturing process except that, after forming an electron transfer layer to a thickness of 250 Å instead of 300 Å, a hole blocking layer having a thickness of 50 Å was formed by selecting one of Compound ET-001 and the target compounds of Table 2. The compound selected from among Compound ET-001 and the target compounds of Table 2 is shown in the following Table 6.

Meanwhile, all the organic compounds required to manufacture the organic light emitting device were vacuum sublimation purified under 10⁻⁶ torr to 10⁻⁸ torr by each material to be used in the organic light emitting device manufacture.

### 2) Driving Voltage and Light Emission Efficiency of Organic Light Emitting Device

For each of the organic light emitting devices of Comparative Example 2 and Examples 113 to 133 manufactured as above, electroluminescent (EL) properties were measured using M7000 manufactured by McScience Inc., and with the measurement results, a lifetime T₉₅ (unit: h, time) that is a time taken to become 95% with respect to initial luminance was measured when standard luminance was 700 cd/m² through a lifetime measurement system (M6000) manufactured by McScience Inc..

Results of measuring the electroluminescent properties, color coordinate and lifetime of the organic light emitting devices Comparative Example 2 and Examples 113 to 133 are as shown in the following Table 6.

**[Table 6]**

| | **Compound** | **Driving Voltage (V)** | **Light Emission Efficiency (cd/A)** | **CIE (x, y)** | **Lifetime (T₉₅)** |
|---|---|---|---|---|---|
| **Example 113** | ET-001 | 4.87 | 6.27 | (0.134, 0.101) | 90 |
| **Example 114** | ET-047 | 4.73 | 6.57 | (0.134, 0.102) | 92 |
| **Example 115** | ET-057 | 5.11 | 6.55 | (0.134, 0.101) | 95 |
| **Example 116** | ET-067 | 4.77 | 6.53 | (0.134, 0.103) | 89 |
| **Example 117** | ET-068 | 4.87 | 6.27 | (0.134, 0.101) | 90 |
| **Example 118** | ET-080 | 4.73 | 6.57 | (0.134, 0.102) | 92 |
| **Example 119** | ET-166 | 5.11 | 6.55 | (0.134, 0.101) | 95 |
| **Example 120** | ET-169 | 4.87 | 6.27 | (0.134, 0.101) | 90 |
| **Example 121** | ET-171 | 4.73 | 6.57 | (0.134, 0.102) | 92 |
| **Example 122** | ET-354 | 5.11 | 6.55 | (0.134, 0.101) | 95 |
| **Example 123** | ET-463 | 5.11 | 5.59 | (0.134, 0.101) | 60 |
| **Example 124** | ET-489 | 4.73 | 6.57 | (0.134, 0.102) | 70 |
| **Example 125** | ET-535 | 5.21 | 5.55 | (0.134, 0.101) | 95 |
| **Example 126** | ET-549 | 4.77 | 6.53 | (0.134, 0.103) | 89 |
| **Example 127** | ET-550 | 4.87 | 6.27 | (0.134, 0.101) | 90 |
| **Example 128** | ET-571 | 4.73 | 6.57 | (0.134, 0.102) | 92 |
| **Example 129** | ET-603 | 5.11 | 6.55 | (0.134, 0.101) | 95 |
| **Example 130** | ET-699 | 5.27 | 6.13 | (0.134, 0.103) | 79 |
| **Example 131** | ET-729 | 5.17 | 6.27 | (0.134, 0.101) | 90 |
| **Example 132** | ET-744 | 5.23 | 6.37 | (0.134, 0.102) | 84 |
| **Example 133** | ET-752 | 5.11 | 6.55 | (0.134, 0.101) | 95 |
| **Comparative Example 2** | - | 5.50 | 5.57 | (0.134, 0.100) | 50 |

From the results of Table 6, it was identified that the organic light emitting device using the hole blocking layer material of the blue organic electroluminescent device had lower driving voltage and significantly improved light emission efficiency and lifetime compared to Comparative Example 2.

### <Experimental Example 3> Manufacture of Organic Light Emitting Device

### <Comparative Example 3 and Examples 134 to 148>

### 1) Manufacture of Organic Light Emitting Device

A transparent ITO electrode thin film obtained from glass for an organic light emitting device (manufactured by Samsung-Corning Co., Ltd.) was ultrasonic cleaned using trichloroethylene, acetone, ethanol and distilled water consecutively for 5 minutes each, stored in isopropanol, and used.

On the transparent ITO electrode (anode), an organic material was formed in a 2-stack white organic light emitting device (WOLED) structure having a structure of a first stack and a second stack. As for the first stack, TAPC was thermal vacuum deposited first to a thickness of 300 Å to form a hole transfer layer. After forming the hole transfer layer, a light emitting layer was thermal vacuum deposited thereon as follows. The light emitting layer was deposited to a thickness of 300 Å by doping FIrpic as a blue phosphorescent dopant to TCz1, a host, by 8%. After that, an electron transfer layer was formed to 400 Å using TmPyPB, and then a charge generation layer was formed to a thickness of 100 Å by doping CS₂CO₃ to TBQB by 20%.

As for the second stack, MoO₃ was thermal vacuum deposited to a thickness of 50 Å first to form a hole injection layer. A hole transfer layer, a common layer, was formed by doping MoO₃ to TAPC by 20% to a thickness of 100 Å and then depositing TAPC to a thickness of 300 Å. A light emitting layer was deposited thereon to a thickness of 300 Å by doping Ir (ppy) 3, a green phosphorescent dopant, to TCz1, a host, by 8%, and an electron transfer layer was formed to a thickness of 600 Å using TmPyPB.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device (Comparative Example 3) was manufactured.

In addition, organic light emitting devices (Examples 134 to 148) were manufactured in the same manner as in the above-described manufacturing process except that one of the target compounds of Table 2 was selected and used instead of TBQB. The compound selected from among the target compounds of Table 2 is shown in the following Table 7.

Meanwhile, all the organic compounds required to manufacture the organic light emitting device were vacuum sublimation purified under 10⁻⁶ torr to 10⁻⁸ torr by each material to be used in the organic light emitting device manufacture.

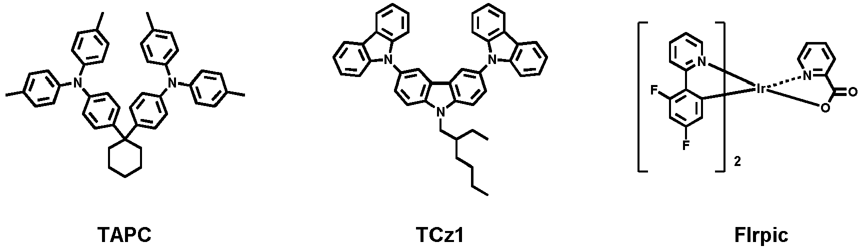

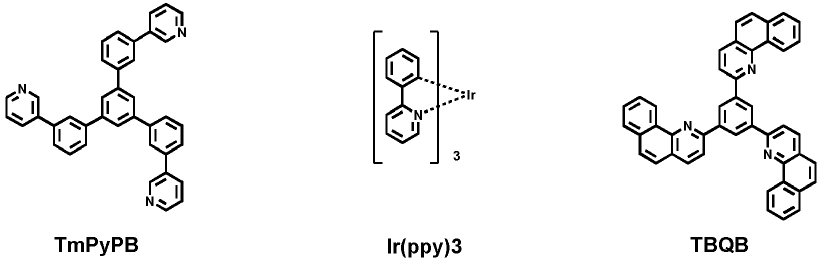

Results of measuring driving voltage, light emission efficiency, color coordinate (CIE) and lifetime of the white organic light emitting devices manufactured according to the present disclosure are as shown in Table 7.

**[Table 7]**

| | **Compound** | **Driving Voltage (V)** | **Light Emission Efficiency (cd/A)** | **CIE (x, y)** | **Lifetime (T₉₅)** |
|---|---|---|---|---|---|
| **Example 134** | ET-010 | 6.78 | 58.24 | (0.213, 0.430) | 48 |
| **Example 135** | ET-011 | 6.84 | 60.44 | (0.212, 0.421) | 42 |
| **Example 136** | ET-012 | 6.44 | 66.32 | (0.211, 0.433) | 49 |
| **Example 137** | ET-016 | 6.92 | 59.68 | (0.214, 0.439) | 52 |
| **Example 138** | ET-036 | 6.27 | 67.99 | (0.212, 0.424) | 53 |
| **Example 139** | ET-060 | 6.82 | 59.18 | (0.214, 0.437) | 48 |
| **Example 140** | ET-098 | 6.65 | 62.53 | (0.212, 0.421) | 50 |
| **Example 141** | ET-109 | 6.56 | 68.99 | (0.212, 0.432) | 49 |
| **Example 142** | ET-110 | 6.49 | 67.51 | (0.212, 0.421) | 42 |
| **Example 143** | ET-401 | 6.89 | 69.81 | (0.211, 0.431) | 52 |
| **Example 144** | ET-402 | 6.80 | 67.85 | (0.212, 0.421) | 48 |
| **Example 145** | ET-487 | 6.56 | 62.53 | (0.211, 0.431) | 43 |
| **Example 146** | ET-517 | 6.52 | 68.89 | (0.214, 0.439) | 50 |
| **Example 147** | ET-652 | 6.49 | 66.43 | (0.212, 0.424) | 49 |
| **Example 148** | ET-712 | 6.65 | 68.81 | (0.211, 0.433) | 52 |
| **Comparative Example3** | TBQB | 7.54 | 54.23 | (0.213, 0.430) | 37 |

From the results of Table 7, it was identified that the organic electroluminescent device using the charge generation layer material of the organic light emitting device having a 2-stack structure of the present disclosure had lower driving voltage and improved light emission efficiency compared to Comparative Example 3. Such a result is considered to be due to the fact that the compound of the present disclosure used as an N-type charge generation layer formed with the disclosed skeleton having proper length and strength, and flatness and a proper hetero-compound capable of binding to metals forms a gap state in the N-type charge generation layer by doping an alkali metal or an alkaline earth metal thereto, and electrons produced from a P-type charge generation layer are readily injected into an electron transfer layer through the gap state produced in the N-type charge generation layer. Accordingly, the P-type charge generation layer may favorably inject and transfer electrons to the N-type charge generation layer, and as a result, driving voltage was lowered, and efficiency and lifetime were improved in the organic light emitting device.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

## Claims

1. A heterocyclic compound represented by the following Chemical Formula 1: 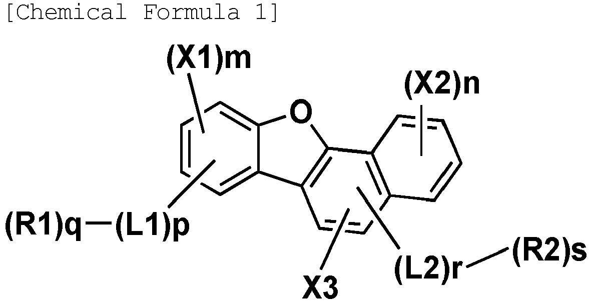 wherein, in Chemical Formula 1,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms;
R1 and R2 are the same as or different from each other, and each independently a cyano group; a substituted or unsubstituted aryl group having 6 to 40 carbon atoms; a substituted or unsubstituted heteroaryl group having 2 to 40 carbon atoms; or a substituted or unsubstituted phosphine oxide group;
X1 to X3 are the same as or different from each other, and each independently hydrogen; deuterium; or a cyano group, m is an integer of 0 to 3, n is an integer of 0 to 4, and when m and n are 2 or greater, substituents in the parentheses are the same as or different from each other;
p and r are an integer of 0 to 4;
q and s are an integer of 1 to 6; and
when p, q, r and s are 2 or greater, substituents in the parentheses are the same as or different from each other.

2. The heterocyclic compound of Claim 1, wherein the "substituted or unsubstituted" means being substituted with one or more substituents selected from the group consisting of a linear or branched alkyl group having 1 to 60 carbon atoms; a linear or branched alkenyl group having 2 to 60 carbon atoms; a linear or branched alkynyl group having 2 to 60 carbon atoms; a monocyclic or polycyclic cycloalkyl group having 3 to 60 carbon atoms; a monocyclic or polycyclic heterocycloalkyl group having 2 to 60 carbon atoms; a monocyclic or polycyclic aryl group having 6 to 60 carbon atoms; a monocyclic or polycyclic heteroaryl group having 2 to 60 carbon atoms; a silyl group; a phosphine oxide group; and an amine group, or being unsubstituted, or being substituted with a substituent linking two or more substituents selected from among the substituents illustrated above.

3. The heterocyclic compound of Claim 1, wherein Chemical Formula 1 is represented by any one of the following Chemical Formulae 3 to 6: 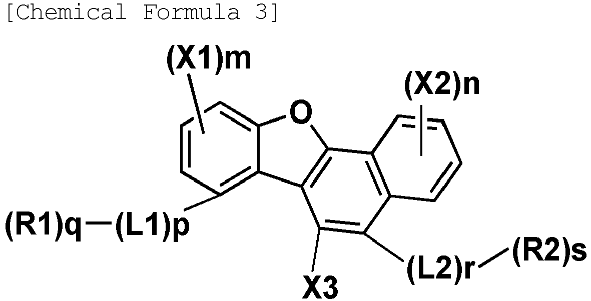 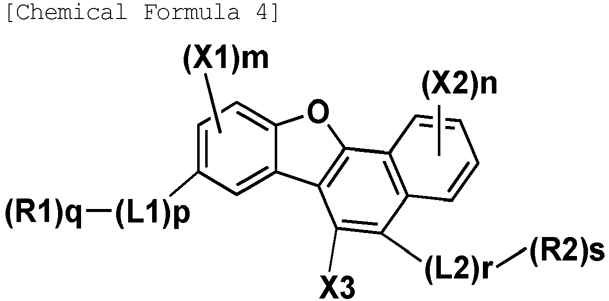 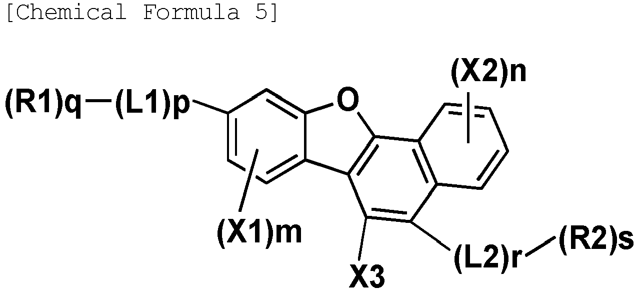 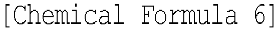 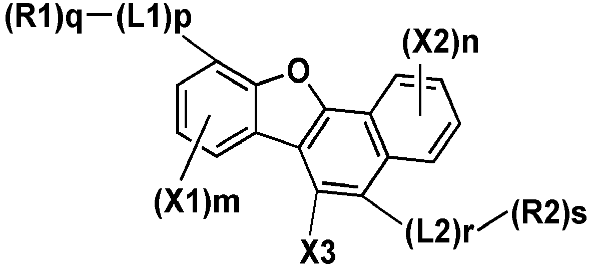 in Chemical Formula 3 to Chemical Formula 6,
each substituent has the same definition as in Chemical Formula 1.

4. The heterocyclic compound of Claim 1, wherein at least one of R1 and R2 comprises a substituted or unsubstituted N-containing heteroaryl group; and
the substituted or unsubstituted N-containing heteroaryl group is a heteroaryl group substituted or unsubstituted, and having one or more =N- bonds.

5. The heterocyclic compound of Claim 1, wherein Chemical Formula 1 is represented by any one of the following compounds:

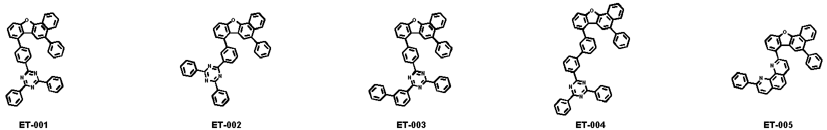

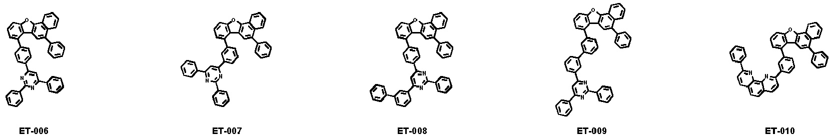

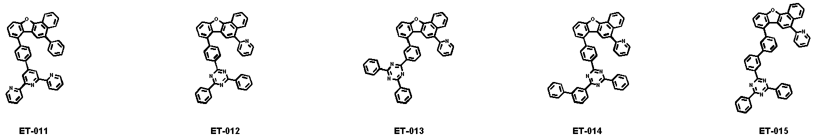

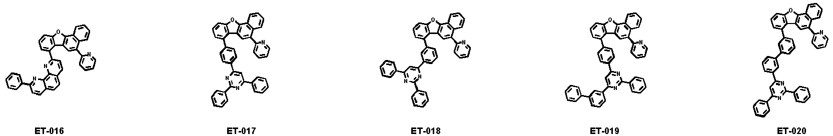

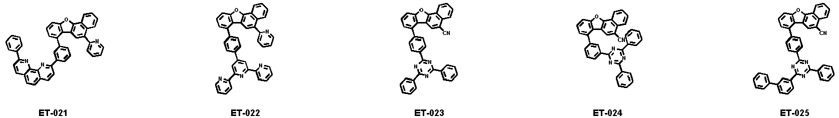

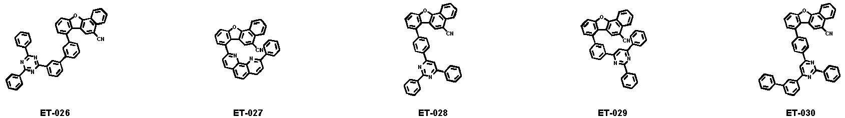

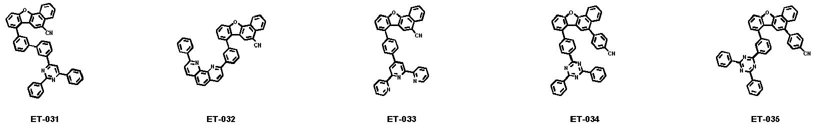

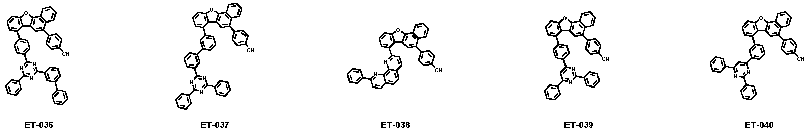

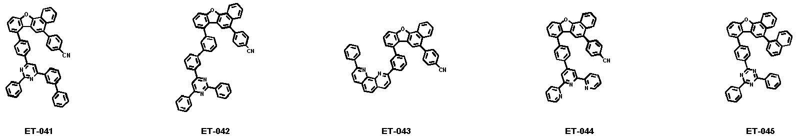

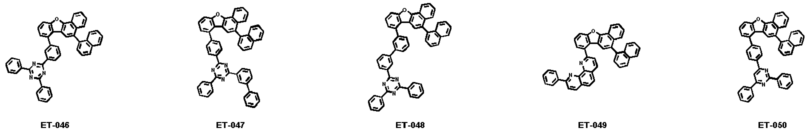

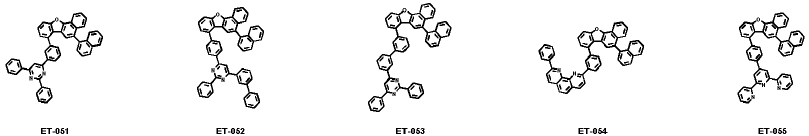

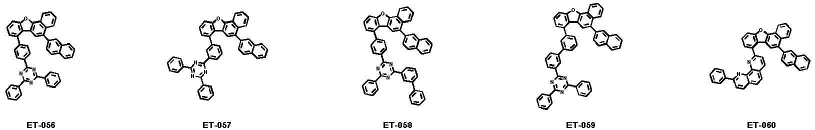

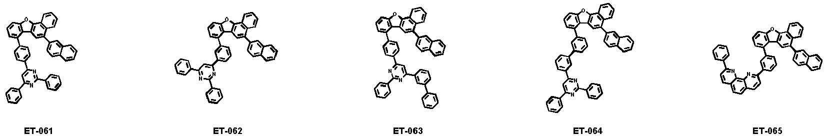

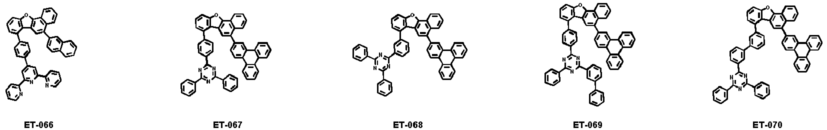

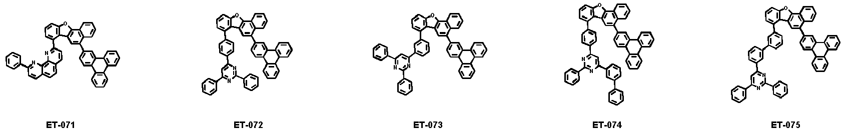

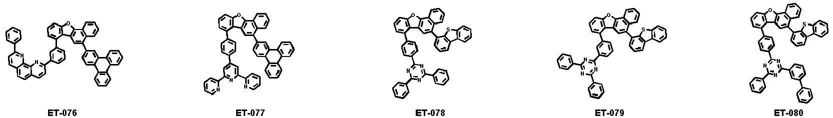

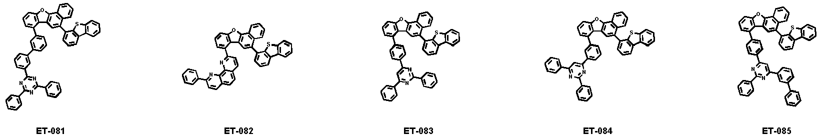

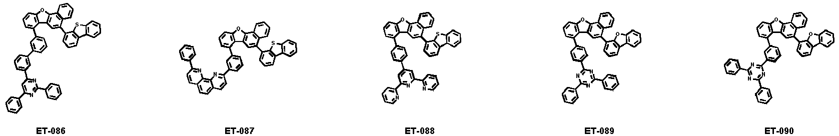

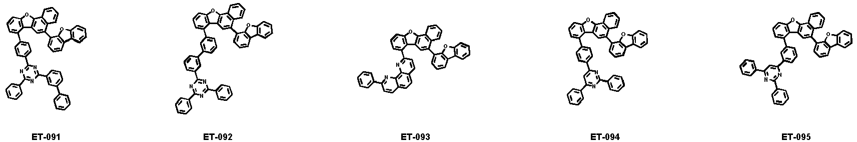

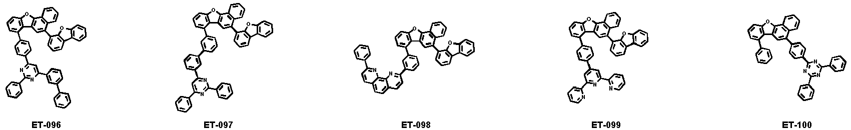

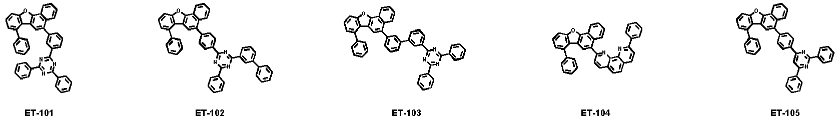

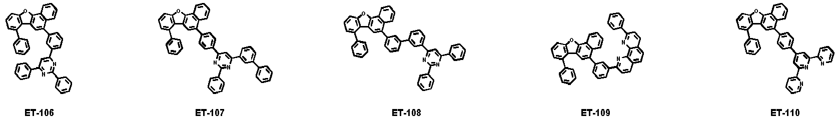

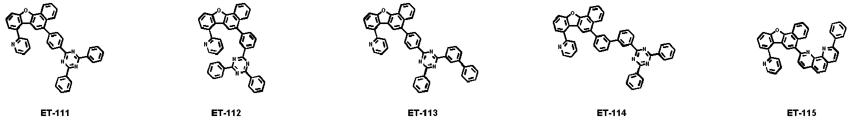

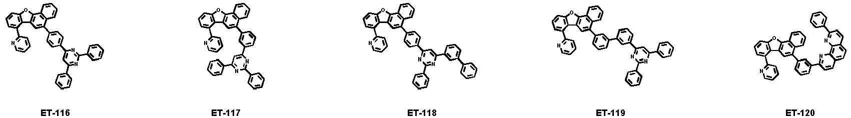

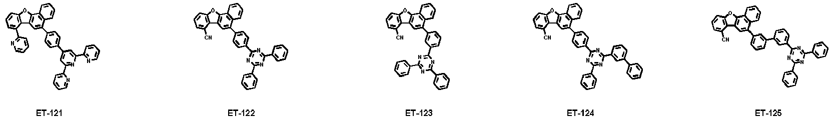

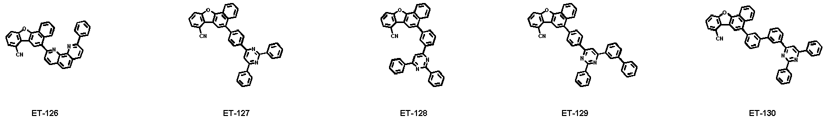

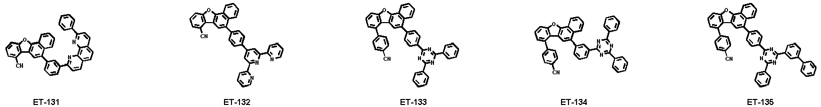

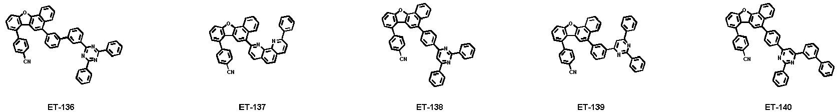

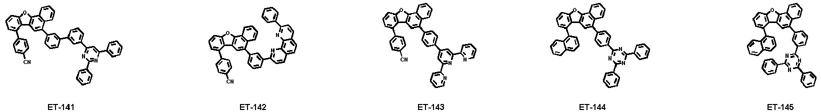

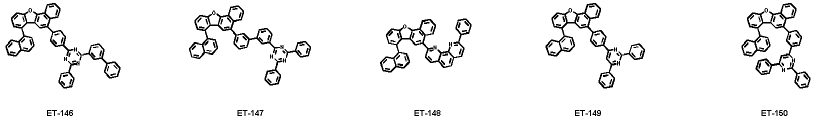

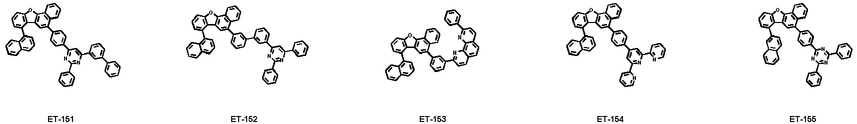

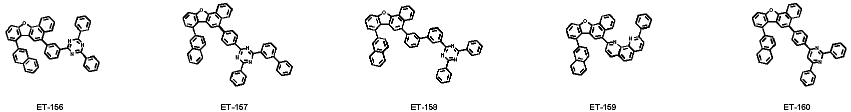

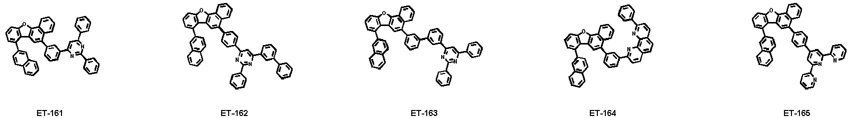

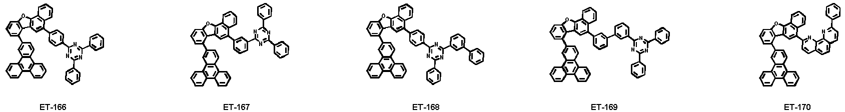

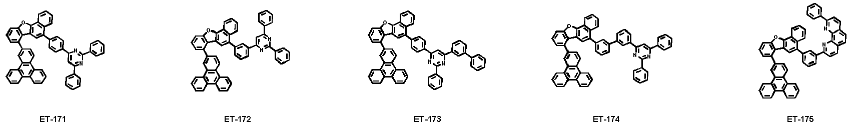

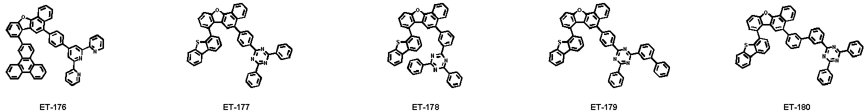

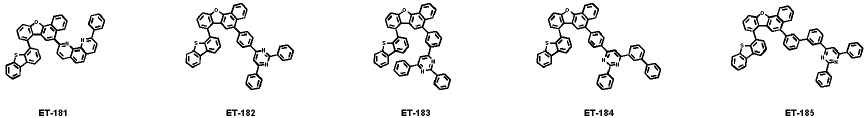

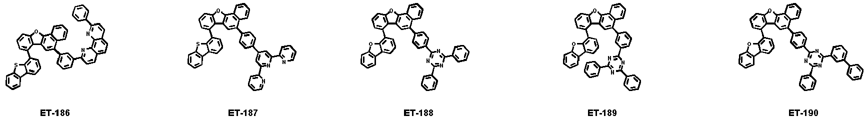

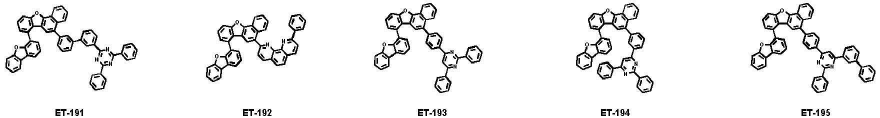

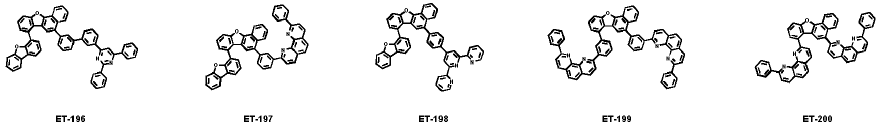

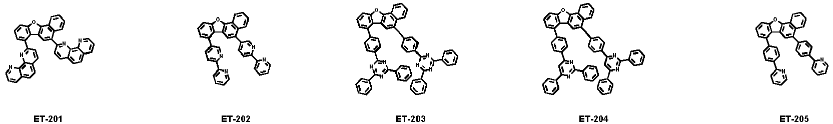

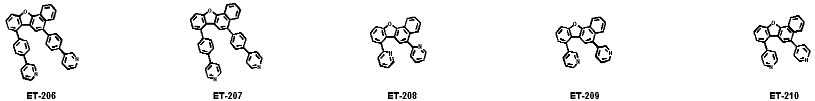

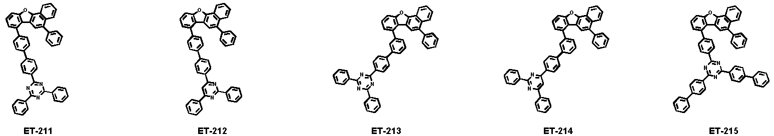

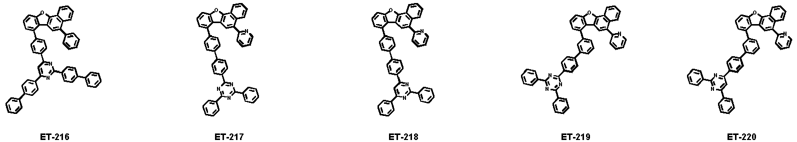

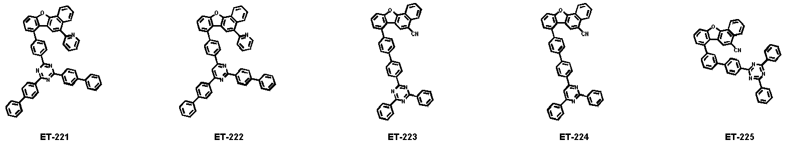

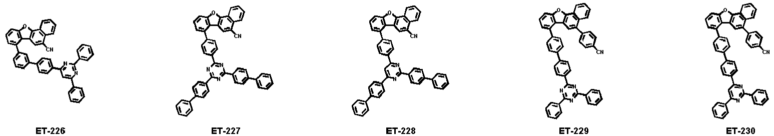

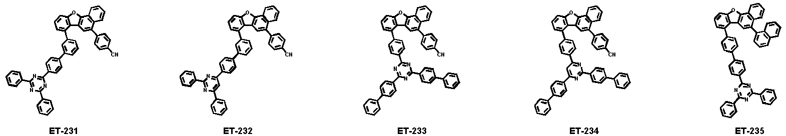

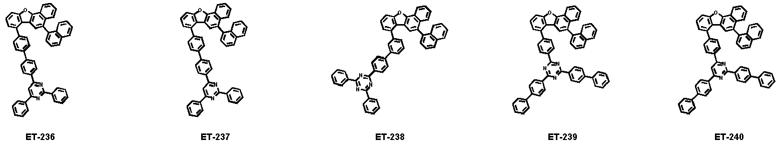

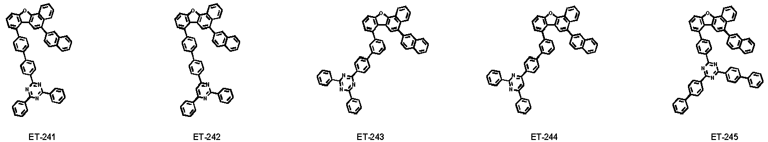

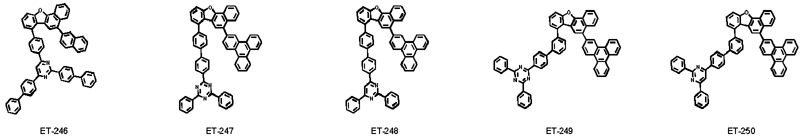

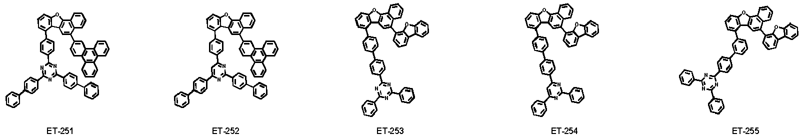

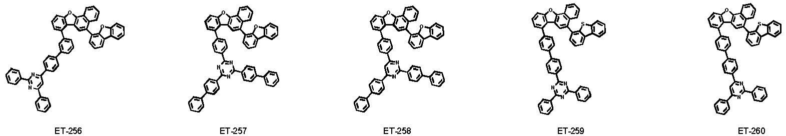

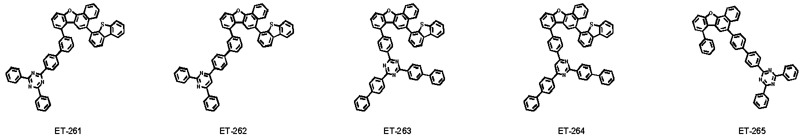

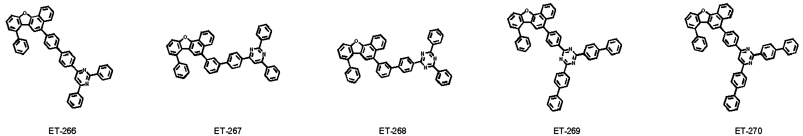

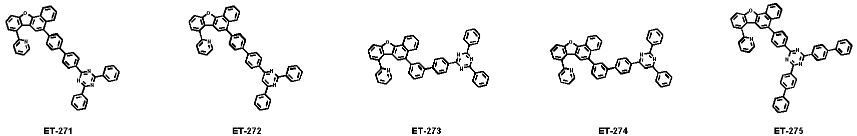

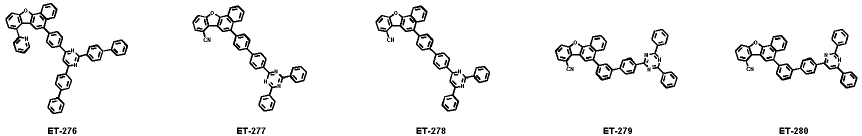

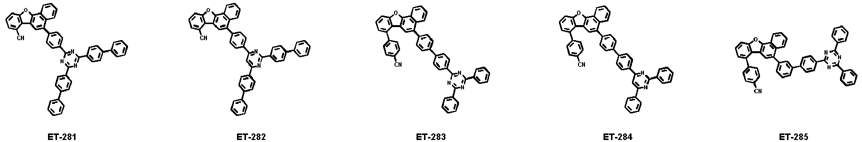

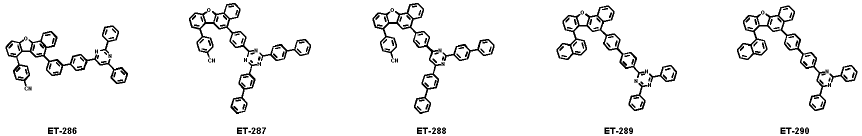

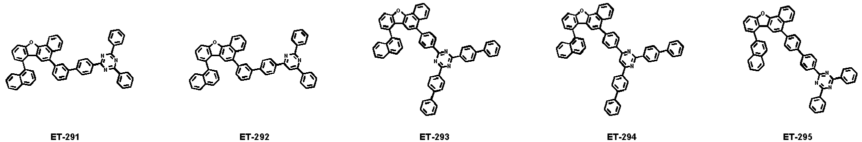

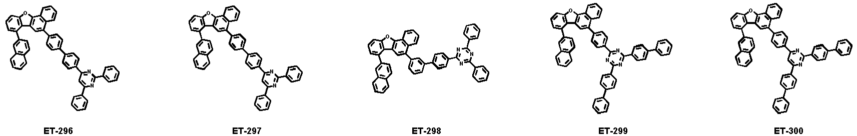

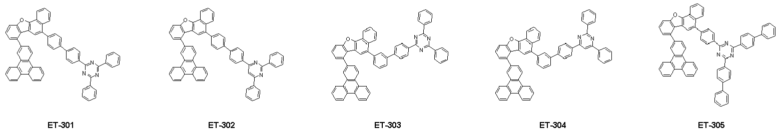

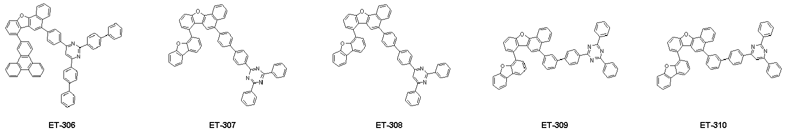

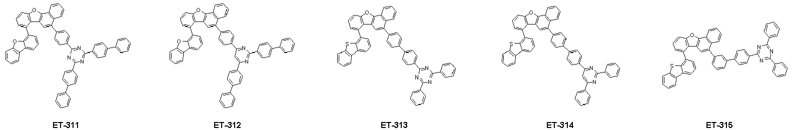

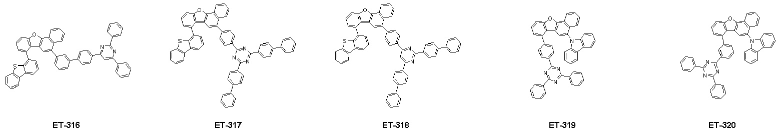

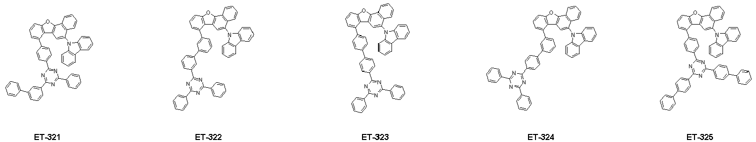

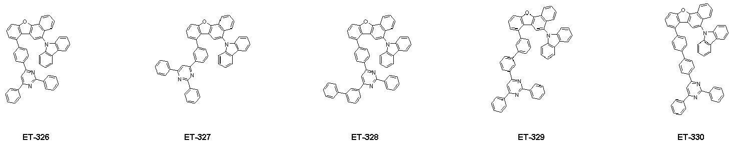

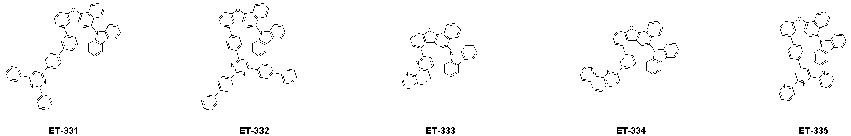

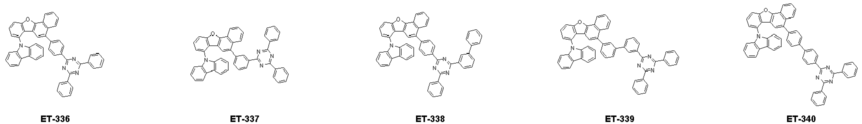

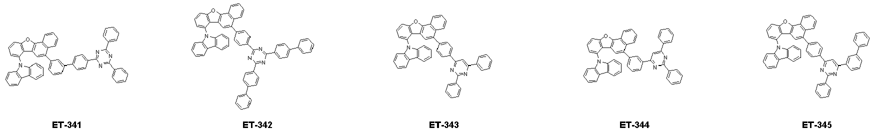

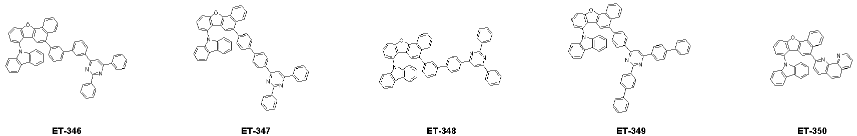

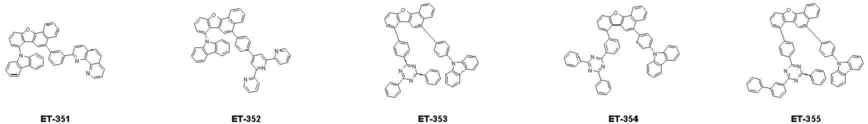

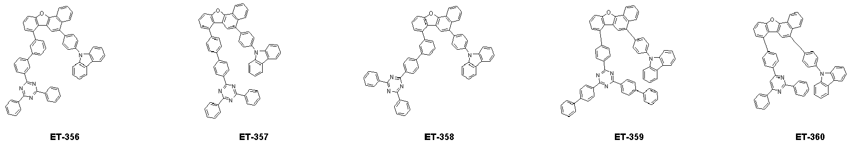

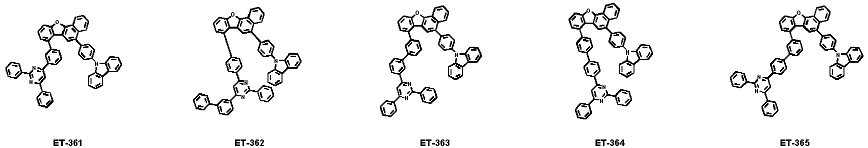

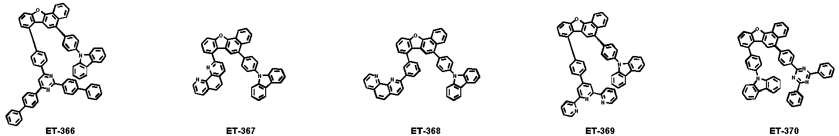

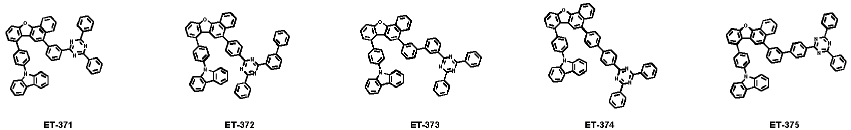

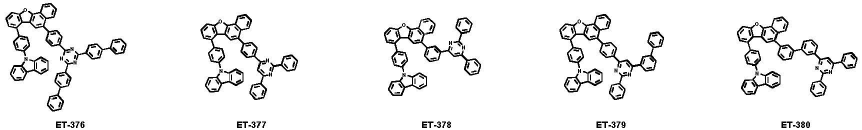

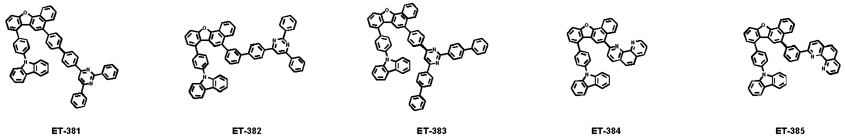

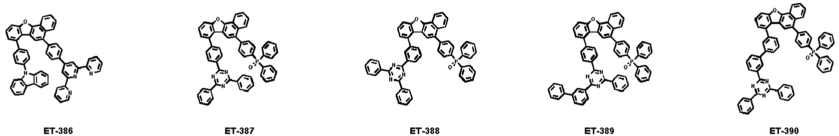

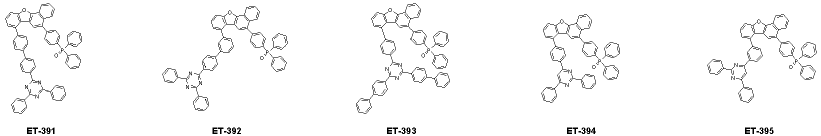

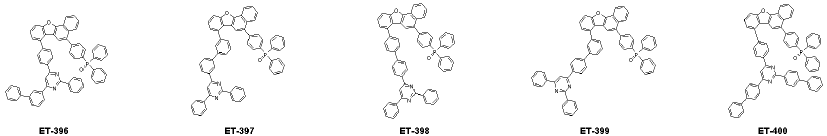

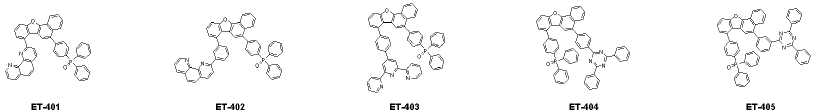

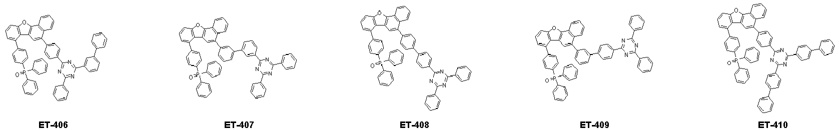

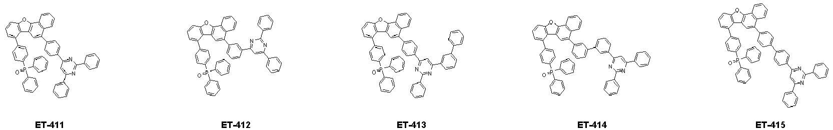

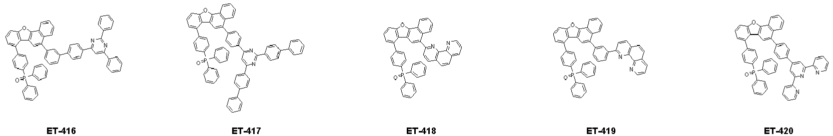

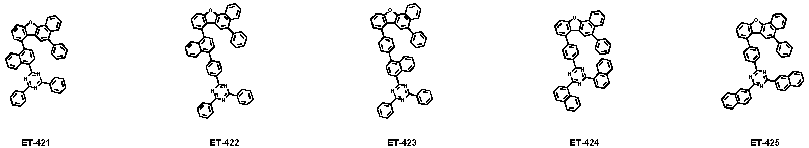

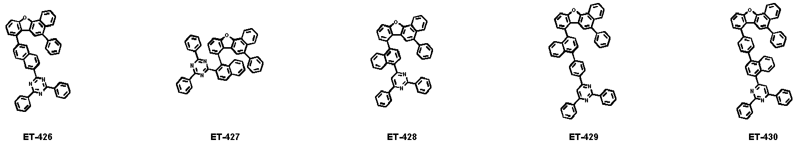

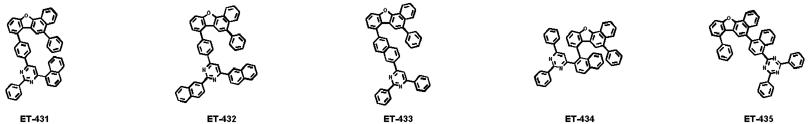

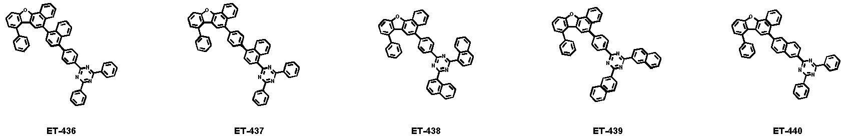

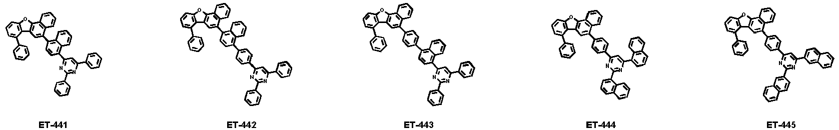

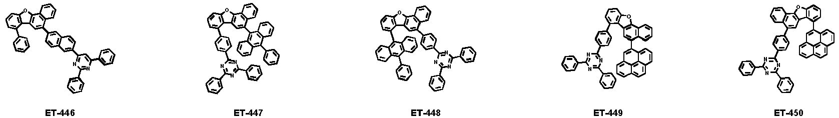

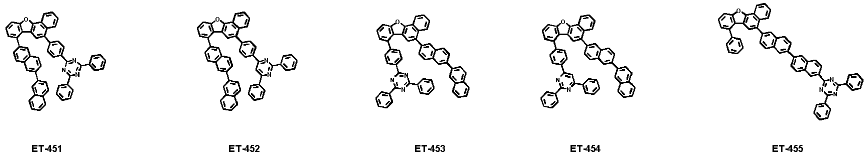

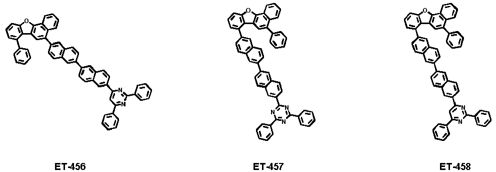

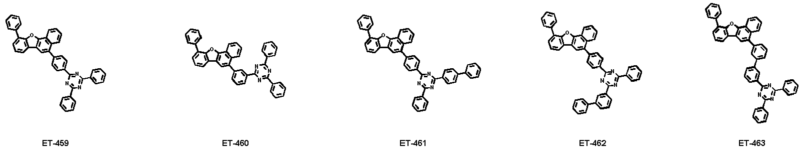

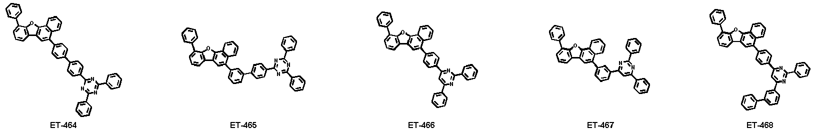

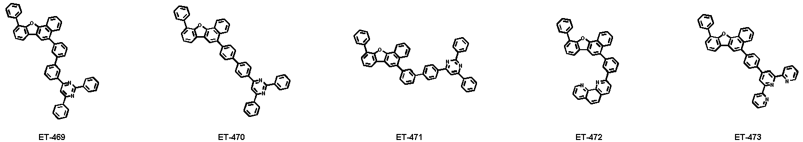

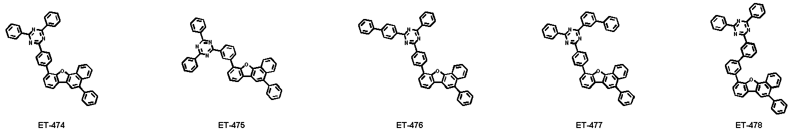

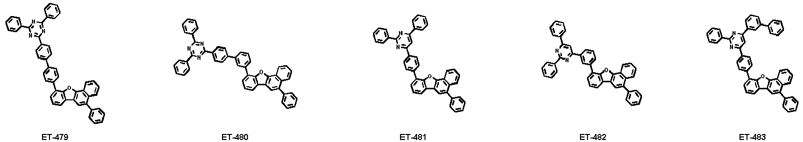

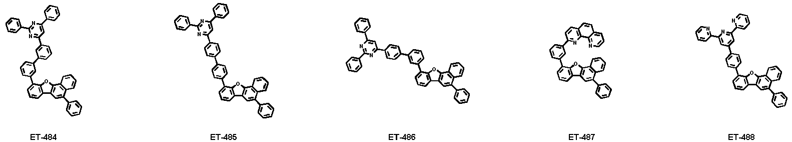

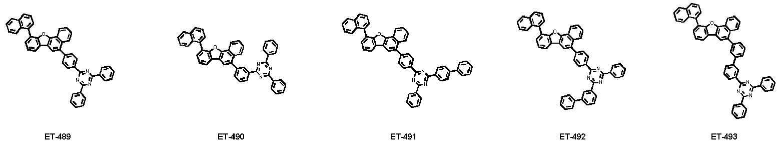

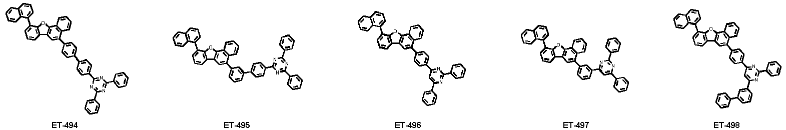

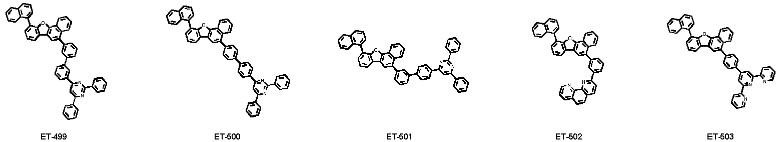

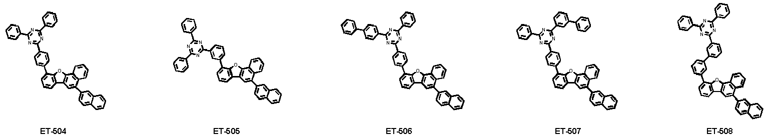

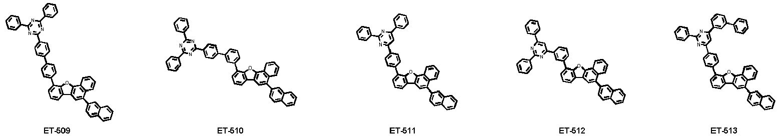

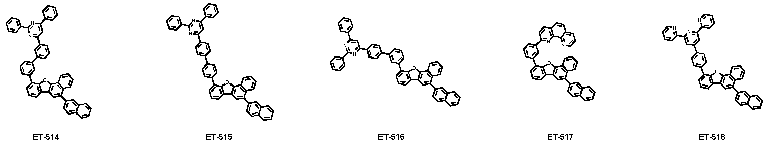

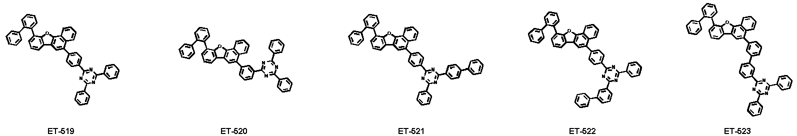

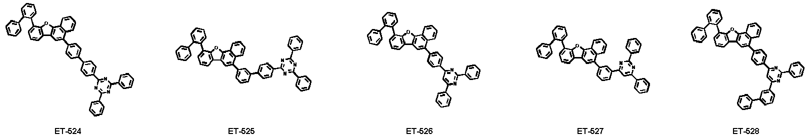

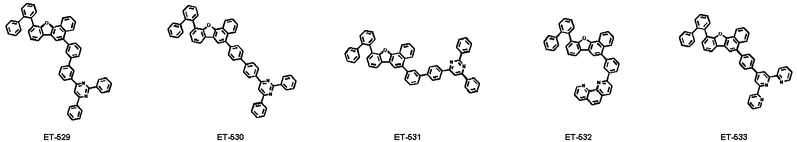

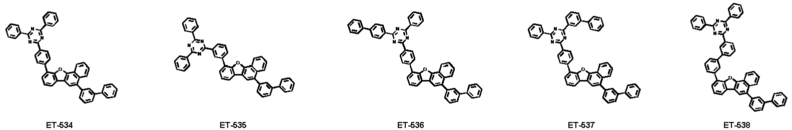

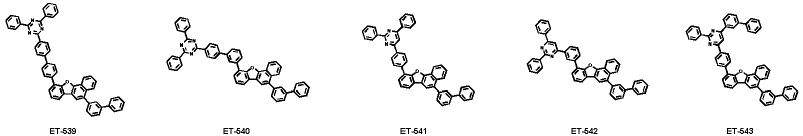

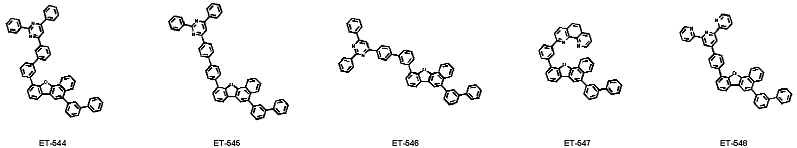

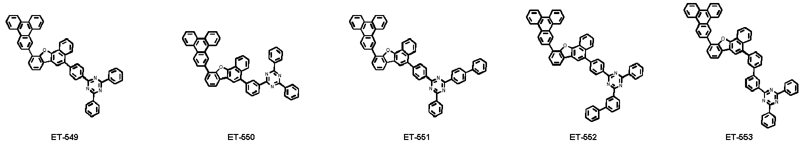

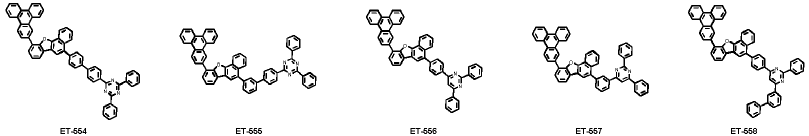

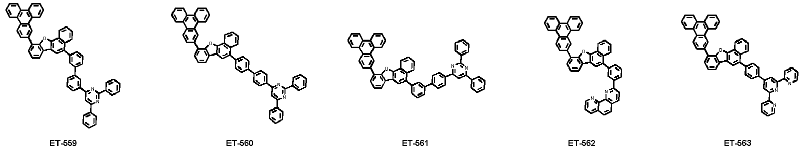

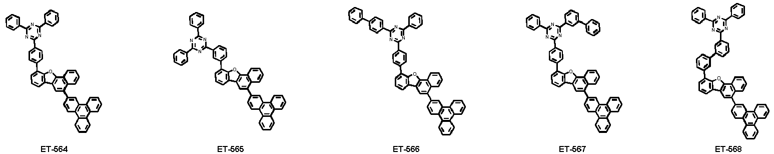

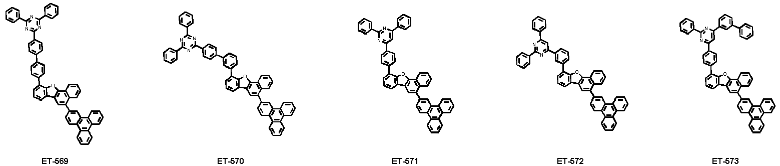

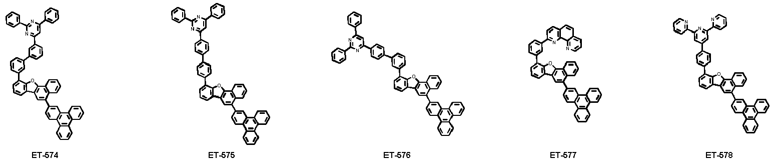

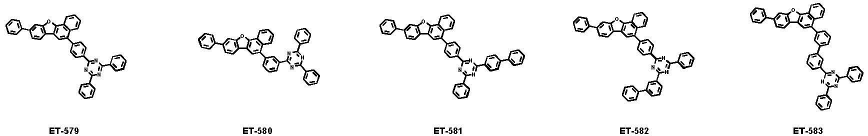

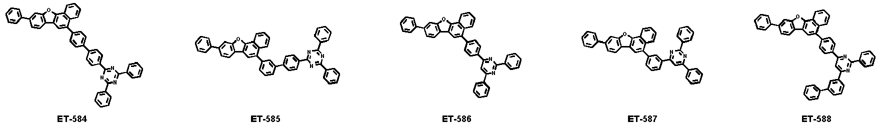

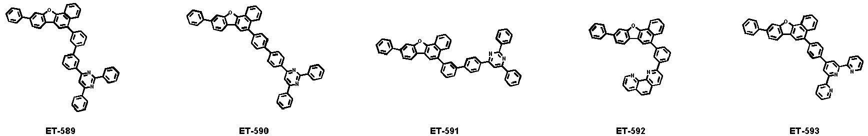

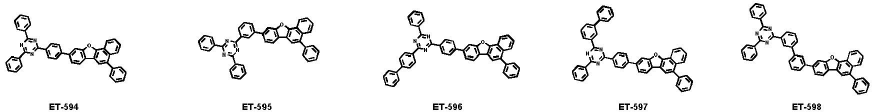

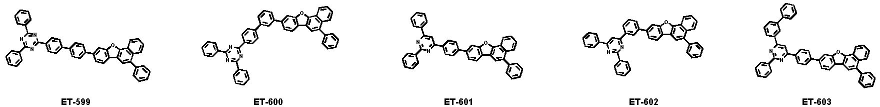

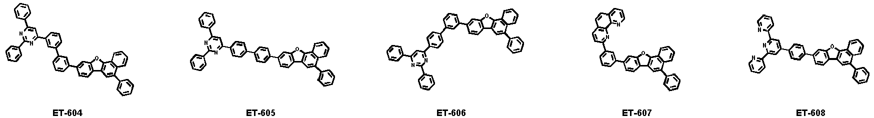

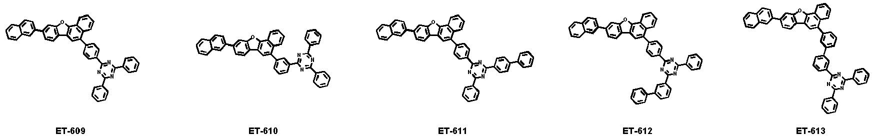

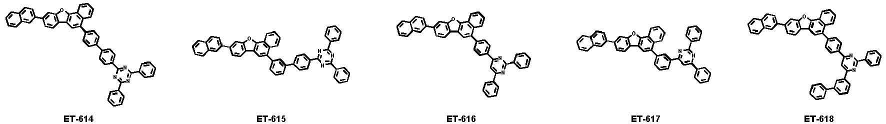

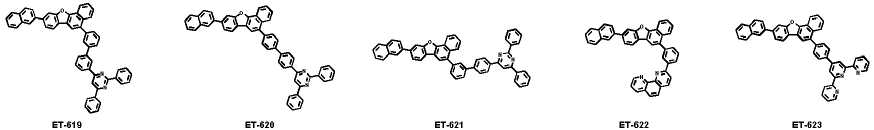

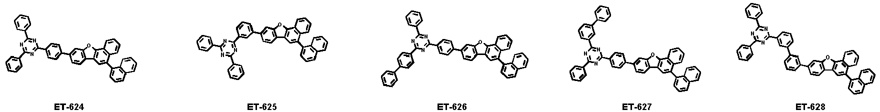

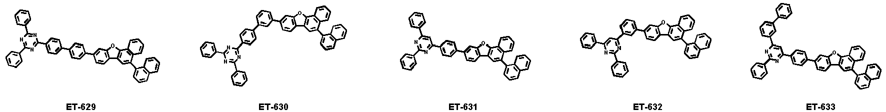

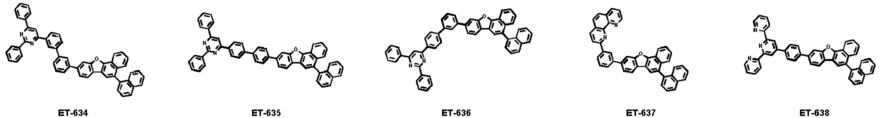

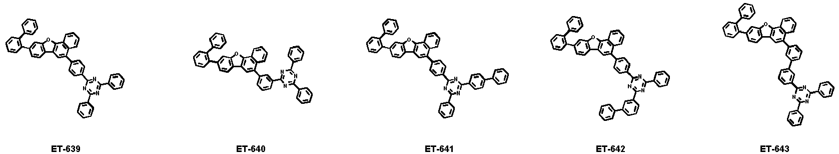

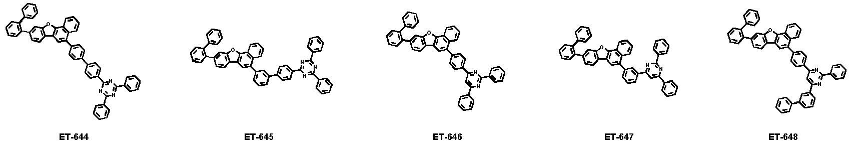

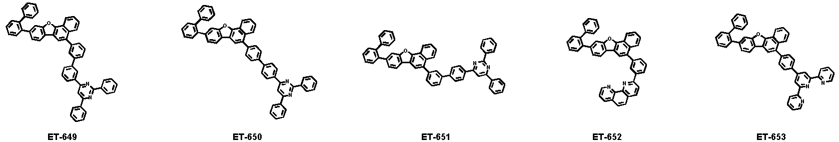

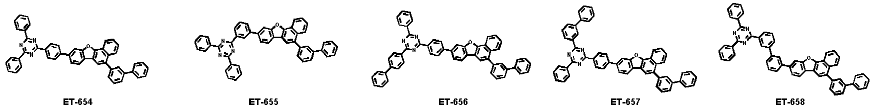

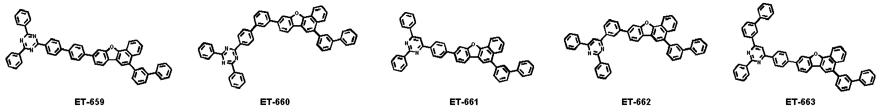

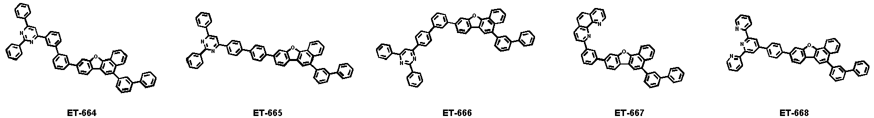

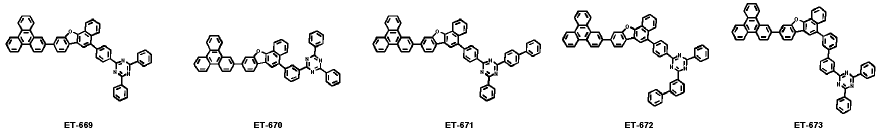

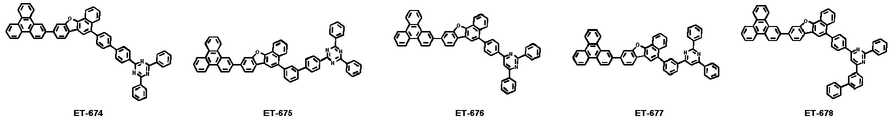

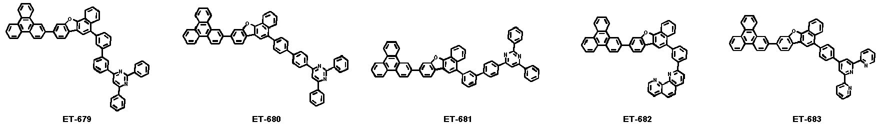

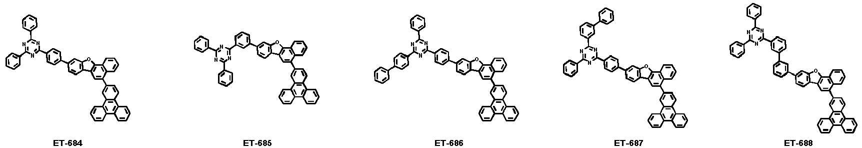

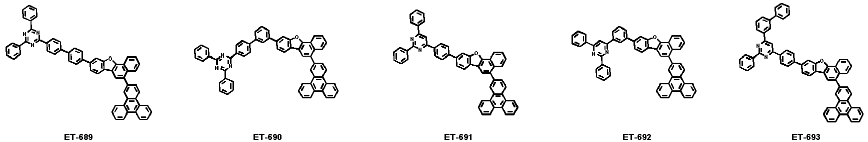

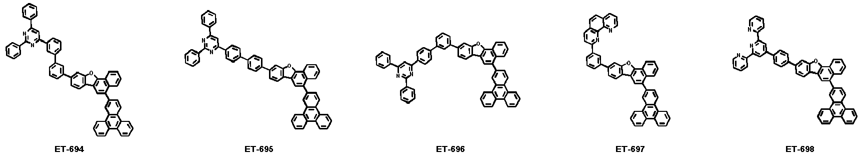

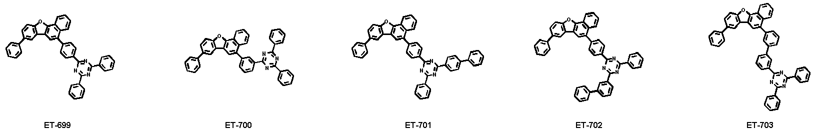

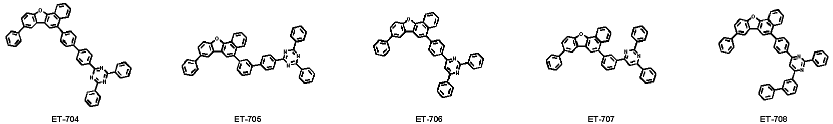

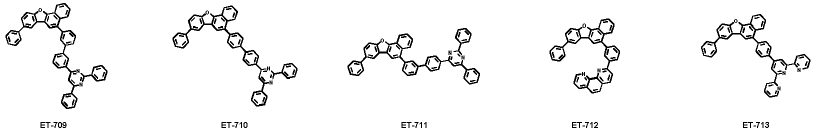

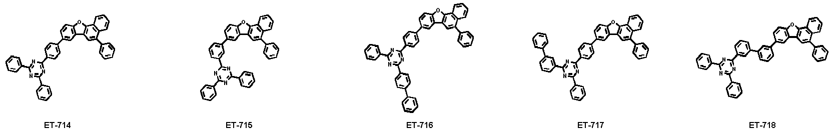

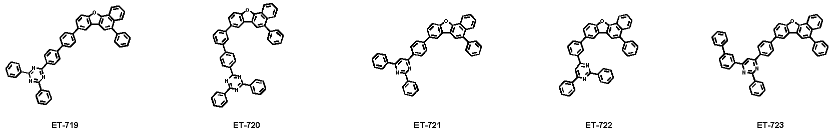

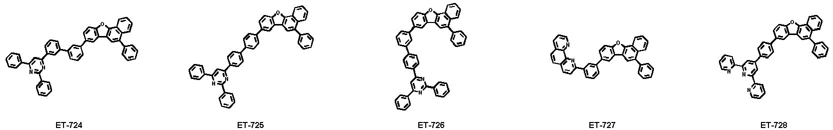

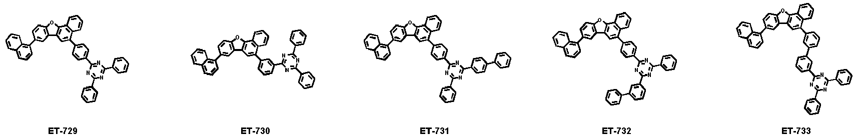

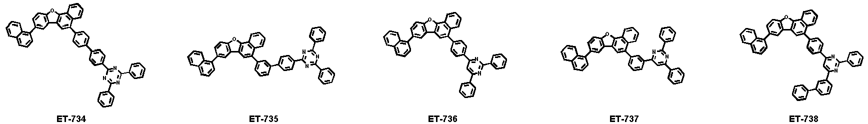

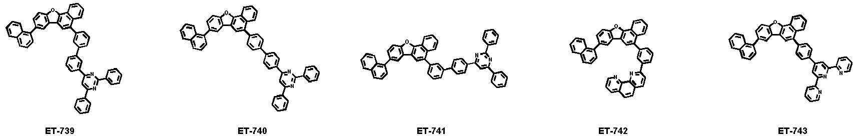

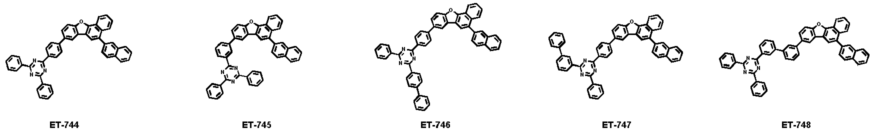

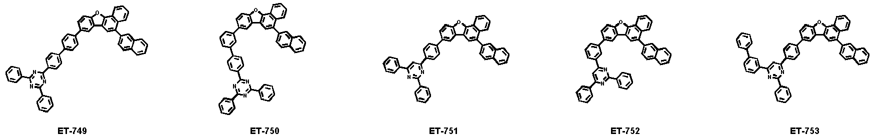

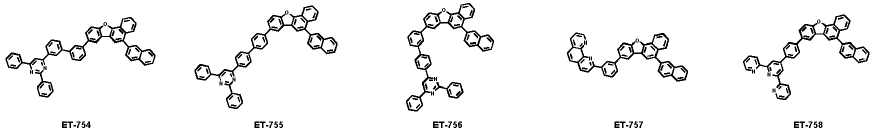

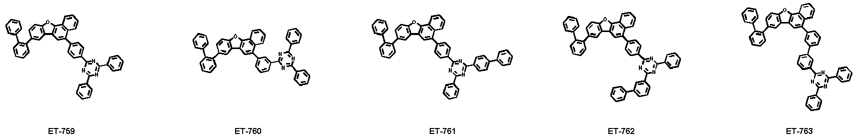

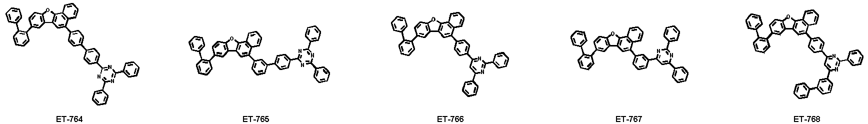

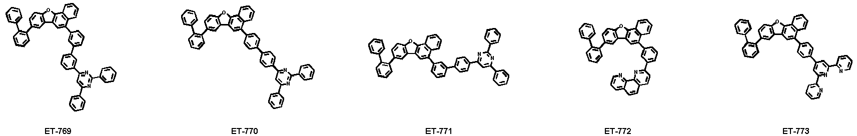

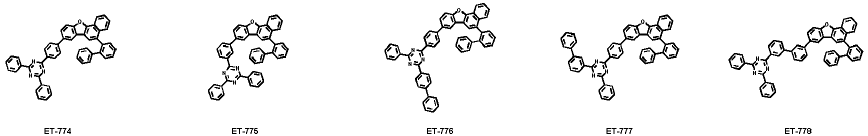

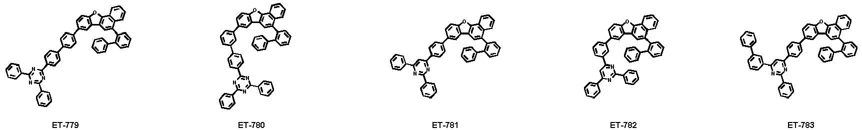

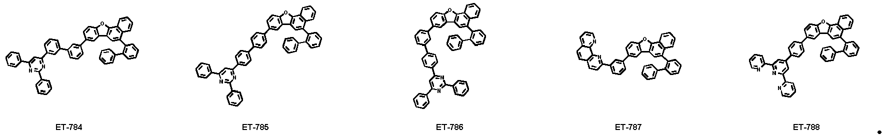

6. An organic light emitting device comprising:
a first electrode;
a second electrode; and
one or more organic material layers provided between the first electrode and the second electrode,
wherein one or more layers of the organic material layers comprise the heterocyclic compound of any one of Claims 1 to 5.

7. The organic light emitting device of Claim 6, wherein the organic material layer comprises an electron transfer layer, and the electron transfer layer comprises the heterocyclic compound.

8. The organic light emitting device of Claim 6, wherein the organic material layer comprises one or more hole blocking layers, and the hole blocking layer comprises the heterocyclic compound.

9. The organic light emitting device of Claim 6 comprising:
a first stack provided on the first electrode and comprising a first light emitting layer;
a charge generation layer provided on the first stack;
a second stack provided on the charge generation layer and comprising a second light emitting layer; and
the second electrode provided on the second stack.

10. The organic light emitting device of Claim 9, wherein the charge generation layer comprises the heterocyclic compound.
